(19) **Europäisches Patentamt / European Patent Office / Office européen des brevets**

(11) **EP 3 948 286 B1**

(12) **EUROPEAN PATENT SPECIFICATION**

(45) Date of publication and mention
of the grant of the patent:
**23.07.2025 Bulletin 2025/30**

(21) Application number: **20706738.0**

(22) Date of filing: **25.02.2020**

(51) International Patent Classification (IPC):
**G01N 33/574** (2006.01)

(52) Cooperative Patent Classification (CPC):
**G01N 33/574; G01N 33/57484;** G01N 2800/52;
G01N 2800/54

(86) International application number:
**PCT/EP2020/054935**

(87) International publication number:
**WO 2020/193045 (01.10.2020 Gazette 2020/40)**

(54) **CANCER PROGNOSIS**

KREBSPROGNOSE

PRONOSTIC DU CANCER

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **28.03.2019 EP 19166011**

(43) Date of publication of application:
**09.02.2022 Bulletin 2022/06**

(73) Proprietor: **F. Hoffmann-La Roche AG
4070 Basel (CH)**

(72) Inventors:
• **RUETTINGER, Dominik
82377 Penzberg (DE)**
• **BAUER-MEHREN, Anna
82377 Penzberg (DE)**
• **BECKER, Tim
47877 Willich (DE)**

(74) Representative: **J A Kemp LLP
80 Turnmill Street
London EC1M 5QU (GB)**

(56) References cited:
• ROSCOE D M ET AL: "Primate antibody response to immunotoxin: serological and computer-aided analysis of epitopes on a truncated form of pseudomonas exotoxin", INFECTION AND IMMUNITY, AMERICAN SOCIETY FOR MICROBIOLOGY, US, vol. 62, no. 11, 1 November 1994 (1994-11-01), pages 5055 - 5065, XP002968542, ISSN: 0019-9567
• VICTORIA LOUISE REID ET AL: "A systematically structured review of biomarkers of dying in cancer patients in the last months of life; An exploration of the biology of dying", PLOS ONE, vol. 12, no. 4, 6 April 2017 (2017-04-06), US, pages e0175123 - e0175123, XP055619718, ISSN: 1932-6203, DOI: 10.1371/ journal.pone.0175123
• MARCO MALTONI ET AL: "Conclusion", JOURNAL OF CLINICAL ONCOLOGY, vol. 23, no. 25, 1 September 2005 (2005-09-01), US, pages 6240 - 6248, XP055677263, ISSN: 0732-183X, DOI: 10.1200/JCO.2005.06.866

## Description

### Field of the Invention

The present invention relates to computer-implemented methods of assessing whether a cancer patient is at high risk or low risk of mortality, as well as methods of predicting the treatment response to an anti-cancer therapy in a cancer patient. The methods of the invention find application in the selection of patients for clinical trials, the selection of patients for treatment with anti-cancer therapies, monitoring cancer patients during treatment with an anti-cancer therapy, and evaluating the results of clinical trials for anti-cancer therapies, for example.

### Background to the invention

Factors influencing life expectancy are highly important in public health (Ganna & Ingelsson, 2015). In oncology, prediction of patient survival is instrumental for optimal patient management (Halabi & Owzar, 2010). By understanding which variables are prognostic of outcome it is possible to gain insights into disease biology, can individualize patient treatment, and may be able to improve the design, conduct, and data analysis of clinical trials.

Currently research on prognostic and predictive factors in oncology is largely based on small sample sizes. Hence, association with mortality is predominantly studied for one risk factor at a time (Banks *et al.,* 2013; Hu et a., 2004; McGee *et al.,* 1999; Thun *et al.,* 1997; Tota-Maharaj *et al.,* 2012). Even existing prognostic scores, such as the Royal Marsden Hospital Score (RMHS) (Nieder & Dalhaug, 2010), the international prognostic index (IPI) (N. Engl. J. Med., 329:987-94, 1993), the Glasgow prognostic score (GPS) or the modified Glasgow prognostic score (mGPS) (Kinoshita *et al.,* 2013; Nozoe *et al.,* 2014; Jin *et al.,* 2017; Grose *et al.,* 2014), are constructed from a small number of risk factors, typically less than five. These small sample sizes do not allow for simultaneous assessment of multiple factors (Altman & Simon, 1994; Graf *et al.,* 1999). A number of biomarkers have been proposed as prognostic indicators of the dying process in cancer patients in the literature and the findings, as well as the weaknesses of the reported studies (such as small sample sizes, in particular relative to the number of parameters being assessed, as well as univariate rather than multivariate analyses being conducted), are summarised in Reid *et al.,* 2017. Although a high grade of evidence is reported for some biomarkers, Reid *et al.,* 2017 provide no evidence that mortality could be accurately predicted based on such biomarkers, and do not describe a model which could be used to make such a prediction.

Accordingly, there is an unmet need for larger sample sizes to allow more accurate predictions that increase confidence in clinical decision making. The recent UK biobank initiative (Sudlow *et al.,* 2015) constitutes an important addition to the available data and was used by Ganna and Ingelsson (2015) to investigate life expectancy in a population-based sample of ~500,000 participants and construct a mortality risk score outperforming the Charlson comorbidity index (Charlson *et al.,* 1987). Nevertheless, given the high impact of cancer on public health (Stock *et al.,* 2018), there remains a need in the art for more accurate tests for predicting mortality and treatment response of cancer patients to improve *inter alia* patient treatment, and the design, conduct, and data analysis of clinical trials.

### Statements of invention

The inventors have developed a new computer-implemented method of assessing mortality risk of a cancer patient or predicting the treatment response to an anti-cancer therapy in a cancer patient, based on a plurality of parameters. The method stems from the discovery that training data including routinely measured parameters for a large number of subjects can be utilised to form a model that produces a more reliable indication of risk of mortality and treatment response than currently known scores.

Specifically, the present inventors conducted a survival analysis using data from the Flatiron Health database for 99,249 people from 12 different cohorts (RoPro1) and 110,538 people from 15 different cohorts (RoPro2), the cohorts being defined by tumour type, and validated the results in two independent clinical studies. Demographic and clinical variables (focusing on routinely collected clinical and laboratory data), diagnosis, and treatment were examined, alongside real-world mortality as the endpoint (Curtis *et al.,* 2018) and survival time from the first line of treatment was assessed. As mentioned above, the focus lay parameters that are routinely collected in clinical practice, which allows the method to be applied in a variety of context without the need to collect patient parameter data specifically for the analysis. This is an advantage over scores which rely on the measurement of parameters which are not routinely measured in the clinic.

A total of 26 parameters (RoPro1) and 29 parameters (RoPro2) were identified which are routinely measured for cancer patients and were shown to be capable of predicting risk of mortality of patients with a wide-variety of cancers with substantially greater accuracy than the Royal Marsden Hospital Score (RMHS), as demonstrated by greater accuracy of prediction of length of time patients remained in a Phase I study (BP29428) investigating the safety, pharmacokinetics, and preliminary anti-tumour activity of emactuzumab and atezolizumab in patients with selected locally advanced or metastatic solid tumours. The present inventors have further shown that the use of 13 of the 26 parameters of the RoPro1 allows the risk of mortality to be predicted with an accuracy close to that achieved with the use of all 26 parameters, and that the use of as few as 4 or the 26 parameters is sufficient to predict risk of mortality of patients with significantly greater accuracy than RMHS. The present inventors have similarly shown that the use of 13 of the 29 parameters of the RoPro2 allows the risk of mortality to be predicted with an accuracy close to that achieved with the use of all 29 parameters and that the use of as few as 4 of the 29 parameters is sufficient to predict risk of mortality of patients with significantly greater accuracy than RMHS.

Specifically, the present inventors have shown that the RoPro1 and RoPro2 strongly outperform the RMHS in correlation with time-to-death (mortality risk) when patient information comprising data corresponding to as few as four parameters selected from the parameters listed under (i) to (xxvi) or (i) to (xxix) below, respectively, are used to calculated the RoPro1 (top 4 parameters: $r^2 = 0.15$; top 5 parameters: $r^2 = 0.16$; top 10 parameters: $r^2 = 0.17$; top 13 parameters: $r^2 = 0.19$; whereby top 4, top 5, top 10 and top 13 refers to the parameters with rank numbers 1-4, 1-5, 1-10, and 1-13 listed in Table 15 for RoPro1, respectively) or Ropro2 (top 4 parameters: $r^2 = 0.174$; top 5 parameters: $r^2 = 0.184$; top 10 parameters: $r^2 = 0.288$; top 13 parameters: $r^2 = 0.299$; whereby top 4, top 5, top 10 and top 13 refers to the parameters with rank numbers 1-4, 1-5, 1-10, and 1-13 listed in Table 15 for RoPro2, respectively).

Thus, data corresponding on any combination of at least four parameters selected from parameters (i) to (xxvi) or (i) to (xxix) below is suitable to form a useful score. For example, at least five, at least six, at least seven, at least eight, at least nine, at least ten, at least eleven, at least twelve; at least thirteen, at least fourteen, at least fifteen, at least sixteen, at least seventeen, at least eighteen, at least nineteen, at least twenty, at least twenty-one, at least twenty-two, at least twenty-three, at least twenty-four, at least twenty-five, at least twenty-six, at least twenty-seven, at least twenty-eight parameters, or twenty-nine parameters selected from parameters (i) to (xxvi) or (i) to (xxix) may be used.

In a preferred embodiment, data corresponding to all thirteen parameters (i) to (xiii) below is selected.

A first aspect of the present invention provides a computer-implemented method of assessing risk of mortality of a cancer patient, the method comprising; using a model to generate a score indicative of a risk of mortality of the cancer patient by inputting cancer patient information to the model; and assessing the risk of mortality by comparing the generated score to one or more predetermined threshold values, or by comparing the generated score to generated scores for other cancer patients in a same group, wherein the patient information comprises data corresponding to each of the following parameters:

(i) Level of albumin in serum or plasma;
(ii) Eastern cooperative oncology group (ECOG) performance status;
(iii) Ratio of lymphocytes to leukocytes in blood;
(iv) smoking status;
(v) Age;
(vi) TNM classification of malignant tumours stage;
(vii) Heart rate;
(viii) Chloride or sodium level in serum or plasma, preferably chloride level in serum or plasma;
(ix) Urea nitrogen level in serum or plasma;
(x) Gender;
(xi) Haemoglobin or haematocrit level in blood, preferable haemoglobin level in blood;
(xii) Aspartate aminotransferase enzymatic activity level in serum or plasma; and
(xiii) Alanine aminotransferase enzymatic activity level in serum or plasma.

The present inventors have further shown that use of the 26 parameters or 29 parameters, or subsets thereof, are suitable for predicting the treatment response of a cancer patient to an anti-cancer therapy.

Disclosed but not claimed is a method of predicting the treatment response of a cancer patient to an anti-cancer therapy, the method comprising inputting cancer patient information to a model to generate a score indicative of the treatment response of the cancer patient. The patient information may comprise data corresponding to each of the following parameters:

(i) Level of albumin in serum or plasma;
(ii) Eastern cooperative oncology group (ECOG) performance status;
(iii) Ratio of lymphocytes to leukocytes in blood;
(iv) Smoking status;
(v) Age;
(vi) TNM classification of malignant tumours stage;
(vii) Heart rate;
(viii) Chloride or sodium level in serum or plasma, preferably chloride level in serum or plasma;
(ix) Urea nitrogen level in serum or plasma;

(x) Gender;
(xi) Haemoglobin or haematocrit level in blood, preferable haemoglobin level in blood;
(xii) Aspartate aminotransferase enzymatic activity level in serum or plasma; and
(xiii) Alanine aminotransferase enzymatic activity level in serum or plasma.

Disclosed but not claimed is that the method may further comprise selecting a patient predicted to benefit from treatment with the anti-cancer therapy, for treatment for treatment with the anti-cancer therapy, or treating a patient predicted to benefit from treatment with the anti-cancer therapy with the anti-cancer therapy.

Also disclosed but not claimed is a method of treating a cancer patient with an anti-cancer therapy, the method comprising:

(i) predicting the treatment response to an anti-cancer therapy in a cancer patient; or
(ii) ordering test results of a method for predicting the treatment response to an anti-cancer therapy in a cancer patient;

wherein the method comprises inputting cancer patient information to a model to generate a score indicative of the treatment response of the cancer patient. The patient information may comprise data corresponding to each of the following parameters:

(i) Level of albumin in serum or plasma;
(ii) Eastern cooperative oncology group (ECOG) performance status;
(iii) Ratio of lymphocytes to leukocytes in blood;
(iv) Smoking status;
(v) Age;
(vi) TNM classification of malignant tumours stage;
(vii) Heart rate;
(viii) Chloride or sodium level in serum or plasma, preferably chloride level in serum or plasma;
(ix) Urea nitrogen level in serum or plasma;
(x) Gender;
(xi) Haemoglobin or haematocrit level in blood, preferable haemoglobin level in blood;
(xii) Aspartate aminotransferase enzymatic activity level in serum or plasma; and
(xiii) Alanine aminotransferase enzymatic activity level in serum or plasma; and

administering a pharmaceutically effective amount of the anti-cancer therapy to a patient predicted to respond to the anti-cancer therapy.

Further disclosed but not claimed is an anti-cancer therapy for use in a method of treating cancer in a patient, the method comprising predicting the treatment response to an anti-cancer therapy in a cancer patient, the method comprising inputting cancer patient information to a model to generate a score indicative of the treatment response of the cancer patient. The patient information may comprise data corresponding to each of the following parameters:

(i) Level of albumin in serum or plasma;
(ii) Eastern cooperative oncology group (ECOG) performance status;
(iii) Ratio of lymphocytes to leukocytes in blood;
(iv) smoking status;
(v) Age;
(vi) TNM classification of malignant tumours stage;
(vii) Heart rate;
(viii) Chloride or sodium level in serum or plasma, preferably chloride level in serum or plasma;
(ix) Urea nitrogen level in serum or plasma;
(x) Gender;
(xi) Haemoglobin or haematocrit level in blood, preferable haemoglobin level in blood;
(xii) Aspartate aminotransferase enzymatic activity level in serum or plasma; and
(xiii) Alanine aminotransferase enzymatic activity level in serum or plasma; and

administering a pharmaceutically effective amount of the anti-cancer therapy to a patient predicted to respond to the anti-cancer therapy.

In some embodiments of the disclosure, the patient information may comprise, or consist of, data corresponding to more than four parameters selected from parameters (i) to (xiii) but fewer than all of the parameters (i) to (xiii). For example the patient information may comprise, or consist of, data corresponding to five, six, seven, eight, nine, ten, eleven, or twelve parameters selected from parameters (i) to (xiii). Alternatively, the patient information may comprise, or consist of, data corresponding to all thirteen parameters selected from parameters (i) to (xiii). Preferably, the patient information comprises, or consist of, data corresponding to at least five, at least six, at least seven, at least eight, at least nine, at least ten, at least eleven, at least twelve, or all thirteen parameters selected from parameters (i) to (xiii). More preferably, the patient information comprises, or consist of, data corresponding to at least five, at least six, at least seven, at least eight, or at least nine parameters selected from parameters (i) to (xiii). For example, the patient information may comprise, or consist of, data corresponding to at least five parameters selected from the parameters (i) to (xiii). Alternatively, the patient information may comprise, or consist of, data corresponding to at least six parameters selected from the parameters (i) to (xiii). As a further alternative, the patient information may comprise, or consist of, data corresponding to at least seven parameters selected from parameters (i) to (xiii). As a yet further alternative, the patient information may comprise, or consist of, data corresponding to at least eight parameters selected from parameters (i) to (xiii). As another alternative, the patient information may comprise, or consist of, data corresponding to at least nine parameters selected from parameters (i) to (xiii).

For example, the patient information may comprise, or consist of, data corresponding to all of parameters (i) to (v). The inventors have found that selecting these parameters can improve the accuracy of the assessment of mortality risk.

Alternatively, the patient information may comprise, or consist of, data corresponding to all of parameters (i) to (xi) and one or both of (xii) and (xiii).

The treatment response to an anti-cancer therapy in a cancer patient may be a complete response, progression-free survival, a partial response, or cancer progression. A complete response (complete remission) may refer to the absence of detectable disease (cancer) in the patient. Progression-free-survival may refer to survival of the patient for a period of time during which there is no worsening of the disease (cancer). A partial response may refer to a decrease in the size of the tumour(s) or reduction in the spread of the cancer in the patient's body. A complete response (also referred to as complete remission) may refer to the absence of detectable disease in the patient. Cancer progression may refer to a worsening of the disease (cancer), such as an increase in tumour size and/or an increase in the number of tumours in the patient's body. Methods of detecting a complete response, particle response, progression-free survival and cancer progression in a cancer patient in response to an anti-cancer therapy are well known in the art.

Unless the context requires otherwise, an anti-cancer therapy as referred to herein may be a known anti-cancer therapy for the cancer in question, such as radiation therapy, chemotherapy, immunotherapy, hormone therapy, and/or surgery. For example, the anti-cancer therapy may be known anti-cancer therapy for advanced non-small-cell lung carcinoma (NSCLC), bladder cancer, chronic lymphocytic leukaemia (CLL), diffuse large **B**-cell lymphoma (DLBCL), hepatocellular carcinoma (HCC), metastatic breast cancer, metastatic colorectal cancer (CRC), metastatic renal cell carcinoma (RCC), multiple myeloma, ovarian cancer, small cell lung cancer (SCLC). The anti-cancer therapy may alternatively be a known anti-cancer therapy for follicular lymphoma, pancreatic cancer, or head & neck cancer.

A patient, as referred to herein, is preferably a human patient. Where the method comprises predicting the treatment response to an anti-cancer therapy, the patient may be a patient who has not previously been treated with said anti-cancer therapy, unless the context requires otherwise.

The patient information may further comprise data corresponding to one or more parameters selected from:

(xiv) Systolic or diastolic blood pressure, preferably systolic blood pressure;
(xv) Lactate dehydrogenase enzymatic activity level in serum or plasma;
(xvi) Body mass index;
(xvii) Protein level in serum or plasma;
(xviii) Platelet level in blood;
(xix) Number of metastatic sites;
(xx) Ratio of eosinophils to leukocytes in blood;
(xxi) Calcium level in serum or plasma;
(xxii) Oxygen saturation level in arterial blood;
(xxiii) Alkaline phosphatase enzymatic activity level in serum or plasma;
(xxiv) Neutrophil to lymphocyte ratio (NLR) in blood;
(xxv) Total bilirubin level in serum or plasma; and
(xxvi) Leukocyte level in blood.

In addition, or alternatively, the patient information may further comprises data corresponding to one or more parameters selected from:

(xxvii) Lymphocyte level in blood;
(xxviii) Carbon dioxide level in blood; and
(xxix) Monocyte level in blood.

**EP 3 948 286 B1**

In some embodiments, the patient information may comprise data corresponding to more than one parameter selected from parameters (xiv) to (xxvi) and/or (xxvii) to (xxix). For example the patient information may additionally or alternatively comprise data corresponding to at least two, at least three, art least four, at least five, at least six, at least seven, at least eight, at least nine, at least ten, at least eleven, at least twelve, or all thirteen of the parameters selected from parameters (xiv) to (xxvi) and/or at least one, at least two, or all three parameters selected from parameters (xxvii) to (xxix). In some embodiments, parameters other than (i) to (xxvi) or (i) to (xxix) may also be included in the patient information and training data.

For example, the patient information may comprise, or consist of, data corresponding to at least four parameters selected from the parameters (i) to (xiii), and at least two further parameters selected from the parameters (i) to (xxvi) or (i) to (xxix).

Alternatively, the patient information may comprise data corresponding to at least seven parameters selected from parameters (i) to (xxix), wherein at least one parameter is selected from parameters (xxvii) to (xxix).

For example, the cancer patient information may comprise data corresponding to at least eight, at least nine, at least ten, at least eleven, at least twelve, at least thirteen, at least fourteen, at least fifteen, at least sixteen, at least seventeen, at least eighteen, at least nineteen, at least twenty, at least twenty-one, at least twenty-two, at least twenty-three, at least twenty-four, at least twenty-five, at least twenty-six, at least twenty-seven, or at least twenty-eight parameters selected from parameters (i) to (xxix), wherein at least one parameter is selected from parameters (xxvii) to (xxix).

The selected parameter(s) from parameters (xxvii) to (xxix), preferably is or includes parameter (xxvii) (lymphocyte level in blood).

As a further alternatively, the patient information may comprise data corresponding to parameter (xxvii) and five or more parameters selected from parameters (i), (ii), (iii), (v), (vi), (vii), (viii), (ix), (xi), (xviii), and (xxiii).

For example, the cancer patient information may comprise data corresponding to parameter (xxvii) and at least six, at least seven, at least eight, at least nine, at least ten, at least eleven, at least twelve, at least thirteen, at least fourteen, at least fifteen, at least sixteen, at least seventeen, at least eighteen, at least nineteen, at least twenty, at least twenty-one, at least twenty-two, at least twenty-three, at least twenty-four, at least twenty-five, at least twenty-six, at least twenty-seven, at least twenty-eight, or all twenty-nine parameters selected from parameters (i) to (xxix), wherein at least one parameter is selected from parameters (xxvii) to (xxix).

The patient information may comprise, or consist of, data corresponding to all of parameters (i) to (xxvi). The inventors have shown that all of these parameters have an independent contribution to the model. Such a score correlates well with time-to-death ($r^2$=0.24 on an example data set) and strongly outperforms the RMHS ($r^2$=0.02 on the same example data set). Preferably, the patient information comprises, or consist of, data corresponding to all of parameters (i) to (xxix). The inventors have shown that all of these parameters have an independent contribution to the model. Such a score shows improved correlation with time-to-death ($r^2$=0.30 on an example data set) and again strongly outperforms the RMHS ($r^2$=0.04 on the same example data set).

In general, using more of the parameters (i) to (xiii), (i) to (xxvi), or (i) to (xxix) thus results in a more accurate model and score, but practically it may be difficult to obtain patient information and/or training data for one or more of the parameters, so the accuracy of the model and score can be balanced against practical constraints.

The model may be formed by performing statistical significance analysis on training data, the training data including the selected parameters for a plurality of subjects. The statistical significance analysis may comprise multivariable cox regression analysis. In some embodiments, one or more other statistical analysis techniques may be used.

The training data may also include information indicative of the risk of mortality of the subjects. For example, the training data may include an indication of overall survival, survival time, time between a subject's first line of treatment and the last documented contact or another indication of mortality risk of each subject. The training data may include censored follow-up times indicating the time elapsed from the first line of treatment to the date of the patient's last documented contact with their clinic. The last contact may be the last visit, medication administration, specimen collection or another contact.

The use of a selection of the parameters (i) to (xxvi) or (i) to (xxix) is advantageous as these parameters are routinely measured and/or available in the clinic. Thus the score is easy to use as the data on the parameters (i) to (xxvi) or (i) to (xxix) is available for most cancer patients, whilst also providing high accuracy of prediction.

A useful score may be obtained even if not all of the parameters are available for a patient. So, the number of parameters selected for the patient information, may be fewer than the number of parameters included in the training data. For example, if a model is formed using training data including all of the parameters (i) to (xxvi) or (i) to (xxix), the patient information can include less than all of these parameters and a score with similar accuracy can still be produced. For example, 13, 14, 15 or 16 parameters from the list (i) to (xxvi) or (i) to (xxix) may be input to a model including all of the parameters (i) to (xxvi) or (i) to (xxix). This increases the ease of use of the method as the method can still assess patients with missing patient information.

Training data may be obtained from a database, which may be derived from electronic health record data. For example, the Flatiron Health database. The database and/or the training data may include structured and/or unstructured data. Subjects in the training data may be cancer patients. Subjects may be included or excluded from the training data based on cancer-type. For example, in order to tailor the method to a patient with a first cancer type, the training data may exclude subjects that do not have the first cancer type.

Parameters may be selected for use in the model based on their availability in the training data and/or patient data. For example, in some embodiments, only parameters that are available for at least 75% of the patients in the database may be selected for the model, or only parameters that are available for more than 25% of the patients in the database may be selected for the model. In order to improve the training data, patients with missing treatment information, may be excluded from the training data. Missing data may be imputed to improve the training data. This may be performed using a suitable algorithm, for example, the missForest R package.

To improve the training data, outlying data may be excluded. For example, for continuous parameters, observations more than 4 standard deviations from the mean may be excluded.

Data may be screened so that insignificant parameters are excluded from the model. The inventors have found the parameters (i) to (xxvi) and (i) to (xxix) to be significant in assessment of mortality risk and the prediction of the treatment response to an anti-cancer therapy, with a model employing parameters (i) to (xxix) outperforming a model employing parameters (i) to (xxvi). Screening may comprise analysing each parameter using Bonferroni-correction and excluding parameters with a p-value larger than a threshold value. For example, parameters with a p-value of 0.05 divided by the number of parameters considered or larger may be excluded from the training data. Parameters may be included in the model in the order of their significance (predictive value) during screening.

The predictive value of parameters (i) to (xxvi) in RoPro1 is detailed in **Table 15** with rank (1) denoting the most predictive parameter (albumin) and rank (26) denoting the least predictive parameter (leukocyte level) among parameters (i) to (xxvi) in this model. The predictive value of parameters (i) to (xxix) in RoPro2 is also detailed in **Table 15,** with rank (1) again denoting the most predictive parameter (albumin) and rank (29) denoting the least predictive parameter (carbon dioxide level) among parameters (i) to (xxix) in this model. Thus, the lower the rank number of a parameter in **Table 15,** the more preferred it is that the patient information comprises data corresponding to said parameter.

As the inventors have found that in RoPro1 parameters (i) to (xxvi) are significant in the numeric order of the parameters as listed in (i) to (xxvi) above (which corresponds to the rank order shown in **Table 15),** a model may be formed by including the selected parameters from parameters (i) to (xxvi) in this order.

In a preferred embodiment, the patient information may thus comprise or consist of data corresponding to the parameters with ranks 1 to 4, 1 to 5, 1 to 6, 1 to 7, 1 to 8, 1 to 9, 1 to 10, 1 to 11, 1 to 12, 1 to 13, 1 to 14, 1 to 15, 1 to 16, 1 to 17, 1 to 18, 1 to 19, 1 to 20, 1 to 21, 1 to 22, 1 to 23, 1 to 24, 1 to 25, or 1 to 26 in RoPro1 as set out in **Table 15,** whereby the parameters with ranks 1 to 4 in RoPro1 correspond to the level of albumin in serum or plasma, ECOG performance status, ratio of lymphocytes to leukocytes in blood, and smoking status.

As the inventors have found that parameters (i) to (xxix) are significant in the rank order shown in **Table 15** for RoPro2 and that such a model predicts OS more accurately than a model based on parameters (i) to (xxvi), in a preferred embodiment a model may be formed by including the selected parameters from parameters (i) to (xxix) in the rank order shown in **Table 15.**

Thus, in a more preferred embodiment, the patient information may comprise or consist of data corresponding to the parameters with ranks 1 to 4, 1 to 5, 1 to 6, 1 to 7, 1 to 8, 1 to 9, 1 to 10, 1 to 11, 1 to 12, 1 to 13, 1 to 14, 1 to 15, 1 to 16, 1 to 17, 1 to 18, 1 to 19, 1 to 20, 1 to 21, 1 to 22, 1 to 23, 1 to 24, 1 to 25, 1 to 26, 1 to 27, 1 to 28, or 1 to 29 in RoPro2 as set out in **Table 15,** whereby the parameters with ranks 1 to 4 in RoPro2 correspond to the level of albumin in serum or plasma, lymphocyte level in blood, ECOG performance status, and leukocyte level in blood.

One or more of the parameters listed in (i) to (xxix) above may be substituted with a suitable substitute parameter which correlates with the relevant parameter. Suitable substitute parameters are listed in Table 15. For example, the chloride level in serum or plasma may be substituted with sodium level in serum or plasma, the haemoglobin level in blood may be substituted with haematocrit level in blood, the alanine aminotransferase (ALT) enzymatic activity level in serum or plasma may be substituted with aspartate aminotransferase (AST) enzymatic activity level in serum or plasma, and/or the systolic blood pressure may be substituted with diastolic blood pressure.

Methods for measuring or assessing parameters (i) to (xxix) above, as well as the substitute parameters referred to herein, are known in the art and are routinely measured in a clinical setting. Measuring these parameters is therefore well within the capabilities of the skilled person. The method of measurement for a given parameter is preferably consistent between the cancer patient being analysed and any training or validation data sets. Where necessary, results from different measurement methods can be normalised to allow comparison between data obtained from said methods.

Exemplary methods for measuring parameters (i) to (xxix) above, as well as the substitute parameters referred to herein, are detailed in Table 15. Where available, Table 15 also sets out the LOINC codes (version 2.65; released 14 December 2018) for parameters referred to herein. The information stored for a given parameter under its LOINC code, including measurement methods where applicable, can be retrieved from: https://search.loinc.orq/searchLOINC/.

The TNM stage of a tumour can be determined according to the 8th Edition of the UICC TNM classification of Malignant Tumors [retrieved on 27 March 2019] from https://www.uicc.org/8th-edition-uicc-tnm-classification-malignant-tumors-published.Measurement of parameters referred to herein, may be in any suitable unit of measurement, such as the measurement units for these parameters set out in Table 15. The unit of measurement for a given parameter is preferably consistent between the cancer patient being analysed and any training or validation data sets. Where necessary, different units of measurement can be converted to a common unit of measurement.

Parameters (i) to (xxix) above, as well as the substitute parameters referred to herein, may be measured at any suitable time point.

For example, in the context of methods comprising assessing risk of mortality of a cancer patient, such as a method of selecting a cancer patient for inclusion in a clinical trial, or for treatment with an anti-cancer therapy, the parameters may be measured prior to the start of the clinical trial or administration of the first dose of the anti-cancer therapy to the patient, respectively. Measurement shortly before, e.g. 6 months or less, 3 months or less, or 1 month or less, before the start of the clinical trial or administration of the first dose of the anti-cancer therapy to the patient.

Where the method is a method of predicting the treatment response to an anti-cancer therapy in a cancer patient, the parameters may be measure before administration of the first dose of the anti-cancer therapy to the patient. Alternatively, the parameters may be measured after administration of the anti-cancer therapy to the patient to predict the treatment response to the anti-cancer therapy during treatment. **In** one embodiment, the parameters may be measured at a first time point and a second time point, whereby the first time point may be before administration of the first dose of the anti-cancer therapy to the patient and the second time point may be after administration of the anti-cancer therapy to the patient, whereby an improvement in the predicted treatment response at the second time point compared with the first time point indicates that patient is responding to the anti-cancer therapy, and a deterioration in the predicted treatment response at the second time point compared with the first time point indicates that patient is not responding or has become resistant to the anti-cancer therapy.

Where the method comprises forming a model by performing multivariable cox regression analysis on training data, the training data may include patient information comprising parameter data for a plurality of subjects. The plurality of subjects may include at least 10000 subjects. Using a large number of subjects increases the accuracy of the model. For example, the plurality of subjects may include at least 15000, 20000, 30000, 40000, 50000, 60000, 70000, 80000, or 90000 subjects.

Forming the model may comprise assigning a respective weighting, w, to each of the respective parameters selected from the list and assigning a respective mean, m, to each of the respective parameters selected from the list for the plurality of subjects, and wherein the output of the model may be given by a sum over the selected parameters according to the formula: output = $\Sigma$ w(input - m). Thus the score may be centred on 0.

Methods of assessing risk of mortality of a cancer patient according to any of the disclosed embodiments may comprise comparing a score generated by the model to one or more predetermined threshold values, or comparing the generated score to generated scores for other cancer patients in a same group, to assess the risk of mortality. The method may, for example, comprise determining whether the generated score is above or below a predetermined threshold value or in a range of values between two different predetermined threshold values.

Methods of predicting the treatment response of a cancer patient to an anti-cancer therapy may comprise comparing a score generated by the model to one or more predetermined threshold values, or comparing the generated score to generated scores for other cancer patients in a same group, to obtain the prediction of the treatment response. The method may, for example, comprise determining whether the generated score is above or below a predetermined threshold value or in a range of values between two different predetermined threshold values.

The risk of mortality may be assessed as a high risk or a low risk. For example, a patient may be assessed as at high risk of mortality if their score is above 0, or above 1, or above 1.05. A patient may be assessed as at low risk of mortality if their score is below 0 or below - 1 or below -1.19. The risk of mortality may be assessed as very high risk if the RoPro score is above 1.13. The risk of mortality may be assessed as lower if the RoPro score is below 1.13. For an advanced NSCLC-specific RoPro score, the risk of mortality may be assessed as very high if the RoPro score is above 0.81. For an advanced melanoma-specific RoPro score, the risk of mortality may be assessed as very high if the RoPro score is above 1.06. For a bladder cancer-specific RoPro score, the risk of mortality may be assessed as very high if the RoPro score is above 0.99. For a CLL-specific RoPro score, the risk of mortality may be assessed as very high if the RoPro score is above 1.16. For a DLBCL-specific RoPro score, the risk of mortality may be assessed as very high if the RoPro score is above 1.17. For a HCC-specific RoPro score, the risk of mortality may be assessed as very high if the RoPro score is above 1.11. For a metastatic breast cancer-specific RoPro score, the risk of mortality may be assessed as very high if the RoPro score is above 1.00. For a metastatic CRC-specific RoPro score, the risk of mortality may be assessed as very high if the RoPro score is above 0.94. For a metastatic RCC-specific RoPro score, the risk of mortality may be assessed as very high if the RoPro score is above 1.22. For a multiple myeloma-specific RoPro score, the risk of mortality may be assessed as very high if the RoPro score is above 1.02. For an ovarian cancer-specific RoPro score, the risk of mortality may be assessed as very high if the RoPro score is above 1.04. For a SCLC-specific RoPro score, the risk of mortality may be assessed as very high if the RoPro score is above 0.89. For a head and neck cancer-specific RoPro score, the risk of mortality may be assessed as very high if the RoPro score is above 0.75. For a follicular cancer-specific RoPro score, the risk of mortality may be assessed as very high if the RoPro score is above 1.60. For a pancreatic cancer-specific RoPro score, the risk of mortality may be assessed as very high if the RoPro score is above 0.87.

Alternatively, the patient may be one of a group of patients and the score may be generated for each of the patients in the group. Then, a patient may be assessed as at high risk of mortality if their score is in the highest 50% or the highest 10% or the highest 5% of scores in the group. A patient may be assessed as at low risk of mortality if their score is in the lowest 50% or the lowest 10% or the lowest 5% of scores in the group. The risk of mortality of a patient may be assessed as high or low based on a comparison of the patient's score with scores of the subjects in the training data. Scores may be generated for a plurality of subjects in the training data and the patient's score may be compared with the distribution of scores in the training data. A patient may be assessed as at high risk of mortality if their score is within the range of the highest 50% or the highest 10% or the highest 5% of scores for the plurality of subjects. A patient may be assessed as at low risk of mortality if their score is within the range of the lowest 50% or the lowest 10% or the lowest 5% of scores for the plurality of subjects.

As used herein, the term ratio may refer to a scaled ratio. For example, the ratio of lymphocytes to leukocytes in blood may be the number of lymphocytes per 100 leukocytes. Similarly, the ratio of eosinophils to leukocytes in blood may be the number of eosinophils per 100 leukocytes.

A method of assessing whether a cancer patient is at high risk or low risk of mortality, may be employed in a number of different contexts.

For example, when performing clinical trials, e.g. for an anti-cancer therapy, it is advantageous to select only those patients for inclusion in the clinical trial that are likely to survive for the full length of the trial. This is beneficial both from the perspective of performing the clinical trial, as data from patients who drop out of trial as a result of mortality can in many cases not be used to evaluate the anti-cancer therapy e.g. for safety or efficacy, increasing the cost of the clinical trial and the time needed to bring the trial to conclusion. **In** addition, the inclusion of patients with a high risk of mortality in clinical trials may mask a therapeutic effect for patient with a low or lower risk of mortality, as the health of the patient at high risk of mortality is too compromised to benefit from the treatment. **In** addition, excluding patients with a high risk mortality from clinical trials benefits patients, as individuals who are unlikely to benefit from the anti-cancer therapy being tested are not exposed to unnecessary treatment, as well as any accompanying side-effects.

Thus, in one embodiment, the present invention provides a method of selecting a cancer patient for inclusion in a clinical trial, e.g. for an anti-cancer therapy, the method comprising assessing whether the cancer patient is at high risk or low risk of mortality using a method as described herein, and selecting a patient assessed to be at low risk of mortality for inclusion in the clinical trial.

For the evaluation of clinical trial results, it is important that patients in the group receiving the anti-cancer therapy being tested and patients in the group(s) receiving the placebo or no treatment, and which act as a control for the treatment group, are well matched, i.e. have the same risk of mortality as assessed using a method as described herein, to ensure that any effects seen in the treatment group are the result of the anti-cancer therapy to be tested and do not result from differences between the different patient groups. For example, if the placebo group has a significantly higher risk of mortality than the treatment group, this may incorrectly suggest that the treatment has a positive effect on patient survival, and vice versa.

**In** another embodiment, the present invention thus provides a method of evaluating the results of a clinical trial, e.g. for an anti-cancer therapy, carried out on cancer patients, the method comprising assessing whether the cancer patients taking part in the clinical trial are at high risk or low risk of mortality using a method as described herein.

In a further embodiment, the present invention relates to a method of selecting cancer patients for inclusion in a clinical trial, e.g. for an anti-cancer therapy, the method comprising identifying a first and a second cancer patient with the same risk of mortality using a method as described herein, and including said patients in the clinical trial. The first cancer patient may receive the anti-cancer therapy and the second cancer patient may not receive the anti-cancer therapy. In this case the second cancer patient acts as a control for the first cancer patient, thereby allowing e.g. the safety or efficacy of the anti-cancer therapy to be evaluated. Preferably both the first and the second patient has a low risk of mortality.

Patients may be judged to have the same risk of mortality if they are both assessed as having a low risk or are both assessed as having a high risk. Alternatively, patients may be judged as having the same risk if their scores are within the same quantile as each other. For example, a set of patients may be grouped into 10% or 5% quantiles based on their scores. Two patients within the top 5% of scores in the set may be judged to have the same risk. Two patients in the second (5-10%) quantile may be judged to have the same risk.

Disclosed but not claimed is a method of comparing a first cancer patient, or cancer patient cohort, to a second cancer patient, or cancer patient cohort, respectively, the method comprising assessing whether the patients, or the patients in the first and second cohorts are at high risk or low risk of mortality using a method as described herein.

As mentioned above, patients at high risk of mortality are unlikely to benefit from anti-cancer therapy. Identifying such patients prior to treatment is advantageous as it avoids exposing patients to therapy which will ultimately prove ineffective, as well as any side-effects associated therewith. Given that many anti-cancer therapies are also associated with high costs, the ability to identify such patients prior to treatment also reduce the cost burden on healthcare systems.

In another embodiment, the present invention therefore relates to a method of selecting a cancer patient for treatment with an anti-cancer therapy, the method comprising assessing whether the cancer patient is at high risk or low risk of mortality using a method as described herein, and selecting a cancer patient assessed to be at low risk of mortality for treatment with the anti-cancer therapy. The method may further comprise treating the cancer patient assessed to be at low risk of mortality with the anti-cancer therapy.

Disclosed but not claimed is a method of treating a cancer patient with an anti-cancer therapy, the method comprising assessing whether the cancer patient is at high risk or low risk of mortality using a method as described herein, and administering a pharmaceutically effective amount of the anti-cancer therapy to a patient assessed to be at low risk of mortality.

Disclosed but not claimed is an anti-cancer therapy for use in a method of treating a cancer patient with an anti-cancer therapy, the method comprising assessing whether the cancer patient is at high risk or low risk of mortality using a method as described herein, and administering a pharmaceutically effective amount of the anti-cancer therapy to a patient assessed to be at low risk of mortality.

Similarly, the mortality risk of a patient during cancer therapy is expected to be indicative of whether the patient is benefitting, or will benefit from the therapy.

Thus, also provided is a method of monitoring a cancer patient during treatment with an anti-cancer therapy, wherein the patient may optionally show disease progression, the method comprising assessing whether the cancer patient is at high risk or low risk of mortality using a method as described herein, wherein a cancer patient assessed to be at low risk of mortality is selected for continued treatment with the anti-cancer therapy, and a cancer patient assessed to be at high risk of mortality is selected to discontinue treatment with the anti-cancer therapy.

Parameters for predicting prognosis in specific cancer types are known in the art, as are methods for measuring or assessing such parameters. The present inventors have shown that further including data for one or more cancer type-specific parameters (such as a parameter specific for predicting prognosis in advanced non-small-cell lung carcinoma (NSCLC), bladder cancer, chronic lymphocytic leukaemia (CLL), diffuse large B-cell lymphoma (DLBCL), hepatocellular carcinoma (HCC), metastatic breast cancer, metastatic colorectal cancer (CRC), metastatic renal cell carcinoma (RCC), multiple myeloma, ovarian cancer, small cell lung cancer (SCLC), head & neck cancer, or pancreatic cancer) in the patient information when assessing e.g. the risk of mortality of a cancer patient, or predicting the treatment response to an anti-cancer therapy in a cancer patient, results in a more accurate mortality risk score for that cancer type than use parameters (i) to (xxvi) or (i) to (xxix) alone. The patient information in a method of assessing the risk of mortality of a cancer patient may thus further comprise data corresponding to one or more parameters selected from parameters known to be indicative of prognosis in advanced NSCLC, bladder cancer, CLL, DLBCL, HCC, metastatic breast cancer, metastatic CRC, metastatic RCC, multiple myeloma, ovarian cancer, SCLC, head & neck cancer, and pancreatic cancer, such as those listed in Table 15. These cancer-specific parameter(s) may be measured at the same time point or at a different time point as the other parameters employed in the method.

The cancer type-specific parameters referred to above are well known in the art, as are methods for measuring these parameters. Exemplary methods are set out in Table 15. Measuring these parameters is therefore well within the capabilities of the skilled person. For example, where the cancer type-specific parameter is a biomarker (e.g. presence or absence of ALK rearrangement, presence or absence of EGFR mutation etc.), information on the presence or absence of the biomarker is included in the patient's electronic health record (EHR). Methods for determining the presence or absence of biomarkers include: sequencing, such as next generation sequencing, fluorescent in situ hybridization (FISH), and immunohistochemistry (IHC).

The dataset from the Flatiron Health database on the basis of which the 26 parameters of the RoPro1 described above were identified consisted of 99,249 patients with one of the following cancer types: advanced melanoma, advanced non-small-cell lung cancer (NSCLC), bladder cancer, chronic lymphocytic leukaemia (CLL), diffuse large B-cell lymphoma (DLBCL), hepatocellular carcinoma (HCC), metastatic breast cancer, metastatic colorectal cancer (CRC), metastatic renal cell carcinoma (RCC), multiple myeloma, ovarian cancer, or small-cell lung carcinoma (SCLC). Furthermore, the present inventors have shown that patient information comprising data corresponding to parameters set forth in (i) to (xxvi) above could be used to predict the risk of mortality in these cancer types. Thus, a cancer, as referred to herein, may be a cancer selected from the group consisting of: melanoma (such as advanced melanoma)), NSCLC (such as advanced NSCLC), bladder cancer, CLL, DLBCL, HCC, metastatic breast cancer, metastatic CRC, metastatic RCC, multiple myeloma, ovarian cancer, and SCLC.

The dataset from the Flatiron Health database on the basis of which the 29 parameters of the RoPro 2 described above were identified consisted of 111,538 patients with one of the following cancer types: advanced melanoma, advanced NSCLC, bladder cancer, CLL, DLBCL, HCC, metastatic breast cancer, metastatic CRC, metastatic RCC, multiple myeloma, ovarian cancer, SCLC, follicular lymphoma, pancreatic cancer or head & neck cancer. Furthermore, the present inventors have shown that patient information comprising data corresponding to parameters set forth in (i) to (xxix) above could be used to predict the risk of mortality in these cancer types. Thus, a cancer, as referred to herein, may be a cancer selected from the group consisting of: melanoma (such as advanced melanoma)), NSCLC (such as advanced NSCLC), bladder cancer, CLL, DLBCL, HCC, metastatic breast cancer, metastatic CRC, metastatic RCC, multiple myeloma, ovarian cancer, SCLC, follicular lymphoma, pancreatic cancer, and head & neck cancer.

**Brief Description of the Figures**

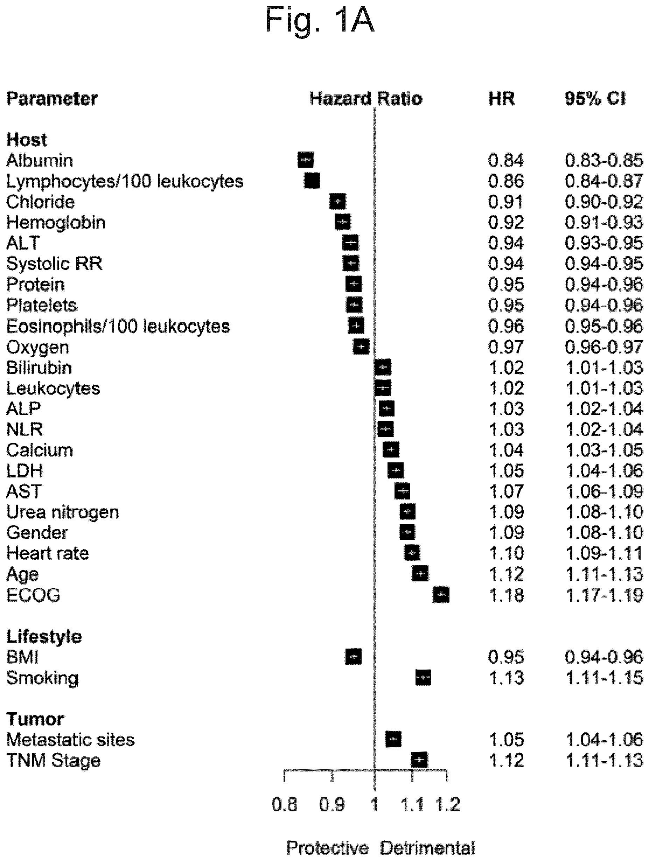

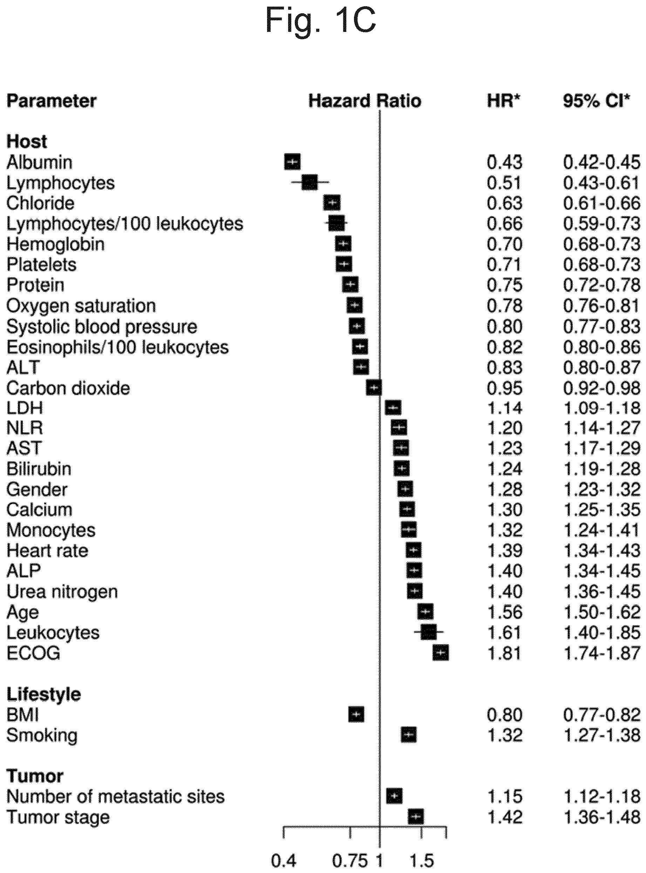

**Figure 1 A** and **C:** shows hazard ration (HR) estimates and corresponding confidence intervals (CI) (on standard normal parameter scale) for RoPro1 and RoPro2, respectively. **2 B** and **D:** shows "wordle" plots of the parameters of RoPro1 and 2, respectively: large font size corresponds to high relevance of the parameter. Parameters are shown by the parameter categories lifestyle, host and tumour. Protective and risk parameters are indicated. Protective parameters have a HR below 1, indicating that higher levels of the parameter are beneficial. Detrimental parameters have a HR above 1, and imply higher risk with higher parameter value. For parameter abbreviations see Table 20.

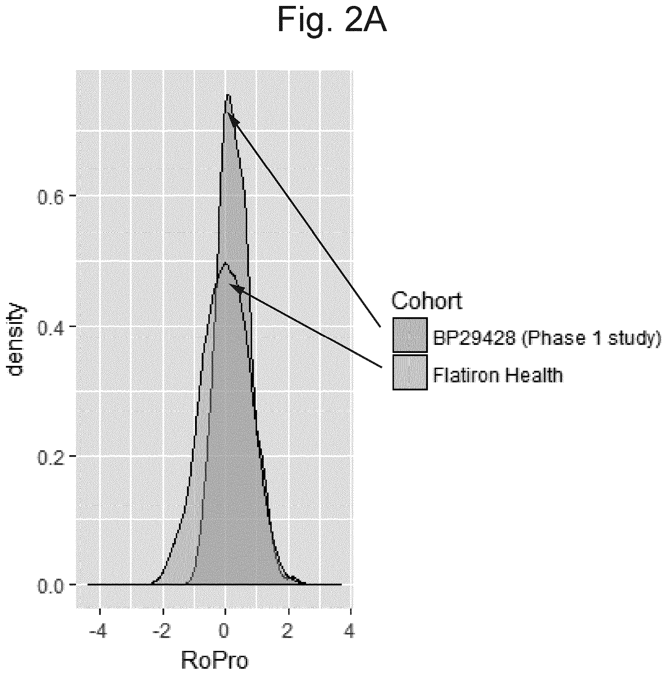

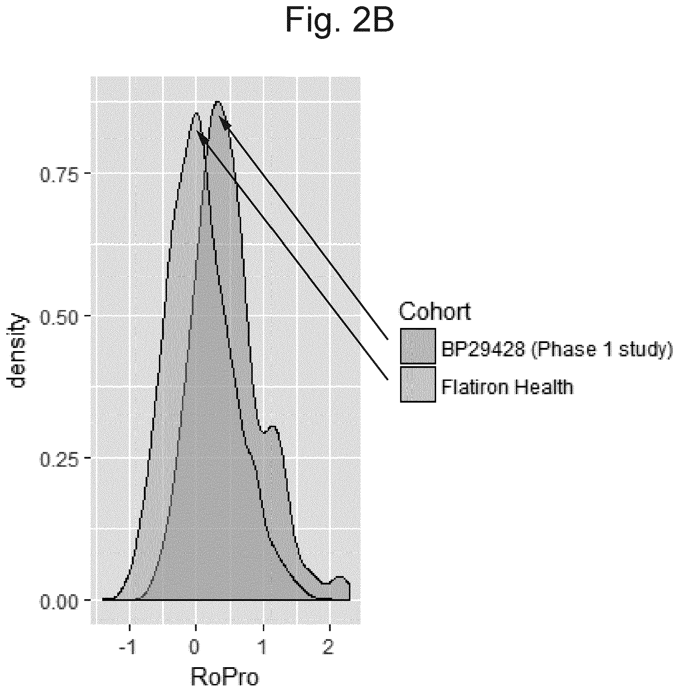

**Figure 2 A:** shows a probability density function plot of the RoPro 1 of patients in the Flatiron Health database compared to patients that took part in the clinical Phase 1 study BP29428 investigating emactuzumab and atezolizumab in solid tumour patients. The results indicate a slight shift to the right of the patient population in BP29428, indicating a higher proportion of poor prognosis patients. **Figure 2 B:** shows a probability density function plot of RoPro1 in Flatiron Health database compared to clinical Phase 1 study BP29428, both restricted to primary bladder cancer. The results indicate a pronounced shift to the right of the patient population in BP29428, indicating a higher proportion of poor prognosis patients, in this case potentially reflecting the difference in the number of prior lines of treatment.

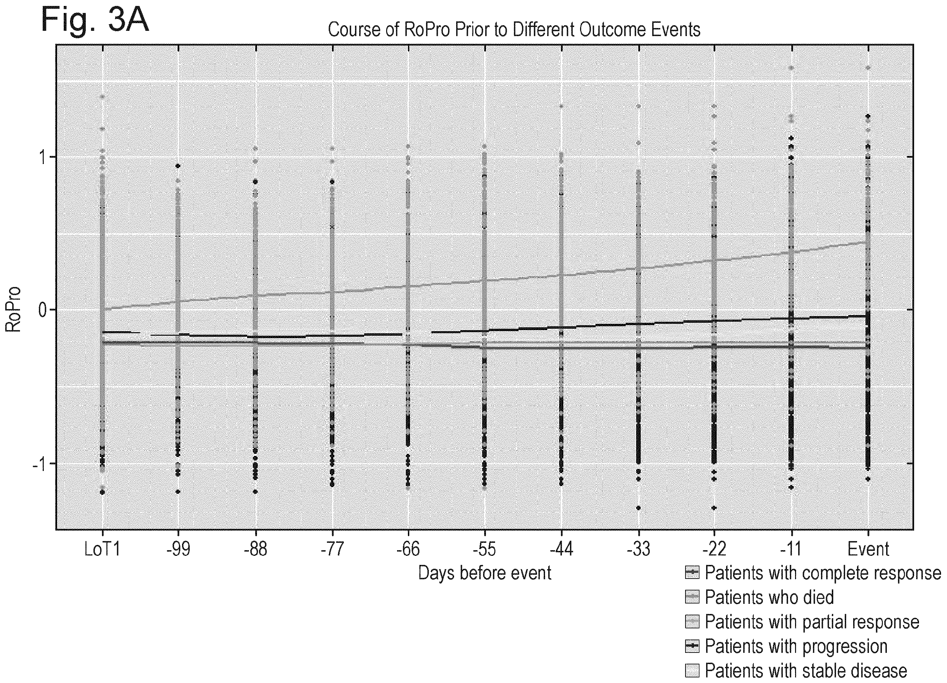

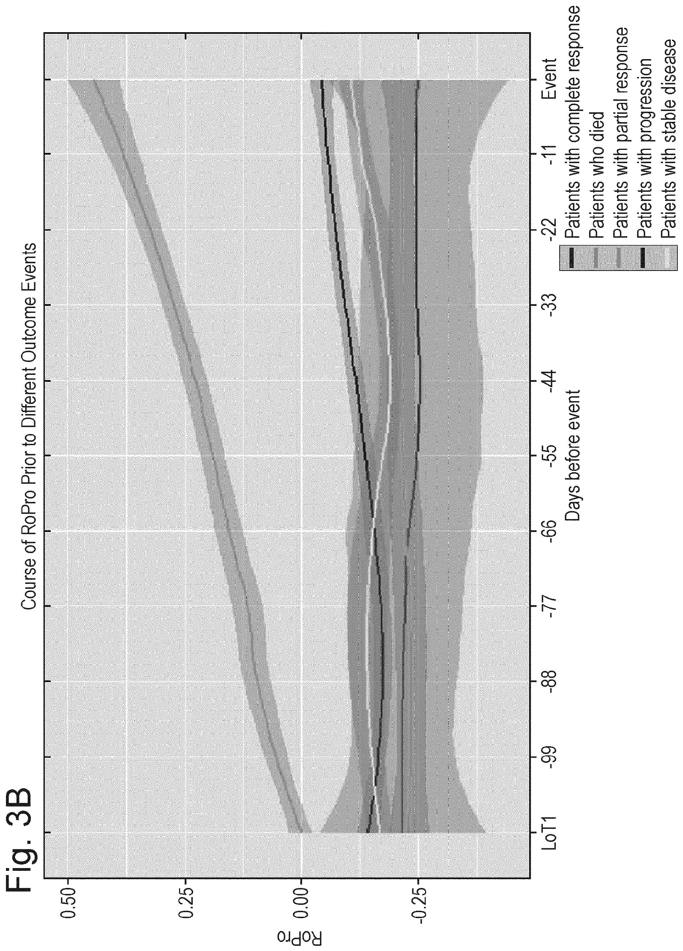

**Figure 3 A** and **B** show longitudinal monitoring of RoPro1 by response group in the OAK Phase 3 clinical study. The x-axis corresponds to different time points. Start of first line of treatment (LoT) is the leftmost point, the date of occurrence of an outcome event is the rightmost point. In between, time points prior to the event are shown, in steps of 11 days. The y-axis corresponds to the RoPro1 (group averages). Each curve represents one of 5 outcome **groups. A:** The curves represent, in order, downwards from the uppermost curve: patients who died, patients with progression, patients with stable disease, and patients with partial response, patients with complete response. **B:** The curves represent, in order, downwards from the uppermost curve at the right of the figure: patients who died, patients with progression, patients with stable disease, patients with partial response, and patients with complete response. Confidence intervals are shown as shaded bands around each curve. **Figure 3** shows that the RoPro1 correlates with treatment response.

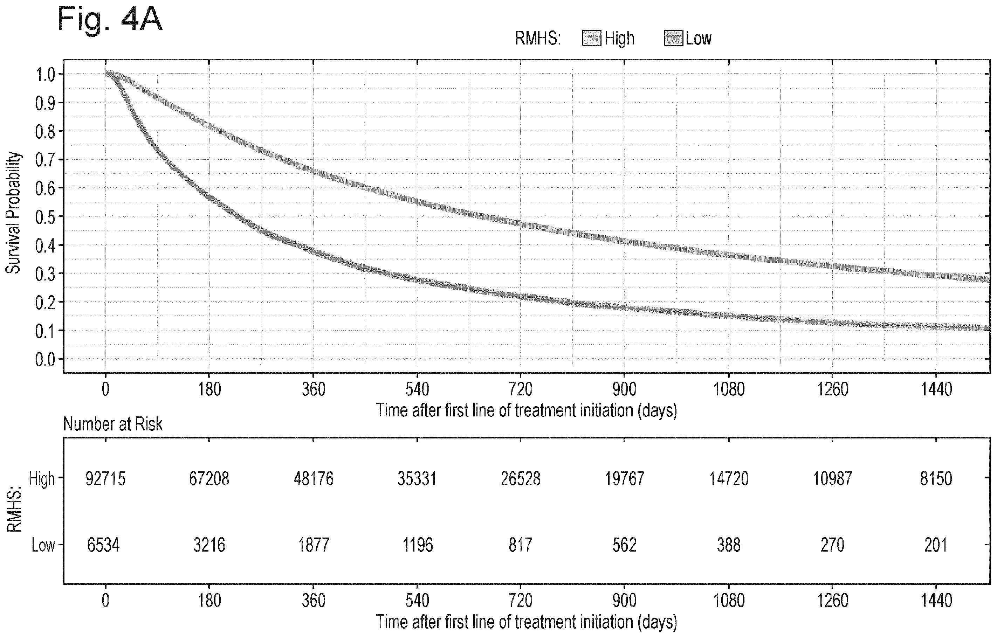

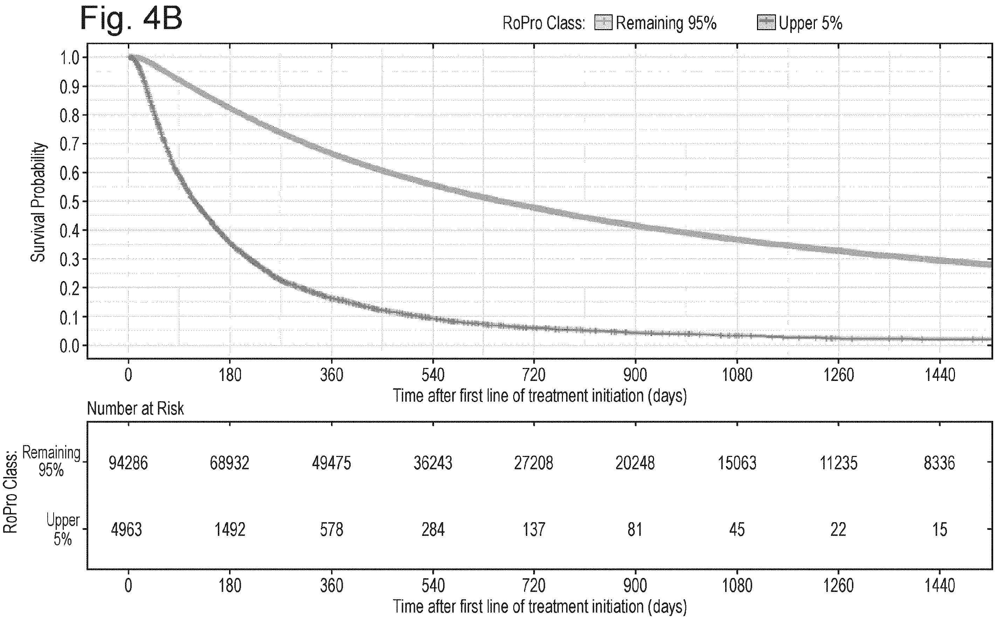

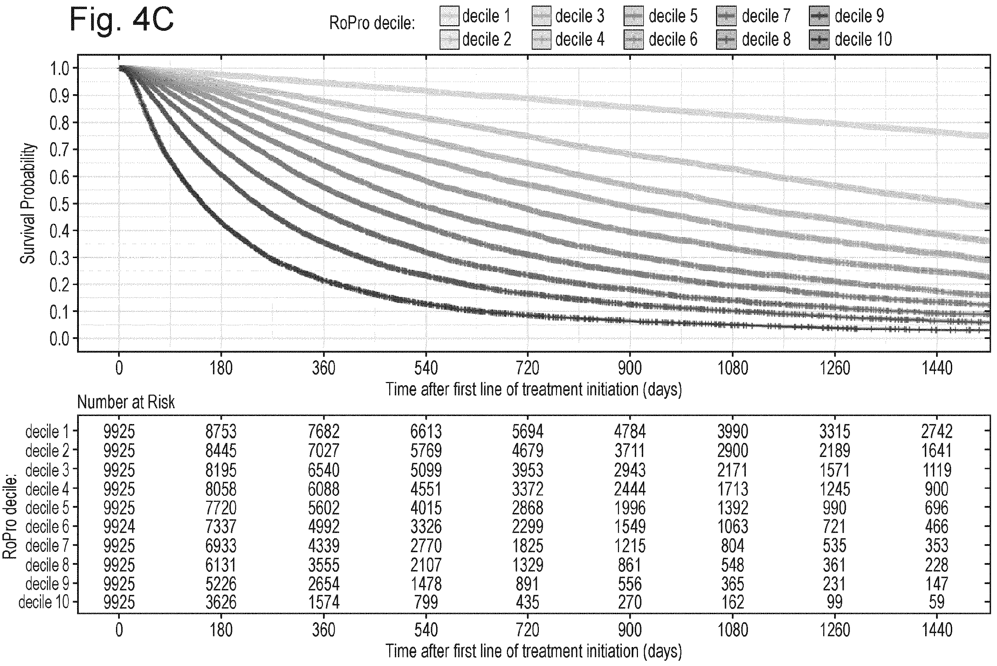

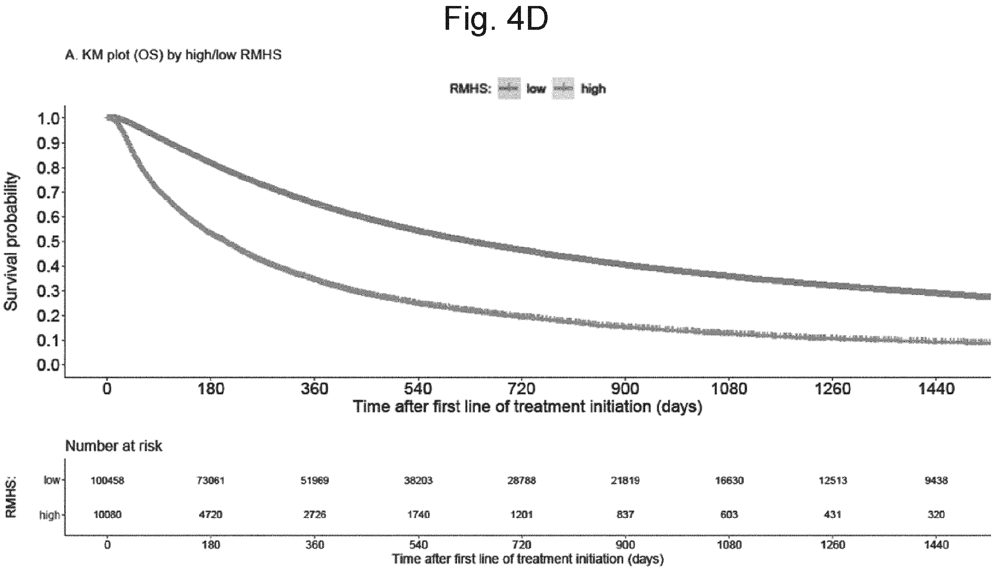

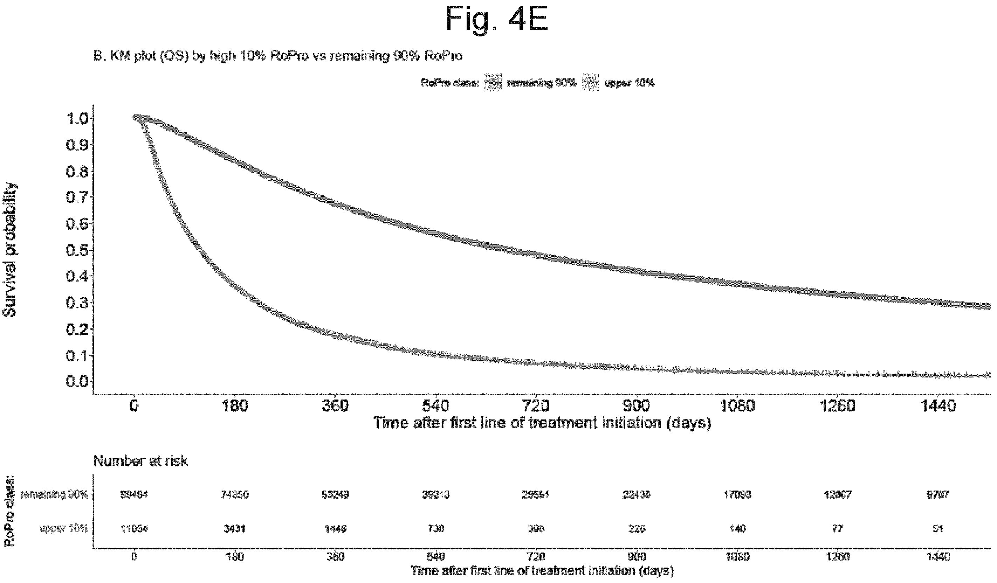

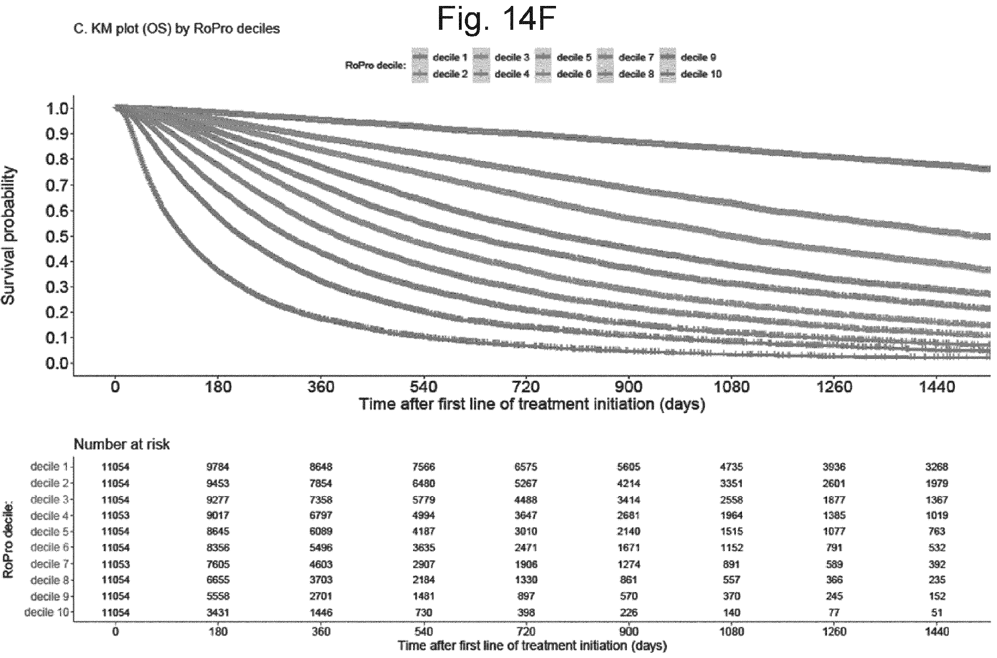

**Figure 4 A** and **D** show KM survival curves plotted for patients with high and low RMHS. The upper graph shows survival probability against time after first line of treatment initiation in days (the upper and lower curves represents patients with high RMHS and low RMHS, respectively). The lower graph indicates the number of patients with a high and low RMHS score against the time after first line of treatment initiation in days. **Figure 4 B and E** show KM survival curves plotted for patients with high and low RoPro1 and 2, respectively. The upper graph shows survival probability against time after first line of treatment initiation in days (the lower curve represents patients with upper 5% of RoPro1 or 2 while the lower curve represents the patients with the remaining 95% of RoPro1 or 2). The lower graph indicates the number of patients with a high and low RoPro1 or 2 against the time after first line of treatment initiation in days. **Figure 4 C** and **F** shows KM survival curves plotted for patients by RoPro 1 and 2 deciles, respectively. The upper graph shows survival probability against time after first line of treatment initiation in days. The curves represent, in order, downwards from the uppermost curve: deciles 1 to 10. The lower graph indicates the number of patients in each decile against the time after first line of treatment initiation in days (y-axis; from top: deciles 1 to 10).

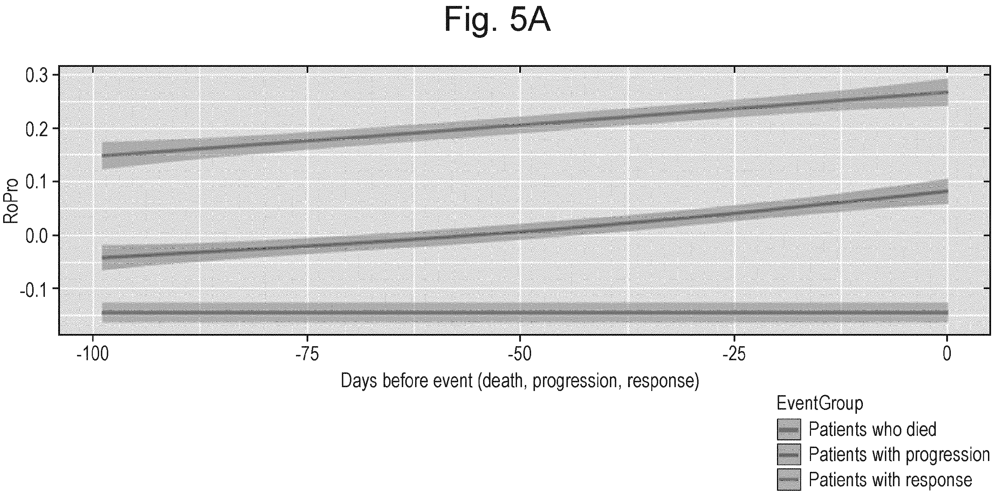

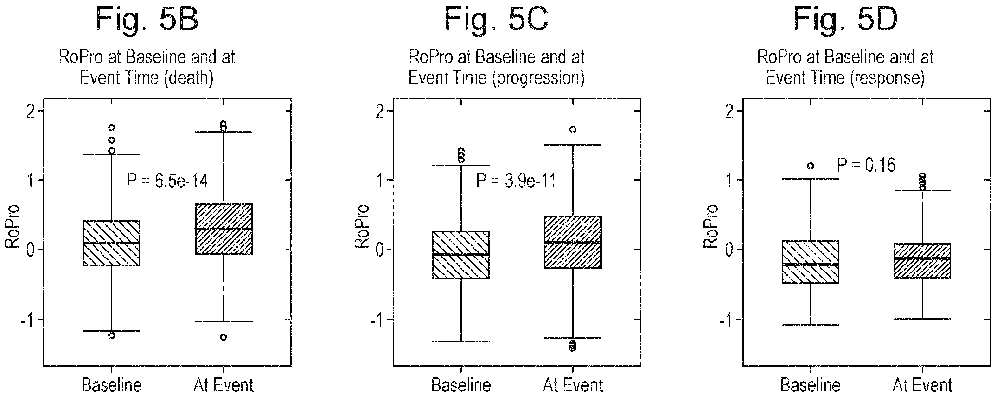

**Figure 5A-D** show the ability of the RoPro2 to discriminate event occurrence in longitudinal monitoring. **A:** shows that the RoPro2 score for patients who died (top curve), patients who progressed (middle curve) or patients with a partial or complete response (bottom curve) in the days before the respective event (death, progression, response). For patients who died, the RoPro2 score significantly worsened towards the event (P=$6.50\times10^{-14}$, **Figure 5B),** mean score 0.09 (SD 0.50) at baseline, 0.30 (SD 0.54) for last measure before death. Patients progressing also showed a significant, but less pronounced **(Figure 5C)** worsening of their RoPro2 score (P=$3.90\times10^{-11}$, Figure 5c). The RoPro2 score of partial (n=191) and complete (n=11) responders did not change significantly towards the response event **(Figure 5D).**

## Detailed Description

An exemplary method of forming a model and a score will now be described. It will be appreciated that alternative statistical techniques for analysing the contribution of the parameters to the risk of mortality may be utilised in order to form the model. The examples includes all of parameters (i) to (xxvi) or (i) to (xxix), but as discussed above, the method may be adapted to use only a selection of these parameters and/or to include parameters other than (i) to (xxvi) or (i) to (xxix), respectively.

Two examples of the model and score are now described as the Roche prognostic score (RoPro) 1 and 2. The RoPro1 is a weighted sum over the 26 parameters, (i) to (xxvi), whereas the RoPro 2 is a weighted sum over the 29 parameters, (i) to (xxix), of the difference between the patient's data and the respective reference parameter means. A higher RoPro indicates higher death hazard and a higher mortality risk and a lower RoPro indicates a lower death hazard and a lower mortality risk.

A general formula of the RoPro is $\Sigma_i \ln(HR(x_i))(m_{ij}-m_i)$, where $HR(x_i)$ is the HR estimated for parameter i, $m_i$ is the parameter mean in the Flatiron Health database data set, and $m_{ij}$ is the value of patient j at parameter i, $i \in I$. The HR weights the contribution of a parameter to the score and the subtraction of the parameter mean centers the score about zero.

An example of a general RoPro1 formula for all indications produced using this method was:
-0.0364(Albumin-38.79)+0.278(ECOG-0.78)+0.00035(LDH-273.99)-0.00921        (Lymphocytes/100leukocytes-25.83) -0.0427(Hemoglobin-12.28)+0.00034(ALP-108.93)+0.0549(NLR-0.57)-0.0265(Chloride-101.58)+0.00603(Heart rate-83.37)       +0.00373(AST-25.98)+0.0103(Age-66.36)+0.0112(Urea        nitrogen-17.33)-0.0162(Oxygen-96.42) +0.109(TNM       stage-2.98)       -0.0067(Protein-69.7)-0.00296(Systolic       RR-129.58)-0.0302(Eosinophils/1001eukocytes-2.32)+0.0621(Bilirubin-0.51)       +0.07228(Calcium-9.37)+0.16482(Sex-0.48)-0.0075(BMI-28.25)+0.256(Smoking-0.36)-0.00048(Platelets-272.01)+0.0531(No.  Of  metastatic  sites-0.44)-0.00334(ALT-24.63)+0.00082(Leukocytes-12.78).

An example of a general RoPro2 formula for all indications produced using this method was:
0.01012(age-66.695)+0.12264(sex-0.502)+0.20044(smoking-0.581)+0.06476(no.  of  metastatic  sites-0.163) +0.23399(ECOG-0.834)+0.09786(NLR-0.583)-0.00801(BMI-27.838)-0.04095(oxygen-96.607)-0.00303(systolicRR-128.707)+0.00521(heart  rate-83.246)-0.04637(Hgb_T0-12.092)+0.00927(leukocytes-11.077)+0.01108(urea  nitrogen-17.53)+0.11564(calcium-9.314)-0.00078(platelets-272.168)-0.00623(lymphocyte-leukocyte-ratio-23.786) +0.00285(AST-26.729)+0.00117(ALP-111.233)-0.00978(protein-69.034)-0.00252(ALT-25.152)-0.04085(albumin-37.798)+0.17365(bilirubin-0.523)-0.01713(lymphoctes-3.683)-0.00467(carbon       dioxide-25.768)-0.02951(chloride-101.369)+0.1176(monocytes-0.729)-0.03171(eosinophils-leukocytes-ratio-2.225)+0.00022(LDH-286.624) +0.08136(tumor stage-3.101).

Descriptions of the parameters in the formula above and examples of how they may be measured and valued in the RoPro model are shown in the Table 15. It will be appreciated that in other embodiments, the parameters may be measured in any other suitable way and that different units may be used. Values assigned to non-numerical parameters, such as gender and smoking history, can be freely selected as long as they are consistently used in the training data and the data input to a score for a patient. **In** the example illustrated by the formula above, female is given a value of 0 and male is given a value of 1. However, an equivalent score will be generated for a patient if, throughout the training data and the patient data, female is given a value of 1 and male is given a value of 0 as the weighting value $(\ln(HR(x_i)))$ generated would have the opposite sign and so the contribution of this parameter to the score would remain the same. The terms gender and sex are used interchangeably herein. This principle also applies to all parameters which may be numerically scaled without impacting the score produced, as long as such scaling is consistent over the training data and patient input data.

In the above formula, ECOG levels are input directly to the model. ECOG levels of 0, 1, 2, 3, and 4 were used. Subjects with an ECOG value of 5 were not included in the training data. As shown in the formula, the mean value in the training data for ECOG level was 0.78.

Further, specific RoPro formulas for each cancer indication, by re-estimation of the weights $\ln(HR(x_i))$ in the specific cohort can be produced using data from subjects with the specific cancer indication. For validation purposes explained below, the general score was applied, without re-estimation of the parameter weights, in independent clinical studies.

To compute a patient's score, the patient's measurements for the 26 or 29 parameters (Albumin, ECOG, LDH, etc.) are entered into the formula. The measured value for each parameter for the patient is inserted into the formula in place of the respective parameter label. Patient RoPro1 for patients in the Flatiron Health database that were used in producing the score ranged from -4.06 to 3.72, with 99% lying in (-2.12;2.00). The Flatiron Health database is discussed in more detail later. The values used for each parameter are discussed in Table 15. For example, for the parameter of "Gender", a value of 1 is given if the patient is male and a value of 0 is given if the patient is female.

The 26 factors (i) to (xxvi), and similarly the 29 factors (i) to (xxix), contribute independently to a quantitative prognostic risk score, the "RoPro".

The RoPro1 and 2 were validated in two independent clinical studies, Phase 1 and 3. Here, strong association with patients' early study-drop out (within less than 3 days), progression-free and overall survival was found. Changes in RoPro over time were indicative of subsequent progression and death.

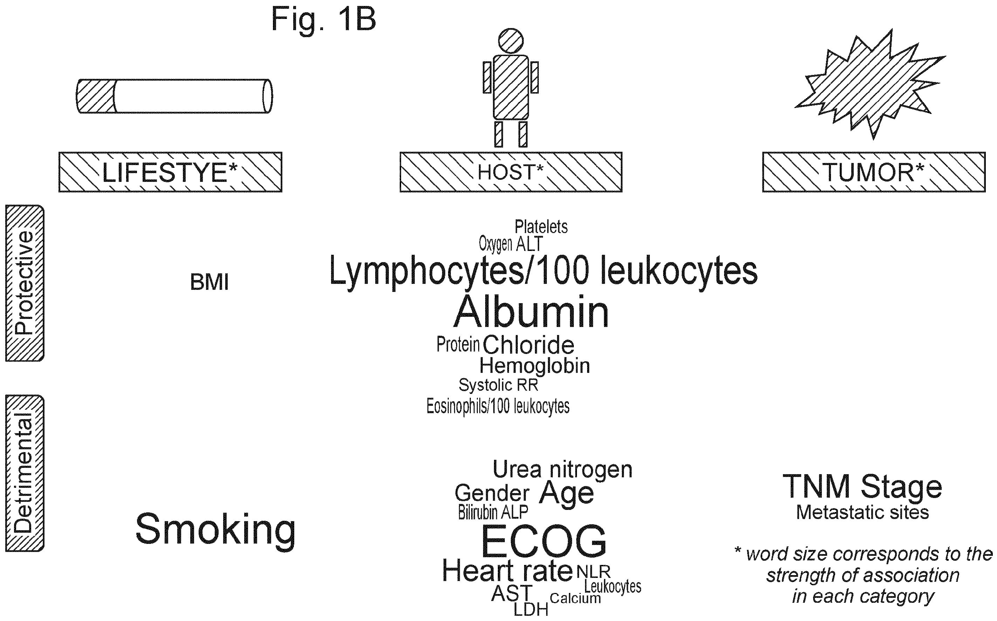

In the development of RoPro1, the inventors found 39 parameters significantly associated with overall survival (OS), whereof parameters (i) to (xxvi) contributed independently in multivariable modelling (table 1A, figure 1A and B). The resulting model correlated with time-to-death ($r^2$=0.24), strongly outperforming the RMHS ($r^2$=0.02 on the same data).

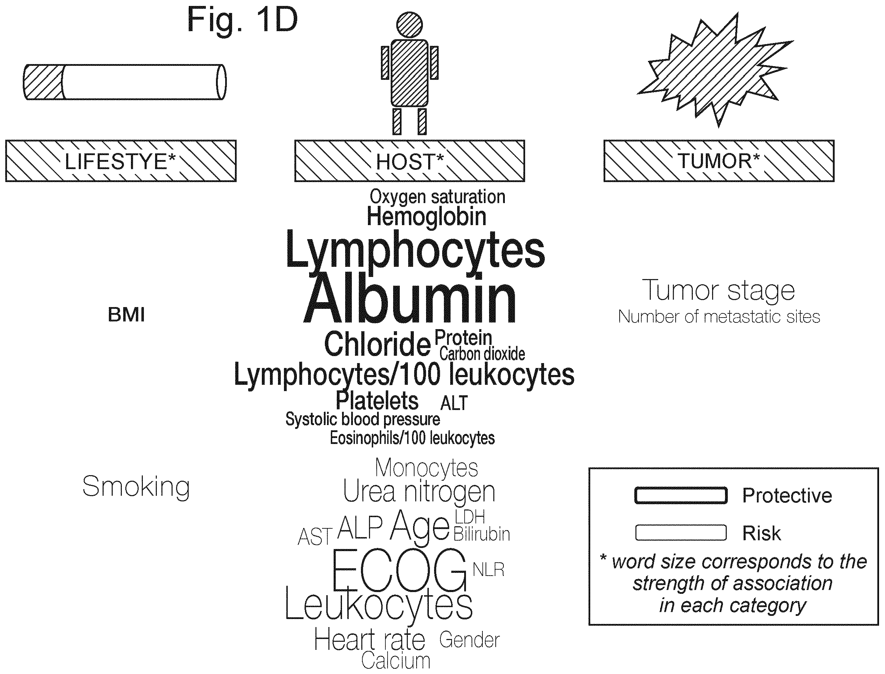

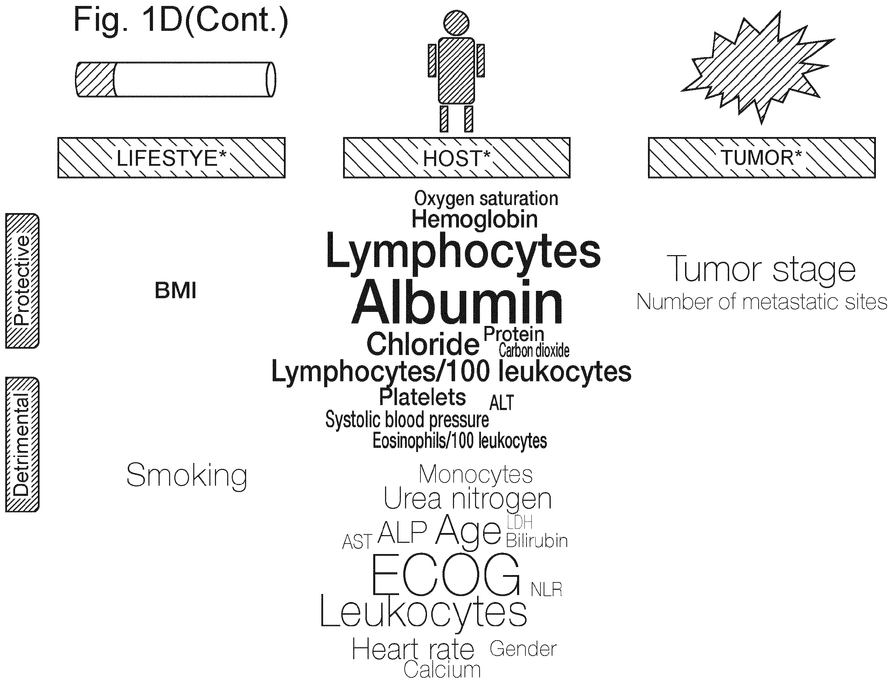

In the development of RoPro2, the inventors similarly found that parameters (i) to (xxix) contributed independently in multivariable modelling (table 1B, figure 1C and D). The resulting model correlated with time-to-death ($r^2$=0. 30), outperforming the RMHS ($r^2$=0.02 on the same data) even more strongly than RoPro1.

**Table 1A:** Final RoPro1 cox regression model (OS) and description of RoPro parameters.

| | Cox regression results | | Parameter description | | | | |
|---|---|---|---|---|---|---|---|
| Parameter | HR* | p-value | tailHR** | units | 2.5%-quantile | median | 97.5%-quantile |
| Albumin | 0.84 [0.83; 0.85] | 2.95E-201 | 0.538 | g/L | 28.00 | 39.33 | 45.00 |
| ECOG | 1.32 [1.30; 1.34] | 2.77E-298 | 1.745 | none | 0.00 | 0.784+ | 2.00 |
| LDH | 1.06 [1.05; 1.07] | 3.10E-28 | 1.149 | U/L | 134.00 | 230.07 | 532.00 |
| Lymphocytes/100 leukocytes | 0.86 [0.84; 0.87] | 2.85E-54 | 0.540 | % | 6.00 | 22.00 | 73.00 |
| Haemoglobin | 0.92 [0.91; 0.93] | 1.07E-51 | 0.751 | g/dL | 8.40 | 12.40 | 15.10 |
| ALP | 1.03 [1.02; 1.04] | 7.64E-12 | 1.062 | U/L | 46.00 | 89.00 | 221.00 |
| NLR | 1.03 [1.02; 1.04] | 6.84E-06 | 1.056 | none | 0 | 0.572+ | 1 |
| Chloride | 0.91 [0.90; 0.921 | 3.03E-85 | 0.690 | mmol/L | 93.00 | 102.00 | 107.00 |
| Heart rate | 1.10 [1.09; 1.11] | 6.04E-95 | 1.394 | bpm | 57.00 | 82.00 | 112.00 |
| AST | 1.07 [1.06; 1.09] | 1.14E-26 | 1.187 | U/L | 10.00 | 21.00 | 56.00 |
| Age | 1.12 [1.11; 1.13] | 2.07E-107 | 1.519 | none | 41.31 | 67.56 | 81.77 |
| Urea nitrogen | 1.09 [1.08; 1.10] | 5.05E-67 | 1.309 | mg/dL | 7 | 16.00 | 31 |
| Oxygen | 0.97 [0.96; 0.97] | 2.36E-21 | 0.893 | % | 92.00 | 96.71 | 99.00 |

(continued)

| | Cox regression results | | Parameter description | | | | |
|---|---|---|---|---|---|---|---|
| Parameter | HR* | p-value | tailHR** | units | 2.5%-quantile | median | 97.5%-quantile |
| TNM Stage | 1.12 [1.11; 1.13] | 3.97E-89 | 1.426 | none | 0.75 | 3.25 | 4.00 |
| Protein | 0.95 [0.94; 0.96] | 1.41E-21 | 0.851 | g/L | 57.00 | 69.00 | 81.00 |
| Systolic blood pressure | 0.94 [0.94; 0.95] | 1.92E-34 | 0.823 | mmHg | 96.00 | 129.00 | 162.00 |
| Eosinophils/100 leukocytes | 0.96 [0.95; 0.96] | 5.25E-22 | 0.860 | % | 0.00 | 2.19 | 5.00 |
| Bilirubin | 1.02 [1.01; 1.03] | 1.05E-05 | 1.051 | mg/dL | 0.20 | 0.44 | 1.00 |
| Calcium | 1.04 [1.03; 1.05] | 9.24E-18 | 1.147 | mg/dL | 8.30 | 9.38 | 10.20 |
| Sex | 1.18 [1.16; 1.20] | 5.54E-63 | 1.179 | none | 0 | 0.483[++] | 1 |
| BMI | 0.95 [0.94; 0.96] | 3.07E-31 | 0.840 | $kg/m^2$ | 17.89 | 27.28 | 41.20 |
| Smoking | 1.29 [1.25; 1.33] | 5.63E-47 | 1.291 | none | 0 | 0.360[+++] | 1 |
| Platelets | 0.95 [0.94; 0.96] | 8.96E-24 | 0.836 | $10^9$/L | 88 | 261.89 | 462 |
| Number of metastatic sites | 1.05 [1.04; 1.06] | 1.71E-27 | 1.112 | none | 0.00 | 0.00 | 2.00 |
| ALT | 0.93 [0.93; 0.94] | 1.09E-21 | 0.846 | U/L | 7.00 | 20.00 | 57.00 |
| Leukocytes | 1.02 [1.00; 1.06] | 2.59E-03 | 1.023 | $10^9$/L | 3.40 | 7.91 | 31.20 |

\* For continuous variables: Hazard ratio (HR) per 1 standard deviation (SD) on normal scale, i.e. estimated on standard-normal transformed parameter.

\*\* tailHR: HR of patient with a particular high parameter value (equal to that of the 97.5%-quantile) as compared to a person with a particular low value (equal to the 2.5%-quantile). Adjusted for other model parameters.

+ mean value

++ 48.3% of patients are male

+++ 36.0% of patients have a confirmed history of smoking

**Table 1B:** Final RoPro2 cox regression model (OS) and description of RoPro parameters.

| Parameter | Cox regression results | | Parameter description | | | |
|---|---|---|---|---|---|---|
| | p-value | HR[1] | 4SD-HR[2] | mean[3] | sd[4] | unit |
| age | 1,9E-115 | 1.01 [1.009;1.011] | 1,56 | 66,69 | 10,96 | years |
| sex | 4,4E-41 | 1.13 [1.11;1.151] | 1,28 | 0,50 | 0,50 | none[5] |
| smoking | 4,5E-37 | 1.222 [1.185;1.26] | 1,32 | 0,58 | 0,35 | none[6] |
| no. of metastatic sites | 5,1E-24 | 1.067 [1.054;1.08] | 1,15 | 0,16 | 0,54 | none |
| ECOG | 1,3E-233 | 1.264 [1.246;1.282] | 1,81 | 0,83 | 0,63 | none |
| NLR | 8,9E-12 | 1.103 [1.072;1.134] | 1,20 | 0,58 | 0,47 | none[7] |
| BMI | 5,2E-43 | 0.992 [0.991;0.993] | 0,80 | 27,84 | 7,15 | kg/m[2] |
| oxygen | 5,2E-43 | 0.96 [0.954;0.965] | 0,78 | 96,61 | 1,49 | % |
| systolic RR | 4,5E-40 | 0.997 [0.997;0.997] | 0,80 | 128,71 | 18,45 | mmHg |
| heart rate | 3,3E-80 | 1.005 [1.005;1.006] | 1,39 | 83,25 | 15,71 | bpm |
| Hgb | 2,0E-72 | 0.955 [0.95;0.96] | 0,70 | 12,09 | 1,91 | g/dL |
| leukocytes | 1,5E-11 | 1.009 [1.007;1.012] | 1,61 | 11,08 | 12,83 | 10*9/L |
| urea nitrogen | 4,4E-81 | 1.011 [1.01;1.012] | 1,40 | 17,53 | 7,66 | mg/dL |
| calcium | 3,3E-44 | 1.123 [1.104;1.141] | 1,30 | 9,31 | 0,57 | mg/dL |
| platelets | 4,5E-70 | 0.999 [0.999;0.999] | 0,71 | 272,17 | 111,15 | 10*9/L |
| lymphocyte-leukocyte-ratio | 1,7E-16 | 0.994 [0.992;0.995] | 0,66 | 23,79 | 16,84 | % |
| AST | 2,8E-15 | 1.003 [1.002;1.004] | 1,23 | 26,73 | 18,16 | U/L |
| ALP | 6,4E-65 | 1.001 [1.001;1.001] | 1,40 | 111,23 | 71,67 | U/L |
| protein | 9,5E-49 | 0.99 [0.989;0.992] | 0,75 | 69,03 | 7,30 | g/L |
| ALT | 2,8E-15 | 0.997 [0.997;0.998] | 0,83 | 25,15 | 17,99 | U/L |
| albumin | <2.2*E-308 | 0.96 [0.958;0.962] | 0,43 | 37,80 | 5,10 | g/L |
| bilirubin | 2,5E-32 | 1.19 [1.156;1.224] | 1,24 | 0,52 | 0,31 | mg/dL |
| lymphocytes | 2,9E-14 | 0.983 [0.979;0.987] | 0,51 | 3,68 | 9,73 | 10*9/L |
| carbon dioxide | 7,1E-04 | 0.995 [0.993;0.998] | 0,95 | 25,77 | 3,01 | mmol/L |
| chloride | 4,6E-144 | 0.971 [0.969;0.973] | 0,63 | 101,37 | 3,87 | mmol/L |
| monocytes | 1,6E-18 | 1.125 [1.096;1.155] | 1,32 | 0,73 | 0,60 | 10*9/L |
| eosinophils-leukocytes-ratio | 7,9E-26 | 0.969 [0.963;0.975] | 0,82 | 2,22 | 1,52 | % |
| LDH | 3,6E-10 | 1.0002 [1.0002;1.0003] | 1,14 | 286,62 | 143,18 | U/L |
| tumor stage | 2,7E-64 | 1.085 [1.075;1.095] | 1,42 | 3,10 | 1,08 | none[8] |

[1] Hazard ratio on scale of variable (= per measurement unit), together with 95% confidence interval
[2] Hazard ratio of patient with parameter value of mean+2SD compared to patient with value of mean-2SD
[3] Mean value in Flatiron Health data
[4] Standard deviation in Flatiron Health data
[5] Coded 1=male, 0=female
[6] Coded 1=History of smoking, 0= No history or unknown
[7] Coded as 1 if NLR>3, 0 if NLR <=3
[8] Coding details can be found in online supplementary table 1

The RoPro1 and 2 and the RMHS were calculated for each subject in the data used to form the RoPro1 and 2 models, respectively. Figures 4A and 4B show survival curves plotted for RoPro1 and RMHS. There is a clear separation of survival curves according to low/high RMHS in Figure 4A, HR 2.22 (2.15;2.28). The plot of RoPro1 in Figure 4B shows better separation of survival curves (HR 4.72 (4.57;4.87), indicating that high RoPro1 correlates stronger with time-to-death than high RMHS. Moreover, survival curves can be shown at fine granularity: the sample can be divided into 10 subgroups of equal size but increasing RoPro1 (10%-quantiles). There is a clear separation of the respective Kaplan-Meyer curves i.e., with the RoPro1 it is not only possible to dissect high and low risk patients, but to specify well-distinguishable grades of risk of death. Median survival was clearly separated along the quantiles, with a median survival of 2286 days in the lowest quantile compared to 147 days in the highest quantile. Patients from the highest-risk group (RoPro>1.05) had HR of 20.48 (19.47-21.55), $P<2.23 \times 10^{-308}$, compared to the lowest-risk group (RoPro<-1.19).

RoPro1 and RMHS were applied to each cohort of the data in the Flatiron Health database separately. In all cohorts, the general RoPro1 strongly outperformed the RMHS with respect to prognosis of time-to-death. Strongest performance improvement with cohort-specific RoPro1 was seen for CLL ($r^2=0.11$ for general RoPro1, $r^2=0.17$ for CLL-specific RoPro1. For metastatic breast cancer, we obtained particular strong improvement of correlation with time-to-death (from $r^2=0.10$ to $r^2=0.17$) by including hormone receptor status, HER2neu status and granulocyte/leukocyte ratio.

The RoPro1 score performed well when applied to independent data sets (sets that do not originate from the Flatiron Health database). Advanced non-small cell lung cancer patients with elevated RoPro1 (upper 10%) had a 6.32-fold (95% CI 5.95-6.73) increased death hazard compared with patients with low RoPro1 (lower 10%) ($P<2.23 \times 10-308$).

The RoPro1 demonstrates strong correlation ($r^2=0.155-0.239$, cohort dependent) with time-to-death, a profound improvement compared with established prognostic scores such as the Royal Marsden Hospital Score (RMHS) ($r^2=0.001-0.033$, cohort dependent).

Individual patient RoPro2 scores (derived by inputting their measurements for each of the 29 variables into the formula) ranged from -3.22 to 3.61, with 99% ranging between -2.33 and 2.22. Higher scores indicate a poorer prognosis for OS.

Figure 4D, E and F presents a comparison of prognostic risk scores based on RMHS or RoPro2. Figure 4D shows a clear separation of survival curves according to high/low RMHS (HR 2.37; 95% CI 2.32-2.43). Figure 4E depicts patients with the highest 10% RoPro2 scores versus the remaining 90%. This analysis according to RoPro2 showed better separation of the survival curves (HR 4.66; 95% CI 4.56-4.77) than for RMHS, indicating that high RoPro2 scores correlated more strongly with time to death than did high RMHS. Moreover, subdividing the sample into ten subgroups of equal size but increasing RoPro2 scores (deciles) showed clear separation of the respective Kaplan-Meier survival curves, reflecting poorer OS in higher-risk subgroups (Figure 4F). Median survival was clearly separated along the deciles, with a median survival of 2,975 days in the lowest versus 118 days in the highest decile. The highest-risk patients (RoPro2 score>1.13) had an HR of 25.79 (95% CI 24.58-27.06), $P<2.23 \times 10-308$, compared with the lowest-risk group (score< -1.26).

When RoPro2 and RMHS are applied to cohorts separately, the general RoPro2 formula again strongly outperforms the RMHS with respect to all performance measures in all cohorts.

Further examples of the results of the validation of the RoPro1 and 2 on data from clinical trials is explained below. In validation analyses in these two independent clinical studies, the score demonstrated strong correlation with early study drop-out (within 3 days), PFS and OS.

*Application to Phase 1 study*

Thorough understanding of patient populations in early oncology clinical trials is important for conclusive data interpretation and development decisions. The RoPro1 and 2 were applied retrospectively to patients of a Phase 1 first-in-human study BP29428 (NCT02323191), which investigated the safety, pharmacokinetics, and preliminary anti-tumour activity of emactuzumab and atezolizumab administered in combination in participants with selected locally advanced or metastatic solid tumours that were not amenable to standard treatment.

The RoPro1 and 2 were used to give patients in the BP29428 study a RoPro1 and RoPro2 value, respectively, and the RoPro1 and RoPro2 value for each patient was compared with the outcome of the patient on the study, to determine the accuracy of the RoPro1 and RoPro2 for determining prognosis of a patient.

The RoPro1 of patients in the Flatiron Health data set and patients that took part in BP29428 was approximately normally distributed with a mean around 0 (Figure 2A). The distribution of the RoPro1 of patients that took part in BP29428 tended to extend more to the right, indicating an increased portion of particular high-risk patients in BP29428 (Figure 2A).

Patients with high RoPro1 have a higher risk of death, HR=4.99 (2.59;9.63), $P=1.5 \times 10^{-6}$, providing improved discrimination vs. RMHS, HR=3.38 (1.79;6.38), $P=1.72 \times 10^{-4}$). Moreover, the RoPro1 could be used to identify early study drop-out. Patients with highest 5% RoPro1 (n=11) remained on average for only 3 days on study (Table 16), while such discrimination was not possible with the RMHS. As shown in Tables 4 A and B, the RoPro1 quantiles more effectively indicate length of time on the study than the RMHS.

In BP29428, primary bladder cancer diagnosis constitutes the largest subgroup (n=62). The subgroup has an elevated portion of advanced patients with on average 1.76 (+/-1.00) prior treatment lines, in contrast to the bladder cohort in the Flatiron Health database used to build the RoPro1 (a mixture of all stages). Accordingly, the RoPro1 distribution of BP29428 is shifted to the right (Figure 2B), i.e. there are more patients with high RoPro in the BP29428 bladder subgroup. Nevertheless, the RoPro1 was strongly associated with OS (P=$4.86 \times 10^{-7}$) also in this subgroup. Moreover, all patients from the highest RoPro1 10%-quantile (n=6) remained only 1 day on study (Table 17), demonstrating that the RoPro1 is useful even if the distribution of the number of prior lines of treatment deviates from the Flatiron Health discovery data.

The correlation of RoPro2 with OS was replicated in phase I study BP29428 (n=217, P=4.56x10-14, $r^2$=0.22, C-index=0.80).

Patients with RoPro2>1.13 (cut-off equal to 90%-quantile in Flatiron Health, n=11) had a poorer OS prognosis (HR 16.36; 95% CI 7.95-33.66), providing improved discrimination versus RMHS (HR 3.38; 95% CI 1.79-6.38).

An example of the application potential of the RoPro2 is its ability to indicate early study dropout. Patients with RoPro2>1.13 (n=11, see cut-off definition above) all left the study early (average time on study 29 days), either due to progressive disease or death. Only one patient stayed on the study until the second treatment cycle. Of note, adhering to the study protocol, all patients had an ECOG of 1. A comparable level of discrimination is not possible using RMHS (see Table 18).

*Application to Phase 3 study*

Retrospective analysis was performed on phase 3 study results to assess the impact of using the RoPro1 and 2 as exclusion criteria and to investigate whether changes of the RoPro1 and 2 over time are indicative of subsequent events.

The OAK phase III study (Rittmeyer A, et al., 2017) (NCT02008227) evaluated efficacy and safety of atezolizumab compared with docetaxel in participants (n=1187) with locally advanced or metastatic NSCLC after failure of platinum-containing chemotherapy.

The RoPro1 and 2 were used to give patients in the NCT02008227 study a RoPro1 or 2 value, respectively, and the RoPro1 and 2 value for each patient was compared with the outcome of the patient on the study, to determine the accuracy of the RoPro1 and 2 for determining prognosis of a patient.

Again, the RoPro1 ($r^2$=0.20, p=$1.09 \times 10^{-59}$) strongly outperformed RMHS ($r^2$=0.06, p=$2.80 \times 10^{-18}$) in prognosis of time-to-death (Table 2). In addition, the RoPro1 was also associated with progression-free survival ($r^2$=0.06, p=$1.06 \times 10^{-17}$).

We assessed the potential impact of using the prognostic score on comparison of OS between the treatment arms (Table 2). In unadjusted analysis, we observed a HR of 0.794 (0.690;0.913) for atezolizumab versus control, in accordance with published results (Graf E *et al.,* 1999). Using the RoPro1 as covariate, the effect estimate improved to 0.780 (0.678;0.898), while adjustment with RMHS slightly weakened the signal (0.801 [0.692;0.922]). An improvement in HR was also observed by excluding patients from the highest 10% RoPro1 (0.766 [0.659;0.891]). Despite the loss of sample size, we obtained improved significance (P=0.0006) compared to analysis of the entire data (P=0.0012).

Table 2: Impact on HR estimation using the prognostic scores in a posteriori analysis of the OAK Phase 3 clinical study

| Model performance of RoPro and RMHS | | | |
|---|---|---|---|
| score | Hazard Ratio | p-value | model-rSq[1] |
| RMHS | 1.98 [1.70;2.31] | 2.80E-18 | 0.056 |
| RoPro | 3.61 [3.09;4.21] | 1.09E-59 | 0.203 |
| Effect of treatment arm | | | |
| term | Hazard Ratio | p-value | model-rSq |
| Atezolizumab | 0.794 [0.69;0.913] | 0.0012 | 0.009 |
| Effect of treatment arm, adjusted for RMHS | | | |
| term | Hazard Ratio | p-value | model-rSq |
| Atezolizumab | 0.801 [1.696;0.922] | 0.0019 | 0.064 |
| RMHS | 1.973 [1.692;2.30] | 4.59E-18 | |
| Effect of treatment arm, adjusted for RoPro | | | |
| term | Hazard Ratio | p-value | model-rSq |
| Atezolizumab | 0.78 [0.678;0.898] | 0.0005 | 0.211 |

(continued)

| Effect of treatment arm, adjusted for RoPro | | | |
|---|---|---|---|
| term | Hazard Ratio | p-value | model-rSq |
| RoPro | 3.65 [3.125;4.264] | 6.08E-60 | |
| Effect of treatment arm, 10% high FI score excluded | | | |
| term | Hazard Ratio | p-value | model-rSq |
| Atezolizumab | 0.766 [0.659;0.891] | 0.0006 | 0.011 |
| Effect of treatment arm, random exclusion of 10% of patients[2] | | | |
| term | Hazard Ratio | p-value | model-rSq |
| Atezolizumab | 0.794 [0.685;0.921] | 0.0023 | 0.009 |
| [1]Correlation with time-to-death (rSq (r-square; $r^2$) from cox regression).<br>[2]Median of 999 replicated data sets | | | |

The correlation of the RoPro2 with OS survival was replicated in the phase III study OAK (n=1,187, P=3.65x10-56, $r^2$=0.19, C-index=0.68). Patients with RoPro2>0.81 (cut-off equal to 90%-quantile in Flatiron Health for dedicated advanced NSCLC RoPro2, n=76) had poorer OS prognosis (HR 3.62; 95% CI 2.82-4.65), again providing improved discrimination versus RMHS (HR 1.97; 95% CI 1.79-2.31). Area under the curve values again outperformed RMHS. Patients from the high RoPro2 class stayed on average only 4.8 month (median 2.4 month) on the study (see Table 19).

The potential impact of using the prognostic score in comparison of OS between the treatment arms was also assessed. In unadjusted analysis, an HR of 0.794 (95% CI 0.690-0.913, P=0.0012) was observed for atezolizumab versus docetaxel, in accordance with published results.20 A lower HR (0.785; 95% CI 0.678-0.909) was observed by excluding patients with RoPro2>0.81. Despite the resulting loss of sample size, the significance level (P=0.0012) was almost identical when compared with analysis of the entire dataset (P=0.0012).

The RoPro1 was also shown to be capable of discriminating event groups in longitudinal monitoring as shown in Figure 3. Patients who died had elevated RoPro1 99 days prior to death, mean RoPro1 0.32 (SD 0.45), and the score significantly (P=8.78x10-15) increased towards the event, mean RoPro1 0.77 (SD 0.48) for last measure prior to death. Patients progressing (black) showed significant increase (P=4.62x10-6) starting 66 days prior to progression, mean RoPro1 0.02 (SD 0.45), to a mean RoPro1 0.15 (SD 0.46) at the event. Partial (n=191 patients) and complete responders (n=11 patients) started with a low RoPro1 (mean -0.10, SD 0.40) which did not change significantly towards the response event (P=0.46).

Finally, the ability of the RoPro2 to discriminate event occurrence in longitudinal monitoring was assessed (Figure 5). For patients who died (top curve), the score significantly worsened towards the event (P=6.50×10-14, Figure 5A, B), mean score 0.09 (SD 0.50) at baseline, 0.30 (SD 0.54) for last measure before death. Patients progressing (middle curve) also showed a significant, but less pronounced (Figure 5A), increase (P=3.90×10-11, Figure 5C). Partial (n=191) and complete (n=11) responders (bottom curve) did not change significantly towards the response event (Figure 5A, D).

As discussed above, the RoPro1 and 2 were validated in two independent clinical studies (Phase 1 and 3), both investigating immunotherapeutic treatments. Our analysis in study BP29428 (combination treatment of emactuzumab and atezolizumab, NCT02323191) showed that the scores not only correlate with OS but also with particular early study drop-out (within less than 3 days) as shown in Table 16 for RoPro1. Hence, using RoPro 1 or 2 to exclude very high risk patients may help protect patients from unnecessary exposure to study procedural burden and potential adverse events. It may also support rapid study conduct and lower trial costs, while not significantly hampering recruitment as only few patients may need to be excluded. Similarly, the exclusion of the 10% patients with highest RoPro1 or 2 in the OAK Phase 3 trial of single agent atezolizumab in NSCLC did increase the effect of treatment when compared with the control arm of chemotherapy, indicating that these high risk patients benefit less from intervention. Our data suggest that the RoPro1 and 2 are able to more precisely differentiate between patients with high drop-out risk given their poor physical health status and patients who may still benefit from study treatment. It is also possible to define an a priori cut-off for patient exclusion/inclusion criteria such that a prespecified portion of patients (e.g. 5%) is to be excluded. Investigating the RoPro1 over time, we saw a steadily increasing trend in patients who subsequently progressed or died, while we were not able to identify a clear correlation of the course of the RoPro1 with response. However, a larger patient number may enable respective findings.

The RoPro1 and 2 are easy to use as, even though they use a large number of parameters, most parameters are routinely measured and/or available in clinical routine. The parameters are combined in one simple score that is easy to use. Further, missing parameters are tolerated for computing a patient's RoPro, meaning that even if patient information is not complete, then a score can still be generated. For example, when patient data is missing 5-10 parameters included in the model, a useful score can still be generated.

Furthermore, the RoPro1 and 2 can be applied across cancer indications demonstrated by the good performance of RoPro1 and 2 in BP29428 where 40% of patients suffered from cancer types other than the 12 used to build the score. Cancer-specific models can be generated using only data from subjects with the specific cancer type and examples of these are described in detail below. The cancer-specific models can outperform the general model, for example, for CLL and metastatic breast cancer, but the general models can still produce useful results.

Ease of use, applicability across cancer indications and increased prognostic power further encourage use of the RoPro1 and 2 for cohort comparison e.g. during dose escalation in FIH studies or for interpretation of study results compared to real world settings such as shown for BP29428 where the RoPro1 and 2 distribution differed from the Flatiron Health cohort. The presence of particular high-risk patients was identified aiding interpretation of overall study results. Furthermore, the continuous monitoring of the RoPro1 over time may increase the confidence for treatment decisions through treatment-emergent adverse events or beyond tumour progression. Our analysis in OAK indicated that high risk patients whose score worsens over time do not benefit from therapy. Observing stable scores or even slightly improving scores might indicate treatment benefits and could - next to other considerations - be used to take the decision to continue treatment.

Additional parameters for the score could include the number and type of prior treatment regimens when applying the RoPro1 or 2 to second or later line cohorts. The RoPro1 and 2 are very useful even when the number of prior treatment lines diverges between groups, as our analysis of the clinical studies demonstrates. Further optional parameters to include in the model and score could include urine, blood, (epi-)genetic biomarkers, and self-reported health (Sudlow C et al., 2015) and refined analyses, for example, association with progression-free survival (PFS).

The RoPro1 and 2 demonstrate the value of analyzing large patient datasets, resulting in a granularity previously not possible. Despite remaining uncertainties and inaccuracies typically encountered with retrospective real world data analysis (Kahn MG *et al.,* 2016), the inventors' findings show that bias is overcome. This is supported by high consistency with literature findings, across-cohort applicability of the score, and successful application to independent clinical study data which yielded model-fit quality comparable to Flatiron Healthdata set.

The RoPro1 was obtained by assessing 131 demographic, clinical, and routine blood parameters within a Cox proportional hazard framework. 99,249 patients were included from 12 different cohorts defined by tumour type (non-small cell lung, small cell lung, melanoma, bladder, breast, colorectal, renal cell, ovarian, hepatocellular, multiple myeloma, chronic lymphocytic leukaemia, diffuse large B cell lymphoma). All treatment regimens were included.

Training data was obtained from the Flatiron Health database derived from electronic health record (EHR) data from over 280 cancer clinics including more than 2.1 million active US cancer patients (https://flatiron.com/real-world-e vidence/), [12-2018]. The Flatiron Health database is longitudinal, demographically and geographically diverse which may be advantageous in forming a widely applicable model. The parameters may be numerical (e.g. age in years) or may be non-numerical (e.g. gender is female or male). Non-numerical parameters are assigned a numerical value for modelling. As discussed above, the values used for each parameter in the RoPro1 and 2 models are discussed in Table 15. For example, for the parameter of "Gender", a value of 1 is given if the patient is male and a value of 0 is given if the patient is female. Values assigned to non-numerical parameters, such as gender and smoking history, can be freely selected as long as they are consistently used in the training data and the data input to a score for a patient. Further, numerical values may be scaled without impacting the score produced, as long as such scaling is consistent over all of the training data and patient data input into the score.

The database includes structured data (e.g., laboratory values, and prescribed drugs) and unstructured data, for example, collected via technology-enabled chart abstraction from physician's notes and other unstructured documents (e.g., biomarker reports).

The database may be organized according to cohorts defined by cancer type: hepatocellular carcinoma (HCC), advanced melanoma, advanced non-small-cell lung carcinoma (NSCLC), small-cell lung carcinoma (SCLC), bladder cancer, metastatic renal cell carcinoma (RCC), metastatic colorectal cancer (CRC), diffuse large B-cell lymphoma (DLBCL), ovarian cancer, metastatic breast cancer, multiple myeloma, chronic lymphocytic leukemia (CLL) and optionally further follicular lymphoma, pancreatic cancer and head & neck cancer. In the Flatiron Health database, patients are similar in age, sex, and race/ethnicity to the US population of patients with melanoma, NSCLC, and RCC according to estimates of disease prevalence in Surveillance, Epidemiology, and End Results data from 2014 [National Cancer Institute. SEER*Stat software, version 8.3.4. http://seer.cancer.gov/seerstat. Accessed February 9, 2018.], but in other embodiments of the invention, databases including patients with alternative characteristics may be used. This may be advantageous in tailoring a model to a particular population. In the Flatiron Health database, all cohort data sets included demographic data, clinical data, such as cancer type, disease stage, and comorbidities, medication prescription data, and routine blood biomarker data.

Data from the December 2018 data release from Flatiron Health including demographics, clinical data, such as cancer type, disease stage, and comorbidities, medication prescription data, and routine blood biomarker data was used to form the example RoPro1. In total, 99,249 patients from 12 cohorts were available for analysis: advanced melanoma (n=3,543), advanced non-small-cell lung carcinoma (NSCLC) (n=33,575), bladder cancer (n=4,570), chronic lymphocytic leukaemia (CLL) (n=8,904), diffuse large B-cell lymphoma (DLBCL) (n=3,396), hepatocellular carcinoma (HCC) (n=1,028), metastatic breast cancer (n=12,425), metastatic colorectal cancer (CRC) (n=14,487), metastatic renal cell carcinoma (RCC) (n=4,057), multiple myeloma (n=5,345), ovarian cancer (n=3,713), small-cell lung carcinoma (SCLC) (n=4,206). For final analyses, 42 general measurements and 89 further cohort-specific biomarkers were available. A description of respective patient characteristics is included in table 1A. Median follow up-time was 23.3 months (+/-21.4 SD), median survival time was 20.4 months (95% CI 20.13 to 20.70).

Data from the May 2019 data release from Flatiron Health including demographics, clinical data, such as cancer type, disease stage, and comorbidities, medication prescription data, and routine blood biomarker data was used to form the example RoPro2. In total, 110,538 patients from 15 cohorts were available for analysis: advanced melanoma, advanced non-small-cell lung carcinoma (NSCLC), bladder cancer, chronic lymphocytic leukaemia (CLL), diffuse large B-cell lymphoma (DLBCL), hepatocellular carcinoma (HCC), metastatic breast cancer, metastatic colorectal cancer (CRC), metastatic renal cell carcinoma (RCC), multiple myeloma, ovarian cancer, small-cell lung carcinoma (SCLC), follicular lymphoma, pancreatic cancer and head& neck cancer. For final analyses, 45 general measurements and 99 further cohort-specific biomarkers were available. A description of respective patient characteristics is included in table 1B. Median survival time was 18.7 months (95% confidence interval [CI] 18.5-18.9).

Only parameters available for more than 25% of the patients were used and patients with missing first line of treatment information were excluded.

To form the model, overall survival (OS) of patients in the training data was investigated using Cox proportional hazard model (Cox DR, Journal of the Royal Statistical Society Series B (Methodological) Vol. 34). Survival time was calculated from the start of the patient's first line of treatment (defined as T0) to the event "death", as coded in the real-world mortality table (Curtis MD et al., 2018). For cohorts including only advanced/metastatic patients, the first line was the first advanced/metastatic line of treatment. Censored follow-up times were computed as the days elapsed from $T_0$ to the date of the patient's last documented contact (visit, medication administration, specimen collection, etc.) with their clinic. For training data for use in modelling, we used the patient's last available measurements prior to $T_0$.

For all continuous parameters, observations more than 4 standard deviations from the mean were excluded. Missing data was imputed separately for each cohort using the missForest R package. (R Core Team, 2013). For sensitivity analysis, the analysis was repeated with missing patient data replaced by the cohort parameter mean.

The data may be screened so that only significant parameters are included in the model. When forming the example model, as an initial screening, each parameter was analysed in its own right. Parameters with a P-value smaller than $\alpha_1 \alpha$=0.05/45=0.0011 (Bonferroni-correction) were considered eligible for modelling and retained in the training data. Parameters were included in the model following the ordering given by their importance in the screening analysis. In order for a parameter to be retained in the model, it was required that the inclusion of the parameter produced significant model improvement (P$\leq\alpha_1$). Parameters that lost significance due to inclusion of another parameter were removed from the model. By construction, this procedure controls the family-wise error rate at $\alpha$=0.05, i.e., all parameters are significant after adjustment for multiple testing.

For continuous variables, the cox model yields hazard ratios (HR) which are given per unit of the investigated parameter: for age, e.g., the HR is the HR per age difference of 1 year. Hence, for parameters with high absolute values, HR estimates may appear to be of negligible relevance, despite overwhelming statistical evidence. Therefore, the "tailHR" was also used. The tailHR is the HR of a patient with a particular high parameter value (equal to that of the 97.5%-quantile) as compared to a patient with a particular low value (2.5%-quantile). Statistical testing, however, was based on the full quantitative modelling.

*Modified RoPros*

As discussed above, the RoPro1 and 2 models and scores can be modified by including other parameters or modifying the training data, for example, to only include patients with a specific cancer-type. The specific models and scores can have improved accuracy over the general RoPro1 and 2 discussed above for the cancer type in question.

*General RoPro1 score with cohort covariate*

This score is given by the formula:
-0.03644 (Albumin-38.79) + 0.27834 (ECOG-0.78) + 0.00035 (LDH-273.99) -0.00921 (Lymphocytes/100 Leukocytes-25.83) -0.04271 (Hemoglobin-12.28) + 0.00034 (ALP-108.93) + 0.05494 (NLR-0.57) -0.02653 (Chloride-101.58) + 0.00603 (Heart rate-83.37) + 0.00373 (AST-25.98) + 0.01032 (Age-66.36) + 0.01123 (Urea nitrogen-17.33) -0.01622 (Oxygen-96.42) + 0.1092 (TNM stage-2.98) -0.00672 (Protein-69.7) -0.00296 (Systolic RR-129.58) - 0.03019 (Eosinophils/100 Leukocytes-2.32) + 0.06207 (Bilirubin-0.51) + 0.07228 (Calcium-9.37) + 0.16482 (Sex-0.48) -0.0075 (BMI-28.25) + 0.25569 (Smoking-0.36) -0.00048 (Platelets-272.01) + 0.05308 (No. of metastatic sites-0.44) -0.00334 (ALT-24.63) + 0.00082 (Leukocytes-12.78) + 0.16954 (AdvancedNSCLC-0.34) + 0.20866 (AdvancedMelanoma-0.04) + 0.33719 (BLADDER-0.05) -0.69241 (CLL-0.09) -1.28587 (DLBCL-0.03) + 0.58248 (HCC-0.01) -0.04801 (Metastatic-BreastCancer-0.13) -0.0726 (MetastaticRCC-0.04) - 0.76472 (MultipleMyeloma-0.05) -0.47683 (OVARIAN-0.04) + 0.20785 (SCLC-0.04)

This score takes into account the type of cancer by including the additional parameters of the cancer type in the score.

*General RoPro2 score with cohort covariate*

This score is given by the formula: 0.00942(age-66.695)+0.13976(gender-0.502)+0.2006(smoking-0.581) +0.07037(no of metastatic sites-0.163)+0.22798(ECOG-0.834)+0.09204(NLR-0.583)-0.00734(BMI-27.838)-0.0393(oxygen-96.607)-0.00283(SBP-128.707)+0.00526(heart rate-83.246)-0.04512(Hgb-12.092)+0.00864(leukocytes-11.077) +0.01171(urea nitrogen-17.53)+0.10861(calcium-9.314)-0.00066(platelets-272.168)-0.00614(lymphocyte-leukocyte-ratio-23.786)+0.00342(AST-26.729)+0.00095(ALP-111.233)-0.00885(protein-69.034)-0.00432(ALT-25.152)-0.04085(albumin-37.798)+0.09984(bilirubin-0.523)-0.01623(lymphocytes-3.683)-0.00467(carbon dioxide-25.768)-0.02848(chloride-101.369)+0.10973(monocytes-0.729)-0.02952(eosinophils-leukocytes-ratio-2.225) +0.00055(LDH-286.624)+0.0806(TumorStage-3.101)+0.24079(AdvancedNSCLC-0.309)+0.17555(AdvancedMelanoma-0.032)+0.23156(BLADDER-0.043)-0.82379(CLL-0.079)-1.32362(DLBCL-0.033) +0.68852(HCC-0.011)-0.09865(MetastaticBreastCancer-0.107)+0.06371(MetastaticRCC-0.036)-0.78937(MultipleMyeloma-0.057)-0.50345(OVARIAN-0.035)+0.28493(SCLC-0.04)+0.33059(HEAD_NECK-0.039)-1.79085(FOLLICULAR-0.004)+0.91282(PANCREATIC-0.045). This score takes into account the type of cancer by including the additional parameters of the cancer type in the score.

When applied to independent cohorts, it can be of interest to extend the RoPro by specific variables, which may be available for that cohort. In such a case, it is possible to construct an extended RoPro by fitting a cox model on the cohort data, using the original RoPro and a new variable X as parameters. By the same principle as used for the construction of the RoPro formula itself, this yields a formula of the form ln(HR(RoPro))*(RoProj - mean(RoPro)) +ln(HR(X))*(Xj-mean(X)). In this formula, the means of RoPro and X are those observed in the study. For the RoPro term, the general formula is used, i.e. the weights of the underlying 29 variables are not re-estimated. Only the weight of the RoPro itself, ln(HR(RoPro)), is estimated from the data, to determine the right balance of the RoPro and the additional variable X. The extended RoPro formula then can be applied a posteriori to the new sample or a priori to yet another sample.

The RoPro distribution can be shifted in samples with specific inclusion criteria. A typical situation may be a study design, in which all patients are required to take a specific value z for a RoPro variable xi. In this case, the RoPro distribution may be shifted by wi(z-mi), where mi is the mean value of xi in the training data and wi is the variable weight ln(HR) in the cox model. The RoPro cut-off value can be shifted by wi(z-mi) to use the RoPro patient exclusion criterion. If a study, for instance, requires that all patients have an ECOG of 1, mean RoPro2 may shifted by 1.264*(1-0.82) ~ 0.12, as compared to the training data (which has a mean ECOG of 0.83). Accordingly, the RoPro2 cut-off of 1.13 should be replaced by 1.25. As a further example, the sub-cohort of HER2 positive metastatic breast cancer patients has a much better prognosis than other metastatic breast cancer patients, despite the fact that HER2 overexpression is known to be a risk factor for the development of breast cancer. This paradox is well known and explained by the availability and systematic application of dedicated targeted therapies. In the RoPro2 application, for patients with positive HER_Status, the respective term -0.708* (HER2_Status-0.206) of the RoPro formula (Supp Table 7), gives the value -0.56. Accordingly, it may be appropriate to shift the RoPro cut-off by -0.56 in this case. It should be noted that the HER2 variable differs from the general RoPro variables, since it directly influences treatment decision. In the RoPro training data, the vast majority of HER2+ patients receive targeted therapy (trastuzumab, pertuzumab, etc.). For HER2+ patients who do not receive target therapy, the respective RoPro term would be miss-specified. In general, due to the interaction between HER2 status and treatment, in efficacy comparisons, it can be decided according to the actual study design if the inclusion of the HER2 term in the RoPro is appropriate.

*Cancer-specific models*

The cancer-specific models and scores discussed below are created in the same way as the general RoPro1 and 2 models described above, with the exception that only training data from the appropriate cancer-type was included.

A RoPro1 score specific to advance melanoma is given by the formula:
-0.03255 (Albumin-39.83) + 0.28838 (ECOG-0.7) + 0.00046 (LDH-286.82) -0.00931 (Lymphocytes/100 Leukocytes-21.7) -0.03681 (Hemoglobin-13.26) + 0.00119 (ALP-90.46) + 0.13144 (NLR-0.61) -0.02788 (Chloride-102.07) + 0.00564 (Heart rate-80.06) + 0.00412 (AST-24.45) + 0.00595 (Age-65.15) + 0.0086 (Urea nitrogen-17.68) -0.04493 (Oxygen-96.89) + 0.12968 (TNM stage-3.03) -0.017 (Protein-69.04) -0.00271 (Systolic RR-131.2) - 0.04086 (Eosinophils/100 Leukocytes-2.53) + 0.00564 (Bilirubin-0.56) + 0.06153 (Calcium-9.34) + 0.14823 (Sex-0.67) -0.00581 (BMI-29.07) -0.00021 (Platelets-255.36) -0.02629 (No. of metastatic sites-0.99) -0.0009 (ALT-25.6) + 0.00519 (Leukocytes-8.2)

**Table 3:** Final RoPro cox regression model (OS) for the advanced melanoma RoPro1.

| Parameter | units | HR[1] [95% CI] | p-value |
|---|---|---|---|
| Albumin | g/L | 0.968 [0.953; 0.983] | 3.30E-05 |
| ECOG | none | 1.334 [1.230; 1.447] | 3.16E-12 |
| LDH | U/L | 1.000 [1.000; 1.001] | 2.31E-04 |
| Lymphocytes/100 leukocytes | % | 0.991 [0.982; 0.999] | 3.41E-02 |
| Hemoglobin | g/dL | 0.964 [0.935; 0.994] | 1.90E-02 |
| ALP | U/L | 1.001 [1.000; 1.002] | 2.02E-02 |
| NLR | none | 1.140 [0.996; 1.306] | 5.73E-02 |
| Chloride | mmol/L | 0.973 [0.958; 0.987] | 1.86E-04 |
| Heart rate | bpm | 1.006 [1.002; 1.009] | 6.53E-04 |
| AST | U/L | 1.004 [1.000; 1.009] | 7.13E-02 |
| Age | none | 1.006 [1.002; 1.010] | 7.54E-03 |
| Urea nitrogen | mg/dL | 1.009 [1.001; 1.016] | 2.18E-02 |
| Oxygen | % | 0.956 [0.923; 0.990] | 1.19E-02 |
| TNM Stage | none | 1.138 [1.078; 1.203] | 3.63E-06 |
| Protein | g/L | 0.983 [0.974; 0.992] | 3.29E-04 |
| Systolic RR | mmHg | 0.997 [0.994; 1.000] | 6.14E-02 |
| Eosinophils/100 leukocytes | % | 0.960 [0.929; 0.992] | 1.49E-02 |
| Bilirubin | mg/dL | 1.006 [0.852; 1.187] | 9.47E-01 |
| Calcium | mg/dL | 1.063 [0.951; 1.189] | 2.80E-01 |
| Sex | none | 1.160 [1.041; 1.292] | 7.32E-03 |
| BMI | kg/m$^2$ | 0.994 [0.987; 1.002] | 1.23E-01 |
| Smoking | none | | - |
| Platelets | 10*9/L | 1.000 [0.999; 1.000] | 5.28E-01 |
| No. of metastatic sites | none | 0.974 [0.945; 1.004] | 8.86E-02 |
| ALT | U/L | 0.999 [0.996; 1.003] | 6.07E-01 |
| Leukocytes | 10*9/L | 1.005 [0.995; 1.016] | 3.35E-01 |
| [1]HR = Hazard ratio on original scale | | | |

A RoPro1 score specific to advanced NSCLC is is given by the formula:

-0.028 (Albumin-37.92) + 0.24063 (ECOG-0.89) + 0.00065 (LDH-265.82) -0.01143 (Lymphocytes/100 Leukocytes-18.89) -0.03854 (Hemoglobin-12.51) + 0.00059 (ALP-105.95) + 0.06241 (NLR-0.78) -0.02719 (Chloride-100.81) + 0.0056 (Heart rate-85.61) + 0.00315 (AST-22.29) + 0.00746 (Age-67.64) + 0.0103 (Urea nitrogen-16.64) -0.02669 (Oxygen-95.74) + 0.12445 (TNM stage-3.31) -0.00811 (Protein-68.83) -0.00285 (Systolic RR-127.43) -0.02494 (Eosinophils/100 Leukocytes-2.35) + 0.08866 (Bilirubin-0.47) + 0.0813 (Calcium-9.36) + 0.19274 (Sex-0.53) -0.00726 (BMI-27.1) + 0.17495 (Smoking-0.87) -0.00033 (Platelets-294.59) + 0.08169 (No. of metastatic sites-0.44) -0.00399 (ALT-23.17) + 0.01166 (Leukocytes-9.29) + 0.05736 (SquamousCell-0.26) -0.23384 (PrimarySiteTumor_PDL1-0.28) -0.47035 (ALK-0.03) -0.38758 (EGFR-0.14) + 0.08973 (KRAS-0.3)

**Table 4:** Final RoPro cox regression model (OS) for the advanced NSCLC RoPro1.

| Parameter | units | HR1 [95% CI] | p-value |
|---|---|---|---|
| Albumin | g/L | 0.972 [0.969; 0.976] | 1.07E-48 |
| ECOG | none | 1.272 [1.243; 1.302] | 1.62E-92 |
| LDH | U/L | 1.001 [1.001; 1.001] | 1.43E-21 |
| Lymphocytes/100 leukocytes | % | 0.989 [0.986; 0.991] | 6.87E-18 |
| Hemoglobin | g/dL | 0.962 [0.954; 0.971] | 1.75E-17 |
| ALP | U/L | 1.001 [1.000; 1.001] | 3.87E-12 |
| NLR | none | 1.064 [1.019; 1.112] | 5.34E-03 |
| Chloride | mmol/L | 0.973 [0.969; 0.977] | 4.11E-39 |
| Heart rate | bpm | 1.006 [1.005; 1.006] | 1.90E-38 |
| AST | U/L | 1.003 [1.001; 1.005] | 2.84E-04 |
| Age | none | 1.007 [1.006; 1.009] | 1.26E-20 |
| Urea nitrogen | mg/dL | 1.010 [1.008; 1.013] | 2.53E-21 |
| Oxygen | % | 0.974 [0.967; 0.980] | 6.02E-15 |
| TNM Stage | none | 1.133 [1.113; 1.153] | 1.09E-42 |
| Protein | g/L | 0.992 [0.989; 0.994] | 2.29E-10 |
| Systolic RR | mmHg | 0.997 [0.996; 0.998] | 3.87E-14 |
| Eosinophils/100 leukocytes | % | 0.975 [0.967; 0.984] | 6.55E-08 |
| Bilirubin | mg/dL | 1.093 [1.023; 1.167] | 7.92E-03 |
| Calcium | mg/dL | 1.085 [1.058; 1.112] | 9.13E-11 |
| Sex | none | 1.213 [1.180; 1.247] | 1.21E-42 |
| BMI | kg/m$^2$ | 0.993 [0.991; 0.995] | 1.81E-12 |
| Smoking | none | 1.191 [1.141; 1.243] | 1.05E-15 |
| Platelets | 10*9/L | 1.000 [1.000; 1.000] | 1.92E-05 |
| No. of metastatic sites | none | 1.085 [1.068; 1.103] | 1.08E-23 |
| ALT | U/L | 0.996 [0.995; 0.997] | 7.26E-10 |
| Leukocytes | 10*9/L | 1.012 [1.009; 1.015] | 2.57E-13 |
| SquamousCell2 | none | 1.059 [1.027; 1.092] | 2.83E-04 |
| PrimarySiteTumor_PDL13 | none | 0.791 [0.710; 0.883] | 2.64E-05 |
| ALK4 | none | 0.625 [0.558; 0.700] | 5.38E-16 |
| EGFR5 | none | 0.679 [0.643; 0.716] | 7.37E-45 |
| KRAS6 | none | 1.094 [1.034; 1.158] | 1.92E-03 |

[1]HR = Hazard ratio on original scale
[2]Squamous cell carcinoma vs non-squamous cell carcinoma
[3]PDL1 status in primary tumour
[4]Presence of ALK rearrangement, consenus of assessment in blood, tumour site, and metastatic site
[5]Presence of EGFR mutation, consenus of assessment in blood, tumour site, and metastatic site
[6]Presence of KRAS mutation, consenus of assessment in blood, tumour site, and metastatic site

A RoPro1 score specific to bladder cancer is is given by the formula:

-0.03776 (Albumin-38.24) + 0.31113 (ECOG-0.86) -0.0000006 (LDH-207.51) -0.01648 (Lymphocytes/100 Leukocytes-19.98) -0.05865 (Hemoglobin-11.79) + 0.00199 (ALP-102.19) -0.02476 (NLR-0.75) -0.02689 (Chloride-101.84) + 0.0067 (Heart rate-81.99) + 0.01477 (AST-20.89) + 0.00582 (Age-70.66) + 0.00883 (Urea nitrogen-21.95) -0.01548 (Oxygen-96.77) -0.05903 (TNM stage-3.12) -0.01324 (Protein-69.3) -0.00097 (Systolic RR-129.7) - 0.0303 (Eosinophils/100 Leukocytes-2.66) + 0.16313 (Bilirubin-0.45) + 0.20268 (Calcium-9.35) + 0.14791 (Sex-0.74) -0.0055 (BMI-27.56) + 0.03807 (Smoking-0) -0.00046 (Platelets-291.95) + 0.08332 (No. of metastatic sites-0.38) -0.00943 (ALT-19.7) + 0.00372 (Leukocytes-8.57) -0.29635 (Surgery-0.5) -0.09292 (NStAge-0.87) + 0.0882 (TStAge-2.5)

**Table 5:** Final RoPro cox regression model (OS) for the bladder cancer RoPro1.

| Parameter | units | HR[1] [95% CI] | p-value |
|---|---|---|---|
| Albumin | g/L | 0.963 [0.952; 0.974] | 2.19E-11 |
| ECOG | none | 1.365 [1.281; 1.454] | 7.48E-22 |
| LDH | U/L | 1.000 [0.999; 1.001] | 9.98E-01 |
| Lymphocytes/100 leukocytes | % | 0.984 [0.976; 0.991] | 1.21E-05 |
| Hemoglobin | g/dL | 0.943 [0.919; 0.968] | 7.67E-06 |
| ALP | U/L | 1.002 [1.001; 1.003] | 1.11E-10 |
| NLR | none | 0.976 [0.867; 1.097] | 6.80E-01 |
| Chloride | mmol/L | 0.973 [0.962; 0.985] | 1.28E-05 |
| Heart rate | bpm | 1.007 [1.004; 1.009] | 1.45E-07 |
| AST | U/L | 1.015 [1.010; 1.020] | 1.05E-08 |
| Age | none | 1.006 [1.001; 1.010] | 1.08E-02 |
| Urea nitrogen | mg/dL | 1.009 [1.005; 1.013] | 4.44E-05 |
| Oxygen | % | 0.985 [0.965; 1.005] | 1.37E-01 |
| TNM Stage | none | 0.943 [0.529; 1.679] | 8.41E-01 |
| Protein | g/L | 0.987 [0.980; 0.994] | 3.46E-04 |
| Systolic RR | mmHg | 0.999 [0.997; 1.001] | 3.59E-01 |
| Eosinophils/100 leukocytes | % | 0.970 [0.942; 0.999] | 4.62E-02 |
| Bilirubin | mg/dL | 1.177 [0.975; 1.421] | 8.91E-02 |
| Calcium | mg/dL | 1.225 [1.125; 1.333] | 2.86E-06 |
| Sex | none | 1.159 [1.063; 1.265] | 8.67E-04 |
| BMI | kg/m$^2$ | 0.995 [0.988; 1.001] | 9.26E-02 |
| Smoking | none | 1.039 [0.597; 1.807] | 8.93E-01 |
| Platelets | 10*9/L | 1.000 [0.999; 1.000] | 3.13E-02 |
| No. of metastatic sites | none | 1.087 [1.043; 1.132] | 6.32E-05 |
| ALT | U/L | 0.991 [0.986; 0.995] | 1.74E-05 |
| Leukocytes | 10*9/L | 1.004 [0.991; 1.016] | 5.58E-01 |
| Surgery[2] | none | 0.744 [0.689; 0.802] | 1.77E-14 |
| NStage[3] | none | 0.911 [0.860; 0.965] | 1.62E-03 |
| TStage[4] | none | 1.092 [1.035; 1.152] | 1.23E-03 |

[1]HR = Hazard ratio on original scale
[2]Inidcates whether the patient has had a cystectomy or other relevant surgery
[3]N stage at initial diagnosis
[4]T stage at initial diagnosis

A RoPro1 score specific to CLL is is given by the formula:

-0.03867 (Albumin-41.27) + 0.41112 (ECOG-0.61) + 0.00035 (LDH-257.53) -0.00048 (Lymphocytes/100 Leukocytes-67.49) + 0.00858 (Hemoglobin-11.9) + 0.00322 (ALP-86.29) + 0.003 (NLR-0.16) -0.0278 (Chloride-103.45) + 0.00728 (Heart rate-77.61) + 0.00348 (AST-24.35) + 0.05572 (Age-69.71) + 0.00942 (Urea nitrogen-20.19) -0.07428 (Oxygen-96.72) + 0.01033 (TNM stage-1.38) -0.00604 (Protein-65.51) -0.00352 (Systolic RR-129.82) + 0.01924 (Eosinophils/100 Leukocytes-1.18) + 0.0243 (Bilirubin-0.64) + 0.04474 (Calcium-9.26) + 0.28007 (Sex-0.62) -0.01238 (BMI-29.11) + 1.28681 (Smoking-0.01) -0.00108 (Platelets-165.63) + 0.6591 (No. of metastatic sites-0.01) -0.00752 (ALT-22.11) -0.00108 (Leukocytes-57.72) -0.02779 (hematocrit-36.55) + 0.01526 (mono_leuko-6.81) + 0.4047 (Status17pDel-0.09)

Table 6: Final RoPro1 cox regression model (OS) for the CLL RoPro1.

| Parameter | units | HR[1] [95% CI] | p-value |
|---|---|---|---|
| Albumin | g/L | 0.962 [0.951; 0.974] | 2.21E-10 |
| ECOG | none | 1.509 [1.401; 1.625] | 1.67E-27 |
| LDH | U/L | 1.000 [1.000; 1.001] | 6.39E-02 |
| Lymphocytes/100 leukocytes | % | 1.000 [0.997; 1.002] | 7.14E-01 |
| Hemoglobin | g/dL | 1.009 [0.972; 1.047] | 6.53E-01 |
| ALP | U/L | 1.003 [1.002; 1.004] | 3.24E-07 |
| NLR | none | 1.003 [0.896; 1.1231 | 9.59E-01 |
| Chloride | mmol/L | 0.973 [0.96; 0.985] | 3.58E-05 |
| Heart rate | bpm | 1.007 [1.004; 1.010] | 8.79E-07 |
| AST | U/L | 1.003 [0.998; 1.009] | 1.86E-01 |
| Age | none | 1.057 [1.051; 1.064] | 1.24E-77 |
| Urea nitrogen | mg/dL | 1.009 [1.004; 1.015] | 4.87E-04 |
| Oxygen | % | 0.928 [0.899; 0.959] | 5.87E-06 |
| TNM Stage | none | 1.010 [0.973; 1.049] | 5.92E-01 |
| Protein | g/L | 0.994 [0.987; 1.001] | 7.31E-02 |
| Systolic RR | mmHg | 0.996 [0.994; 0.999] | 2.22E-03 |
| Eosinophils/100 leukocytes | % | 1.019 [0.980; 1.060] | 3.38E-01 |
| Bilirubin | mg/dL | 1.025 [0.934; 1.124] | 6.05E-01 |
| Calcium | mg/dL | 1.046 [0.956; 1.144] | 3.28E-01 |
| Sex | none | 1.323 [1.214; 1.442] | 2.01E-10 |
| BMI | kg/m$^2$ | 0.988 [0.982; 0.993] | 1.28E-05 |
| Smoking | none | 3.621 [2.755; 4.760] | 2.87E-20 |
| Platelets | 10*9/L | 0.999 [0.998; 1.000] | 3.29E-04 |
| No. of metastatic sites | none | 1.933 [1.531; 2.440] | 2.96E-08 |
| ALT | U/L | 0.993 [0.988; 0.997] | 1.03E-03 |
| Leukocytes | 10*9/L | 0.999 [0.998; 1.000] | 6.69E-03 |
| hematocrit | % | 0.973 [0.959; 0.986] | 8.27E-05 |
| monocytes/100 leukocytes | % | 1.015 [1.007; 1.024] | 2.42E-04 |
| Status17pDel[2] | none | 1.499 [1.334; 1.685] | 1.12E-11 |
| [1]HR = Hazard ratio on original scale [2]Patient's status for 17p deletion | | | |

A RoPro1 score specific to DLBCL is is given by the formula:
-0.01324 (Albumin-38.76) + 0.41084 (ECOG-0.78) + 0.00051 (LDH-330) -0.00259 (Lymphocytes/100 Leukocytes-21.57) -0.02629 (Hemoglobin-12.33) + 0.00246 (ALP-94.94) -0.14718 (NLR-0.67) -0.01611 (Chloride-101.62) + 0.00186 (Heart rate-83.71) + 0.00324 (AST-27.74) + 0.03897 (Age-65.41) + 0.0115 (Urea nitrogen-18.02) -0.08422 (Oxygen-97.03) + 0.11178 (TNM stage-2.8) -0.01996 (Protein-67.65) + 0.00247 (Systolic RR-129.35) + 0.03503 (Eosinophils/100 Leukocytes-2.42) -0.0067 (Bilirubin-0.6) -0.08837 (Calcium-9.45) + 0.21277 (Sex-0.54) -0.01197 (BMI-29.21) -0.32111 (Smoking-0) -0.00116 (Platelets-264.91) + 0.01659 (No. of metastatic sites-0.64) -0.00427 (ALT-25.71) + 0.01014 (Leukocytes-8.06) + 0.48816 (BM_CD5-0.19)

**Table 7:** Final RoPro1 cox regression model (OS) for the DLCBL RoPro1.

| Parameter | units | HR[1] [95% CI] | p-value |
|---|---|---|---|
| Albumin | g/L | 0.987 [0.969; 1.005] | 1.47E-01 |
| ECOG | none | 1.508 [1.349; 1.686] | 4.87E-13 |
| LDH | U/L | 1.001 [1.000; 1.001] | 1.81E-03 |
| Lymphocytes/100 leukocytes | % | 0.997 [0.989; 1.006] | 5.60E-01 |
| Hemoglobin | g/dL | 0.974 [0.932; 1.018] | 2.42E-01 |
| ALP | U/L | 1.002 [1.001; 1.004] | 2.08E-04 |
| NLR | none | 0.863 [0.717; 1.039] | 1.20E-01 |
| Chloride | mmol/L | 0.984 [0.964; 1.005] | 1.32E-01 |
| Heart rate | bpm | 1.002 [0.998; 1.006] | 3.86E-01 |
| AST | U/L | 1.003 [0.999; 1.008] | 1.55E-01 |
| Age | none | 1.040 [1.032; 1.047] | 2.07E-26 |
| Urea nitrogen | mg/dL | 1.012 [1.003; 1.020] | 7.20E-03 |
| Oxygen | % | 0.919 [0.877; 0.963] | 3.99E-04 |
| TNM Stage | none | 1.118 [1.036; 1.207] | 4.23E-03 |
| Protein | g/L | 0.980 [0.969; 0.992] | 1.15E-03 |
| Systolic RR | mmHg | 1.002 [0.999; 1.006] | 2.11E-01 |
| Eosinophils/100 leukocytes | % | 1.036 [0.988; 1.086] | 1.44E-01 |
| Bilirubin | mg/dL | 0.993 [0.861; 1.147] | 9.27E-01 |
| Calcium | mg/dL | 0.915 [0.824; 1.017] | 1.01E-01 |
| Sex | none | 1.237 [1.073; 1.426] | 3.37E-03 |
| BMI | kg/m$^2$ | 0.988 [0.978; 0.999] | 2.82E-02 |
| Smoking | none | 0.725 [0.298; 1.767] | 4.80E-01 |
| Platelets | 10*9/L | 0.999 [0.998; 1.000] | 9.08E-04 |
| No. of metastatic sites | none | 1.017 [0.928; 1.114] | 7.22E-01 |
| ALT | U/L | 0.996 [0.991; 1.000] | 6.89E-02 |
| Leukocytes | 10*9/L | 1.010 [0.998; 1.023] | 1.14E-01 |
| BM_CD5[2] | none | 1.629 [1.273; 2.085] | 1.05E-04 |

[1]HR = Hazard ratio on original scale
[2]CD5 expression status in bone marrow as reported by IHC or Flow Cytometry

A RoPro1 score specific to HCC is is given by the formula:
-0.04636 (Albumin-34.77) + 0.09009 (ECOG-0.85) -0.00024 (LDH-242.97) -0.00747 (Lymphocytes/100 Leukocytes-21.85) -0.03135 (Hemoglobin-12.56) + 0.0009 (ALP-190.83) + 0.01655 (NLR-0.66) -0.01749 (Chloride-101.53) + 0.00797 (Heart rate-79.59) + 0.00375 (AST-81.06) + 0.00472 (Age-66.35) + 0.01482 (Urea nitrogen-16.98) -0.05141 (Oxygen-97.04) + 0.24331 (TNM stage-3.33) + 0.00045 (Protein-71.86) + 0.00075 (Systolic RR-128.81) -0.04362 (Eosinophils/100 Leukocytes-2.55) + 0.10363 (Bilirubin-1.23) + 0.07832 (Calcium-9.13) + 0.13751 (Sex-0.8) + 0.00405 (BMI-27.52) -0.87098 (Smoking-0.01) + 0.00044 (Platelets-194.03) + 0.02497 (No. of metastatic sites-0.2) -0.00273 (ALT-56.13) + 0.0234 (Leukocytes-6.53) + 0.34357 (IsAscites-0.25)

**Table** 8: Final RoPro 1 cox regression model (OS) for the HCC RoPro1.

| Parameter | units | HR[1] [95% CI] | p-value |
|---|---|---|---|
| Albumin | g/L | 0.955 [0.936; 0.974] | 6.05E-06 |
| ECOG | none | 1.094 [0.964; 1.242] | 1.64E-01 |
| LDH | U/L | 1.000 [0.999; 1.001] | 6.18E-01 |
| Lymphocytes/100 leukocytes | % | 0.993 [0.980; 1.006] | 2.59E-01 |
| Hemoglobin | g/dL | 0.969 [0.923; 1.018] | 2.10E-01 |
| ALP | U/L | 1.001 [1.000; 1.002] | 1.30E-02 |
| NLR | none | 1.017 [0.825; 1.253] | 8.77E-01 |
| Chloride | mmol/L | 0.983 [0.96; 1.006] | 1.43E-01 |
| Heart rate | bpm | 1.008 [1.003; 1.013] | 4.08E-03 |
| AST | U/L | 1.004 [1.002; 1.006] | 7.55E-05 |
| Age | none | 1.005 [0.996; 1.013] | 2.62E-01 |
| Urea nitrogen | mg/dL | 1.015 [1.005; 1.025] | 3.32E-03 |
| Oxygen | % | 0.950 [0.897; 1.006] | 7.67E-02 |
| TNM Stage | none | 1.275 [1.134; 1.435] | 5.05E-05 |
| Protein | g/L | 1.000 [0.989; 1.012] | 9.38E-01 |
| Systolic RR | mmHg | 1.001 [0.996; 1.005] | 7.39E-01 |
| Eosinophils/100 leukocytes | % | 0.957 [0.908; 1.009] | 1.06E-01 |
| Bilirubin | mg/dL | 1.109 [1.019; 1.207] | 1.67E-02 |
| Calcium | mg/dL | 1.081 [0.911; 1.285] | 3.72E-01 |
| Sex | none | 1.147 [0.945; 1.394] | 1.66E-01 |
| BMI | kg/m$^2$ | 1.004 [0.991; 1.018] | 5.55E-01 |
| Smoking | none | 0.419 [0.154; 1.139] | 8.83E-02 |
| Platelets | 10*9/L | 1.000 [0.999; 1.001] | 3.61E-01 |
| No. of metastatic sites | none | 1.025 [0.903; 1.165] | 7.01E-01 |
| ALT | U/L | 0.997 [0.994; 1.000] | 5.48E-02 |
| Leukocytes | 10*9/L | 1.024 [0.982; 1.068] | 2.75E-01 |
| IsAscites[2] | % | 1.410 [1.172; 1.696] | 2.70E-04 |

[1]HR = Hazard ratio on original scale
[2]Indicates if the patient had documented evidence of ascites at or within 60 days prior to starting systemic therapy

A RoPro1 score specific to metastatic breast cancer is is given by the formula:
-0.03505 (Albumin-40.26) + 0.27677 (ECOG-0.65) + 0.00036 (LDH-279.9) -0.02201 (Lymphocytes/100 Leukocytes-25.19) -0.05064 (Hemoglobin-12.57) + 0.0005 (ALP-116.55) + 0.0224 (NLR-0.39) -0.00573 (Chloride-102.11) + 0.00426 (Heart rate-84.54) + 0.00453 (AST-30.73) + 0.00347 (Age-62.48) + 0.0056 (Urea nitrogen-15.74) -0.00592 (Oxygen-96.52) -0.05314 (TNM stage-2.79) -0.00482 (Protein-69.92) -0.00228 (Systolic RR-132.42) - 0.04465 (Eosinophils/100 Leukocytes-2.18) + 0.00263 (Bilirubin-0.48) + 0.07961 (Calcium-9.47) -0.06088 (Sex-0.01) -0.00505 (BMI-29.66) + 0.30504 (Smoking-0) -0.00041 (Platelets-259.77) + 0.01433 (No. of metastatic sites-0.65) -0.00285 (ALT-26.76) -0.01321 (Leukocytes-7.11) -0.00912 (granulocytes_leuko-65.19) -0.66376 (Status_ER-0.75) - 0.32253 (Status_PR-0.21) -0.64684 (Status_HER2-0.58)

**Table 9:** Final RoPro1 cox regression model (OS) for the metastatic breast cancer RoPro1.

| Parameter | units | HR[1] [95% CI] | p-value |
|---|---|---|---|
| Albumin | g/L | 0.966 [0.958; 0.973] | 4.42E-19 |

(continued)

| Parameter | units | HR[1] [95% CI] | p-value |
|---|---|---|---|
| ECOG | none | 1.319 [1.262; 1.378] | 1.14E-34 |
| LDH | U/L | 1.000 [1.000; 1.001] | 5.78E-04 |
| Lymphocytes/100 leukocytes | % | 0.978 [0.971; 0.985] | 2.79E-09 |
| Hemoglobin | g/dL | 0.951 [0.934; 0.968] | 2.30E-08 |
| ALP | U/L | 1.001 [1.000; 1.001] | 2.40E-04 |
| NLR | none | 1.023 [0.955; 1.096] | 5.24E-01 |
| Chloride | mmol/L | 0.994 [0.985; 1.003] | 2.12E-01 |
| Heart rate | bpm | 1.004 [1.003; 1.006] | 1.47E-06 |
| AST | U/L | 1.005 [1.003; 1.006] | 2.82E-09 |
| Age | none | 1.003 [1.001; 1.006] | 4.48E-03 |
| Urea nitrogen | mg/dL | 1.006 [1.001; 1.010] | 1.67E-02 |
| Oxygen | % | 0.994 [0.987; 1.001] | 8.28E-02 |
| TNM Stage | none | 0.948 [0.925; 0.972] | 3.01E-05 |
| Protein | g/L | 0.995 [0.990; 1.000] | 5.85E-02 |
| Systolic RR | mmHg | 0.998 [0.996; 0.999] | 5.12E-04 |
| Eosinophils/100 leukocytes | % | 0.956 [0.936; 0.977] | 5.25E-05 |
| Bilirubin | mg/dL | 1.003 [0.903; 1.113] | 9.61E-01 |
| Calcium | mg/dL | 1.083 [1.034; 1.134] | 6.88E-04 |
| Sex | none | 0.941 [0.754; 1.174] | 5.90E-01 |
| BMI | kg/m$^2$ | 0.995 [0.992; 0.998] | 2.66E-03 |
| Smoking | none | 1.357 [0.841; 2.189] | 2.11E-01 |
| Platelets | 10*9/L | 1.000 [0.999; 1.000] | 1.63E-02 |
| No. of metastatic sites | none | 1.014 [0.988; 1.042] | 2.96E-01 |
| ALT | U/L | 0.997 [0.995; 0.999] | 1.67E-03 |
| Leukocytes | 10*9/L | 0.987 [0.978; 0.996] | 3.40E-03 |
| granulocytes/100 leukocytes | % | 0.991 [0.984; 0.997] | 6.68E-03 |
| Status ER[2] | % | 0.515 [0.481; 0.551] | 1.26E-81 |
| Status PR[3] | none | 0.724 [0.680; 0.772] | 3.23E-23 |
| Status HER2[4] | none | 0.524 [0.487; 0.563] | 3.64E-69 |

[1]HR = Hazard ratio on original scale
[2]Estrogen receptor status
[3]Progesterone receptor status
[4]Human epidermal growth factor receptor 2 as reported by IHC or Flow Cytometry

A RoPro1 score specific to metastatic CRC is is given by the formula:
-0.04114 (Albumin-38.65) + 0.31521 (ECOG-0.7) + 0.00027 (LDH-321.84) -0.01522 (Lymphocytes/100 Leukocytes-22.63) -0.01996 (Hemoglobin-12.03) + 0.00019 (ALP-141.96) + 0.02894 (NLR-0.49) -0.02129 (Chloride-101.75) + 0.00589 (Heart rate-82.9) + 0.00545 (AST-30.64) + 0.01066 (Age-63.59) + 0.01004 (Urea nitrogen-15.01) -0.02764 (Oxygen-97.15) + 0.11828 (TNM stage-3.48) -0.00019 (Protein-70.32) -0.0026 (Systolic RR-130.17) - 0.00342 (Eosinophils/100 Leukocytes-2.81) + 0.11769 (Bilirubin-0.55) + 0.05607 (Calcium-9.31) + 0.08277 (Sex-0.56) -0.00752 (BMI-28.13) + 0.0168 (Smoking-0) -0.00067 (Platelets-298.33) + 0.05164 (No. of metastatic sites-0.51) -0.00523 (ALT-27.14) -0.00154 (Leukocytes-8.04) + 0.50779 (Status_BRAF-0.11) + 0.17346 (Status_KRAS-0.45) + 0.2077 (MSImod_Primary-0.06)

**Table 10:** Final RoPro1 cox regression model (OS) for the metastatic CRC RoPro1.

| Parameter | units | HR[1] [95% CI] | p-value |
|---|---|---|---|
| Albumin | g/L | 0.960 [0.953; 0.966] | 1.92E-30 |
| ECOG | none | 1.371 [1.315; 1.428] | 3.55E-51 |
| LDH | U/L | 1.000 [1.000; 1.000] | 7.88E-04 |
| Lymphocytes/100 leukocytes | % | 0.985 [0.981; 0.989] | 4.05E-12 |
| Hemoglobin | g/dL | 0.980 [0.965; 0.996] | 1.19E-02 |
| ALP | U/L | 1.000 [1.000; 1.000] | 4.86E-02 |
| NLR | none | 1.029 [0.963; 1.100] | 3.94E-01 |
| Chloride | mmol/L | 0.979 [0.971; 0.987] | 8.31E-08 |
| Heart rate | bpm | 1.006 [1.004; 1.008] | 4.21E-13 |
| AST | U/L | 1.005 [1.004; 1.007] | 5.25E-09 |
| Age | none | 1.011 [1.008; 1.013] | 9.70E-20 |
| Urea nitrogen | mg/dL | 1.010 [1.006; 1.014] | 1.17E-06 |
| Oxygen | % | 0.973 [0.957; 0.989] | 8.09E-04 |
| TNM Stage | none | 1.126 [1.091; 1.161] | 1.31E-13 |
| Protein | g/L | 1.000 [0.995; 1.004] | 9.34E-01 |
| Systolic RR | mmHg | 0.997 [0.996; 0.999] | 5.81E-05 |
| Eosinophils/100 leukocytes | % | 0.997 [0.982; 1.012] | 6.56E-01 |
| Bilirubin | mg/dL | 1.125 [1.065; 1.188] | 2.30E-05 |
| Calcium | mg/dL | 1.058 [0.999; 1.119] | 5.26E-02 |
| Sex | none | 1.086 [1.036; 1.139] | 6.76E-04 |
| BMI | kg/m$^2$ | 0.993 [0.989; 0.996] | 2.40E-05 |
| Smoking | none | 1.017 [0.696; 1.486] | 9.31E-01 |
| Platelets | 10*9/L | 0.999 [0.999; 1.000] | 1.71E-07 |
| No. of metastatic sites | none | 1.053 [1.027; 1.080] | 6.08E-05 |
| ALT | U/L | 0.995 [0.993; 0.997] | 6.47E-09 |
| Leukocytes | 10*9/L | 0.998 [0.993; 1.004] | 5.65E-01 |
| Status_BRAF[2] | none | 1.662 [1.479; 1.867] | 1.14E-17 |
| Status_KRAS[3] | none | 1.189 [1.126; 1.256] | 5.05E-10 |
| MSI_Primary[4] | none | 1.231 [1.066; 1.421] | 4.59E-03 |
| [1]HR = Hazard ratio on original scale<br>[2]Presence of BRAF mutation<br>[3]Presence of KRAS mutation<br>[4]Asessed in primary tissue, MSI-H and loss of MMR protein expression are subsumized into one class | | | |

A RoPro1 score specific to metastatic RCC is is given by the formula:

-0.04107 (Albumin-38.71) + 0.22807 (ECOG-0.81) + 0.00066 (LDH-239.9) -0.0166 (Lymphocytes/100 Leukocytes-21.81) -0.03591 (Hemoglobin-12.34) + 0.00042 (ALP-102.79) -0.07151 (NLR-0.59) -0.04034 (Chloride-101.67) + 0.00675 (Heart rate-80.99) + 0.01167 (AST-21.98) + 0.00478 (Age-65.5) + 0.01264 (Urea nitrogen-20.71) -0.00236 (Oxygen-96.65) + 0.10591 (TNM stage-3.11) -0.01258 (Protein-70.59) -0.00152 (Systolic RR-130.68) -0.03656 (Eosinophils/100 Leukocytes-2.54) -0.08249 (Bilirubin-0.5) + 0.05246 (Calcium-9.46) -0.01377 (Sex-0.7) -0.00665 (BMI-30.17) -0.04439 (Smoking-0.57) -0.00052 (Platelets-286.2) + 0.03115 (No. of metastatic sites-0.71) -0.01082 (ALT-23.41) + 0.03307 (Leukocytes-8.02) -0.40166 (Nephrectomy-0.68) -0.37353 (clearCell-0.7)

**Table 11:** Final RoPro1 cox regression model (OS) for the metastatic RCC RoPro1.

| Parameter | units | HR[1] [95% CI] | p-value |
|---|---|---|---|
| Albumin | g/L | 0.960 [0.949; 0.971] | 3.52E-12 |
| ECOG | none | 1.256 [1.166; 1.354] | 2.27E-09 |
| LDH | U/L | 1.001 [1.000; 1.001] | 3.81E-03 |
| Lymphocytes/100 leukocytes | % | 0.984 [0.976; 0.991] | 2.45E-05 |
| Hemoglobin | g/dL | 0.965 [0.938; 0.993] | 1.35E-02 |
| ALP | U/L | 1.000 [0.999; 1.001] | 3.71E-01 |
| NLR | none | 0.931 [0.832; 1.041] | 2.11E-01 |
| Chloride | mmol/L | 0.960 [0.948; 0.973] | 4.05E-09 |
| Heart rate | bpm | 1.007 [1.004; 1.010] | 6.23E-06 |
| AST | U/L | 1.012 [1.007; 1.016] | 5.35E-07 |
| Age | none | 1.005 [1.000; 1.009] | 4.25E-02 |
| Urea nitrogen | mg/dL | 1.013 [1.007; 1.018] | 3.35E-06 |
| Oxygen | % | 0.998 [0.971; 1.025] | 8.63E-01 |
| TNM Stage | none | 1.112 [1.054; 1.173] | 1.00E-04 |
| Protein | g/L | 0.988 [0.980; 0.995] | 1.44E-03 |
| Systolic RR | mmHg | 0.998 [0.996; 1.001] | 2.08E-01 |
| Eosinophils/100 leukocytes | % | 0.964 [0.933; 0.996] | 2.64E-02 |
| Bilirubin | mg/dL | 0.921 [0.764; 1.109] | 3.85E-01 |
| Calcium | mg/dL | 1.054 [0.980; 1.133] | 1.56E-01 |
| Sex | none | 0.986 [0.899; 1.082] | 7.71E-01 |
| BMI | kg/m$^2$ | 0.993 [0.988; 0.999] | 1.94E-02 |
| Smoking | none | 0.957 [0.879; 1.041] | 3.04E-01 |
| Platelets | 10*9/L | 0.999 [0.999; 1.000] | 4.48E-02 |
| No. of metastatic sites | none | 1.032 [0.993; 1.071] | 1.05E-01 |
| ALT | U/L | 0.989 [0.986; 0.993] | 1.74E-08 |
| Leukocytes | 10*9/L | 1.034 [1.015; 1.053] | 3.79E-04 |
| Nephrectomy[2] | none | 0.669 [0.598; 0.748] | 2.01E-12 |
| clearCell[3] | none | 0.688 [0.628; 0.754] | 1.55E-15 |
| [1]HR = Hazard ratio on original scale [2]Whether the patient has had a nephrectomy [3]Clear cell carcinoma yes/no | | | |

A score specific to multiple myeloma is is given by the formula:

-0.03132 (Albumin-37.7) + 0.40645 (ECOG-0.83) + 0.00093 (LDH-203.06) -0.00781 (Lymphocytes/100 Leukocytes-29.79) -0.04835 (Hemoglobin-10.98) + 0.00219 (ALP-82.55) -0.07473 (NLR-0.3) -0.01956 (Chloride-102.06) + 0.00436 (Heart rate-80.65) + 0.00191 (AST-24.06) + 0.03613 (Age-68.6) + 0.01272 (Urea nitrogen-22.03) -0.01123 (Oxygen-97.07) + 0.23461 (TNM stage-1.97) -0.00372 (Protein-82.13) -0.00398 (Systolic RR-134.69) -0.04269 (Eosinophils/100 Leukocytes-2.42) + 0.30565 (Bilirubin-0.5) + 0.01936 (Calcium-9.44) + 0.13929 (Sex-0.54) -0.00452 (BMI-29.26) + 0.9616 (Smoking-0) -0.00132 (Platelets-223.01) + 0.05053 (No. of metastatic sites-0.09) -0.00309 (ALT-23.53) + 0.00522 (Leukocytes-6.95) + 0.3403 (AbnormalitiesIdentified-0.26) -0.23267 (MProteinIgA-0.19) -0.23776 (MProteinIgG-0.54) -0.45875 (LightChainKappa-0.56) -0.32882 (LightChainLambda-0.34)

**Table 12:** Final RoPro1 cox regression model (OS) for the multiple myeloma RoPro1.

| Parameter | units | HR[1] [95% CI] | p-value |
|---|---|---|---|
| Albumin | g/L | 0.969 [0.959; 0.979] | 1.65E-09 |
| ECOG | none | 1.501 [1.386; 1.626] | 2.19E-23 |
| LDH | U/L | 1.001 [1.000; 1.002] | 1.10E-02 |
| Lymphocytes/100 leukocytes | % | 0.992 [0.986; 0.998] | 9.02E-03 |
| Hemoglobin | g/dL | 0.953 [0.926; 0.980] | 7.45E-04 |
| ALP | U/L | 1.002 [1.001; 1.004] | 2.38E-03 |
| NLR | none | 0.928 [0.814; 1.058] | 2.65E-01 |
| Chloride | mmol/L | 0.981 [0.966; 0.995] | 1.07E-02 |
| Heart rate | bpm | 1.004 [1.001; 1.008] | 1.69E-02 |
| AST | U/L | 1.002 [0.998; 1.006] | 3.36E-01 |
| Age | none | 1.037 [1.031; 1.043] | 2.13E-31 |
| Urea nitrogen | mg/dL | 1.013 [1.008; 1.017] | 6.35E-09 |
| Oxygen | % | 0.989 [0.954; 1.025] | 5.41E-01 |
| TNM Stage | none | 1.264 [1.159; 1.380] | 1.39E-07 |
| Protein | g/L | 0.996 [0.993; 1.000] | 2.57E-02 |
| Systolic RR | mmHg | 0.996 [0.993; 0.999] | 2.28E-03 |
| Eosinophils/100 leukocytes | % | 0.958 [0.925; 0.992] | 1.73E-02 |
| Bilirubin | mg/dL | 1.358 [1.139; 1.618] | 6.56E-04 |
| Calcium | mg/dL | 1.020 [0.958; 1.085] | 5.45E-01 |
| Sex | none | 1.149 [1.041; 1.270] | 6.03E-03 |
| BMI | $kg/m^2$ | 0.995 [0.989; 1.002] | 1.99E-01 |
| Smoking | none | 2.616 [1.299; 5.266] | 7.07E-03 |
| Platelets | 10*9/L | 0.999 [0.998; 0.999] | 7.39E-05 |
| No. of metastatic sites | none | 1.052 [0.894; 1.237] | 5.42E-01 |
| ALT | U/L | 0.997 [0.993; 1.001] | 1.21E-01 |
| Leukocytes | 10*9/L | 1.005 [1.001; 1.009] | 8.00E-03 |
| AbnormalitiesIdentified[2] | none | 1.405 [1.244; 1.587] | 4.31E-08 |
| MProteinIgA[3] | none | 0.792 [0.684; 0.918] | 1.91E-03 |
| MProteinIgG[4] | none | 0.788 [0.696; 0.893] | 1.80E-04 |
| LightChainKappa[5] | none | 0.632 [0.544; 0.735] | 2.46E-09 |
| LightChainLambda[6] | none | 0.720 [0.615; 0.842] | 4.02E-05 |
| [1]HR = Hazard ratio on original scale<br>[2]Whether the test genetic identified abnormalities<br>[3]Whether the patient's immunoglobulin class of M protein is IgA<br>[4]Whether the patient's immunoglobulin class of M protein is IgG<br>[5]Whether the patient's involved light chain is Kappa<br>[6]Whether the patient's involved light chain is Lambda | | | |

A RoPro1 score specific to ovarian cancer is is given by the formula:
-0.03337 (Albumin-38.74) + 0.35052 (ECOG-0.77) + 0.00022 (LDH-261.65) -0.00104 (Lymphocytes/100 Leukocytes-23.59) -0.01649 (Hemoglobin-11.84) + 0.0017 (ALP-92.92) + 0.0684 (NLR-0.49) + 0.00501 (Chloride-101.92) + 0.00396 (Heart rate-84.27) + 0.00725 (AST-23.74) + 0.01471 (Age-64.54) + 0.02218 (Urea nitrogen-15.47) -0.00578 (Oxygen-96.68) + 0.46146 (TNM stage-3.13) -0.00401 (Protein-69.42) -0.00307 (Systolic RR-128.24) -0.06112 (Eosinophils/100 Leukocytes-2.53) -0.11501 (Bilirubin-0.4) + 0.07324 (Calcium-9.38) + 0.00015 (Sex-0) + 0.58224 (BMI-28.63) -0.00098 (Smoking-0) + 0.06139 (Platelets-333.29) -0.00837 (No. of metastatic sites-0.21) + 0.01713 (ALT-21.46) -0.3742 (Leukocytes-0.82) + 0.41366 (clearCell-0.07)

**Table 13:** Final RoPro1 cox regression model (OS) for the ovarian cancer RoPro1.

| Parameter | units | HR[1] [95% CI] | p-value |
|---|---|---|---|
| Albumin | g/L | 0.967 [0.951; 0.984] | 1.01E-04 |
| ECOG | none | 1.420 [1.298; 1.553] | 1.76E-14 |
| LDH | U/L | 1.000 [1.000; 1.0011 | 4.42E-01 |
| Lymphocytes/100 leukocytes | % | 0.999 [0.990; 1.008] | 8.22E-01 |
| Hemoglobin | g/dL | 0.984 [0.945; 1.024] | 4.26E-01 |
| ALP | U/L | 1.002 [1.001; 1.002] | 1.61E-05 |
| NLR | none | 1.071 [0.928; 1.236] | 3.49E-01 |
| Chloride | mmol/L | 1.005 [0.986; 1.025] | 6.10E-01 |
| Heart rate | bpm | 1.004 [1.000; 1.008] | 3.83E-02 |
| AST | U/L | 1.007 [1.003; 1.012] | 2.61E-03 |
| Age | none | 1.015 [1.009; 1.020] | 1.64E-07 |
| Urea nitrogen | mg/dL | 1.022 [1.014; 1.031] | 3.55E-07 |
| Oxygen | % | 0.994 [0.980; 1.008] | 4.23E-01 |
| TNM Stage | none | 1.586 [1.459; 1.725] | 4.32E-27 |
| Protein | g/L | 0.996 [0.986; 1.006] | 4.46E-01 |
| Systolic RR | mmHg | 0.997 [0.994; 1.000] | 5.59E-02 |
| Eosinophils/100 leukocytes | % | 0.941 [0.898; 0.986] | 1.06E-02 |
| Bilirubin | mg/dL | 0.891 [0.638; 1.246] | 5.01E-01 |
| Calcium | mg/dL | 1.076 [0.944; 1.227] | 2.75E-01 |
| Sex | none | - | - |
| BMI | kg/m$^2$ | 1.000 [0.994; 1.007] | 9.66E-01 |
| Smoking | none | 1.790 [0.438; 7.323] | 4.18E-01 |
| Platelets | 10*9/L | 0.999 [0.998; 1.000] | 3.42E-04 |
| No. of metastatic sites | none | 1.063 [0.992; 1.140] | 8.28E-02 |
| ALT | U/L | 0.992 [0.986; 0.997] | 4.00E-03 |
| Leukocytes | 10*9/L | 1.017 [0.994; 1.041] | 1.48E-01 |
| ExtentOfDebulking[2] | | 0.688 [0.555; 0.852] | 6.20E-04 |
| clearCell[3] | | 1.512 [1.128; 2.028] | 5.74E-03 |
| [1]HR = Hazard ratio on original scale<br>[2]Extent of debulking following surgical treatment for the patient's initial diagnosis of ovarian cancer<br>[3]Clear cell carcinoma yes/no | | | |

A RoPro1 score specific to SCLC is given by the formula:
-0.01875 (Albumin-38.7) + 0.14992 (ECOG-0.94) + 0.00018 (LDH-349.5) -0.00919 (Lymphocytes/100 Leukocytes-21.1) -0.01399 (Hemoglobin-12.88) -0.00014 (ALP-112.17) + 0.00145 (NLR-0.7) -0.01345 (Chloride-99.97) + 0.00372 (Heart rate-85.7) + 0.00486 (AST-29.1) + 0.01048 (Age-66.8) + 0.01096 (Urea nitrogen-16.02) -0.00712 (Oxygen-95.65) + 0.28747 (TNM stage-3.55) -0.00597 (Protein-68.55) -0.0045 (Systolic RR-128.2) -0.02105 (Eosinophils/100 Leukocytes-1.99) -0.07518 (Bilirubin-0.5) + 0.00318 (Calcium-9.36) + 0.16448 (Sex-0.48) -0.01073 (BMI-27.94) + 0.11721 (Smoking-0.98) -0.00127 (Platelets-274.14) + 0.02463 (No. of metastatic sites-0.38) -0.00172 (ALT-27.89) -0.00026 (Leukocytes-9) + 0.58755 (SCLCstage-0.65)

**Table 14:** Final RoPro1 cox regression model (OS) for the SCLC RoPro1.

| Parameter | units | HR[1] [95% CI] | p-value |
|---|---|---|---|
| Albumin | g/L | 0.981 [0.970; 0.993] | 1.13E-03 |
| ECOG | none | 1.162 [1.093; 1.235] | 1.45E-06 |
| LDH | U/L | 1.000 [1.000; 1.000] | 4.73E-02 |
| Lymphocytes/100 leukocytes | % | 0.991 [0.985; 0.997] | 3.43E-03 |
| Hemoglobin | g/dL | 0.986 [0.963; 1.010] | 2.56E-01 |
| ALP | U/L | 1.000 [0.999; 1.000] | 5.83E-01 |
| NLR | none | 1.001 [0.899; 1.115] | 9.79E-01 |
| Chloride | mmol/L | 0.987 [0.978; 0.996] | 4.27E-03 |
| Heart rate | bpm | 1.004 [1.001; 1.006] | 3.31E-03 |
| AST | U/L | 1.005 [1.002; 1.008] | 4.17E-04 |
| Age | none | 1.011 [1.006; 1.015] | 1.04E-05 |
| Urea nitrogen | mg/dL | 1.011 [1.005; 1.017] | 4.16E-04 |
| Oxygen | % | 0.993 [0.971; 1.015] | 5.28E-01 |
| TNM Stage | none | 1.333 [1.223; 1.453] | 6.12E-11 |
| Protein | g/L | 0.994 [0.986; 1.002] | 1.26E-01 |
| Systolic RR | mmHg | 0.996 [0.993; 0.998] | 1.31E-05 |
| Eosinophils/100 leukocytes | % | 0.979 [0.947; 1.012] | 2.12E-01 |
| Bilirubin | mg/dL | 0.928 [0.807; 1.067] | 2.91E-01 |
| Calcium | mg/dL | 1.003 [0.916; 1.098] | 9.45E-01 |
| Sex | none | 1.179 [1.090; 1.275] | 3.70E-05 |
| BMI | kg/m[2] | 0.989 [0.984; 0.995] | 1.83E-04 |
| Smoking | none | 1.124 [0.871; 1.4521 | 3.69E-01 |
| Platelets | 10*9/L | 0.999 [0.998; 0.999] | 8.66E-09 |
| No. of metastatic sites | none | 1.025 [0.978; 1.074] | 3.00E-01 |
| ALT | U/L | 0.998 [0.996; 1.0011 | 1.76E-01 |
| Leukocytes | 10*9/L | 1.000 [0.989; 1.010] | 9.60E-01 |
| SCLCstage[2] | none | 1.800 [1.594; 2.032] | 2.34E-21 |
| [1]HR = Hazard ratio on original scale [2]Extensive disease vs. limited disease as obtained by clinical assessment | | | |

Cohort specific RoPro2 models show consistency of variable relevance is generally high across cohorts. The strongest performance improvement with cohort-specific re-estimated variable weights was seen for chronic lymphocytic leukemia (CLL) ($r^2$=0.12, C-index=0.70, 3-month-AUC=0.81 for general RoPro2; $r^2$=0.17, C-index=0.74, 3-month-AUC=0.83 for CLL-specific RoPro2). The metastatic breast cancer model showed the greatest improvement ($r^2$=0.12, C-index=0.66, 3-month-AUC=0.83 for general RoPro2; $r^2$=0.21, C-index=0.72, 3-month-AUC=0.83 for specific RoPro2) by including cancer-specific biomarkers (hormone receptor status and human epidermal growth factor receptor-2 [HER2]-neu status).

A RoPro2 score specific to Advanced NSCLC is given by the formula: 0.00653(AGE-67.981)+0.16544(Gender-0.537)+0.29991(Smoking-0.877)+0.12237(No. of metastatic sites-0.174)+0.19453(ECOG-0.922)+0.02572(NLR-0.766)-0.00725(BMI-26.899)-0.03658(oxygen-95.996)-0.00259(SBP-126.983)+0.00518(heart rate-85.716)-0.04524(Hgb-12.327)+0.00529(Leukocytes-9.807)+0.01222(urea nitrogen-17.129)+0.14336(calcium-9.323)-0.00051(platelets-298.177)-0.01381(lymphocytes/100 leukocytes in blood-16.727)+0.00251(AST-22.414)+0.00144(ALP-102.824)-0.01326(protein-68.696)-0.00416(ALT-23.117)-0.03692(albumin-37.062)+0.18286(bilirubin-0.467)+0.01683(lymphocytes-1.465)-0.00542(carbon dioxide-26.047)-0.03064(chloride-100.545)+0.11354(monocytes-0.672)-0.03235(eosinophils/100 leukocytes in blood-2.07)+0.00055(LDH-280.888)+0.10307(tumor stage-3.465)+0.05508(SquamousCell-0.269)-0.18049(PrimarySiteTumor_PDL1-0.278).

A RoPro2 score specific to Advanced Melanoma is given by the formula: 0.0062(AGE-65.539)+0.1059(Gender-0.68)-0.13496(Smoking-0.352)-0.01106(No. of metastatic sites-0.368)+0.2725(ECOG-0.758)+0.10207(NLR-0.594)-0.00161(BMI-28.896)-0.03303(oxygen-97.009)-0.0029(SBP-130.345)+0.00699(heart rate-79.392)-0.02029(Hgb-13.099)-0.00206(Leukocytes-8.308)+0.01633(urea nitrogen-18.031)+0.08373(calcium-9.297)-0.00025(platelets-258.758)-0.01065(lymphocytes/100 leukocytes in blood-21.258)+7e-04(AST-24.439)+0.00058(ALP-94.702)-0.01989(protein-68.102)+0.00155(ALT-25.62)-0.04059(albumin-38.916)+0.03968(bilirubin-0.564)+0.0433(lymphocytes-1.674)+1e-04(carbon dioxide-25.859)-0.02939(chloride-102.027)+0.09766(monocytes-0.624)-0.0515(eosinophils/100 leukocytes in blood-2.692)+0.00068(LDH-313.076)+0.13055(tumor stage-3.017).

A RoPro2 score specific to bladder cancer is given by the formula: 0.00347(AGE-71.109)+0.07937(Gender-0.748)+0.05865(Smoking-0.73)+0.14505(No. of metastatic sites-0.144)+0.23068(ECOG-0.895)+0.023(NLR-0.692)-0.00651(BMI-27.624)-0.00666(oxygen-96.81)-7e-04(SBP-128.949)+0.00511(heart rate-81.449)-0.04131(Hgb-11.637)+0.0192(Leukocytes-8.918)+0.01188(urea nitrogen-22.192)+0.16766(calcium-9.292)-0.00095(platelets-286.043)-0.01657(lymphocytes/100 leukocytes in blood-18.953)+0.01467(AST-22.276)+0.00247(ALP-106.899)-0.01493(protein-68.58)-0.00939(ALT-20.732)-0.04466(albumin-37.235)+0.00392(bilirubin-0.464)+0.00895(lymphocytes-1.524)-0.01209(carbon dioxide-24.819)-0.02813(chloride-101.957)+0.06395(monocytes-0.661)-0.02002(eosinophils/100 leukocytes in blood-2.567)-3e-05(LDH-233.144)-0.07393(tumor stage-3.532)-0.26819(Surgery-0.504)+0.11056(TStage-2.465).

A RoPro2 score specific to CLL (chronic lymphocytic leukemia) is given by the formula: 0.05779(AGE-69.951)+0.26067(Gender-0.626)+0.71525(Smoking-0.35)+0.70923(No. of metastatic sites-0.008)+0.39314(ECOG-0.622)-0.22371 (NLR-0.077)-0.00915(BMI-28.769)-0.01203(oxygen-96.811)-0.00232(SBP-129.642)+0.00826(heart rate-77.465)-0.07827(Hgb-11.767)+0.00096(Leukocytes-40.123)+0.00878(urea nitrogen-20.195)+0.01911(calcium-9.216)-0.00137(platelets-160.155)-0.00201(lymphocytes/100 leukocytes in blood-67.021)+0.00313(AST-24.08)+0.00352(ALP-87.048)-0.00622(protein-65.126)-0.00792(ALT-21.752)-0.03883(albumin-40.74)+0.0879(bilirubin-0.621)-0.00713(lymphocytes-28.523)-0.01213(carbon dioxide-25.898)-0.03209(chloride-103.766)+0.05868(monocytes-1.885)+0.03505(eosinophils/100 leukocytes in blood-1.16)+0.00037(LDH-273.307)-0.00708(tumor stage-1.392).

A RoPro2 score specific to DLBCL (Diffuse large B-cell carcinoma) is given by the formula: 0.038(AGE-66.079)+0.22192(Gender-0.547)-0.22649(Smoking-0.351)+0.11979(No. of metastatic sites-0.012)+0.31201(ECOG-0.784)+0.00719(NLR-0.631)-0.01538(BMI-28.581)-0.02739(oxygen-96.915)+0.00373(SBP-128.501)+0.00047(heart rate-83.492)-0.02201 (Hgb-12.038)-0.01588(Leukocytes-7.943)+0.02116(urea nitrogen-18.144)-0.007(calcium-9.344)-0.00098(platelets-260.621)-0.01131(lymphocytes/100 leukocytes in blood-21.008)+0.00335(AST-26.771)+0.00298(ALP-95.829)-0.01163(protein-66.364)-0.01085(ALT-24.473)-0.02293(albumin-37.656)+0.17582(bilirubin-0.563)+0.10388(lymphocytes-1.589)+0.00502(carbon dioxide-25.833)-0.01428(chloride-101.524)+0.22388(monocytes-0.638)+0.00275(eosinophils/100 leukocytes in blood-2.293)+6e-04(LDH-329.933)+0.06839(tumor stage-2.791)+0.44252(BM_CD5-0.168).

A RoPro2 score specific to HCC (hepatocellular carcinoma) is given by the formula: 0.00592(age-66.277)+0.12226(Gender-0.807)-19.43777(Smoking-0.352)-0.00323(No. of metastatic sites-0.094)+0.10789(ECOG-0.922)+0.15643(NLR-0.592)+0.00388(BMI-27.783)-0.01924(oxygen-97.2)-0.00132(SBP-128.568)+0.00801(heart rate-80.125)-0.01479(Hgb-12.489)+0.06616(Leukocytes-6.7)+0.02277(urea nitrogen-16.886)+0.10928(calcium-9.092)+0.00019(platelets-195.961)-0.00532(lymphocytes/100 leukocytes in blood-21.082)+0.00517(AST-68.984)+0.00093(ALP-189.229)-0.00514(protein-71.962)-0.00396(ALT-50.691)-0.04574(albumin-34.109)+0.36517(bilirubin-1.154)-0.0059(lymphocytes-1.339)-0.03001(carbon dioxide-24.955)-0.02278(chloride-101.52)-0.28334(monocytes-0.591)-0.05039(eosinophils/100 leukocytes in blood-2.729)+0.00134(LDH-306.135)+0.15332(tumor stage-3.111).

A RoPro2 score specific to metastatic breast cancer is given by the formula: 0.00269(age-62.989)+0.01453(Gender-0.011)+0.1938(Smoking-0.351)+0.00116(No. of metastatic sites-0.339)+0.2144(ECOG-0.76)+0.13996(NLR-0.449)-0.00682(BMI-29.567)-0.03728(oxygen-96.722)-0.00404(SBP-131.38)+0.0038(heart rate-85.049)-0.05297(Hgb-12.364)-0.02052(Leukocytes-7.427)+0.00939(urea nitrogen-15.999)+0.10216(calcium-9.434)-0.00071(platelets-266.78)-0.00192(lymphocytes/100 leukocytes in blood-23.863)+0.00719(AST-31.774)+0.00065(ALP-120.469)-0.00802(protein-69.839)-0.00303(ALT-28.294)-0.0403(albumin-39.387)-0.03278(bilirubin-0.49)-0.0146(lymphocytes-1.686)-0.00091(carbon dioxide-25.745)-0.01655(chloride-101.831)+0.34615(monocytes-0.532)-0.03497(eosinophils/100 leukocytes in blood-2.15)+0.00051(LDH-302.721)-0.05209(tumor stage-2.853)+0.00353(N24_T0-66.322)-0.67362(Status_ER-0.752)-0.31455(Status_PR-0.576)-0.70817(Status_HER2-0.206).

A RoPro2 score specific to metastatic CRC (colorectal cancer) cancer is given by the formula: 0.01002(age-63.765)+0.05288(Gender-0.558)+0.01467(Smoking-0.351)+0.04867(No. of metastatic sites-0.184) +0.2995(ECOG-0.724)+0.12341(NLR-0.561)-0.00452(BMI-28.025)-0.04628(N28_T0-97.301)-0.00216(SBP-129.373) +0.00387(heart rate-82.507)-0.00925(Hgb-11.801)-0.00807(Leukocytes-8.157)+0.01254(urea nitrogen-15.177) +0.07242(calcium-9.242)-0.00045(platelets-294.471)-0.00861(lymphocytes/100 leukocytes in blood-21.651) +0.00332(AST-31.853)+0.00074(ALP-139.89)-0.00212(protein-69.152)-0.00485(ALT-26.673)-0.04476(albumin-37.617)+0.22807(bilirubin-0.539)-0.02575(lymphocytes-1.635)-0.01573(carbon dioxide-25.449)-0.03205(chloride-101.807)+0.1334(monocytes-0.647)-0.004(eosinophils/100 leukocytes in blood-2.868)+0.00044(LDH-339.711) +0.09735(tumor stage-3.496)+0.53129(Status_BRAF-0.107)+0.18914(Status_KRAS-0.451).

A RoPro2 score specific to metastatic RCC (renal cell carcinoma) is given by the formula: 0.00638(age-66.118)-0.01476(Gender-0.699)+0.00179(Smoking-0.572)+0.06387(No. of metastatic sites-0.272) +0.16002(ECOG-0.86)+0.05433(NLR-0.575)-0.01004(BMI-29.805)-0.0425(N28_T0-96.707)-0.00266(SBP-130.368) +0.00795(heart rate-81.061)-0.00346(Hgb-12.161)+0.04464(Leukocytes-8.072)+0.0106(urea nitrogen-21.186) +0.04739(calcium-9.416)-0.00033(platelets-286.676)-0.00984(lymphocytes/100 leukocytes in blood-21.154) +0.00739(AST-22.983)+0.00049(ALP-109.25)-0.01171(protein-69.997)-0.01121(ALT-24.263)-0.05349(albumin-37.749)-0.0202(bilirubin-0.499)-0.04447(lymphocytes-1.578)-0.00319(carbon dioxide-25.558)-0.03636(chloride-101.488)-0.0937(monocytes-0.626)-0.03918(eosinophils/100 leukocytes in blood-2.579)+0.00121(LDH-261.093) +0.0851(tumor stage-3.145)-0.31275(Nephrectomy-0.66)-0.37341(clearCell_RCC-0.695).

A RoPro2 score specific to multiple myeloma is given by the formula: 0.03943(age-68.434)+0.14741(Gender-0.542)-0.09141(Smoking-0.351)+0.10112(No. of metastatic sites-0.037) +0.32641(ECOG-0.901)-0.05074(NLR-0.267)-0.00195(BMI-29.017)-0.02522(oxygen-97.041)-0.00427(SBP-133.73) +0.00449(heart rate-80.919)-0.0559(Hgb-10.824)-0.00576(Leukocytes-6.364)+0.01195(urea nitrogen-21.864) +0.00914(calcium-9.33)-0.00135(platelets-220.519)-0.00925(lymphocytes/100 leukocytes in blood-28.37) +0.00706(AST-23.754)+0.00196(ALP-84.221)-0.00472(protein-78.166)-0.00766(ALT-23.431)-0.03242(albumin-36.515)+0.26533(bilirubin-0.493)+0.02637(lymphocytes-1.75)-0.003(carbon dioxide-24.927)-0.0161(chloride-101.955)+0.19518(monocytes-0.507)-0.02945(eosinophils/100 leukocytes in blood-2.3)+0.00071(LDH-216.938) +0.17142(tumor stage-1.987)-0.12075(MProteinIgG-0.542)-0.46168(LightChainKappa-0.572)-0.38144(LightChainLambda-0.337).

A RoPro2 score specific to ovarian cancer is given by the formula: 0.00998(age-65.176)+1.02303(Smoking-0.35)+0.11866(No. of metastatic sites-0.082)+0.25502(ECOG-0.778) +0.21763(NLR-0.555)-0.00348(BMI-28.264)-0.05212(oxygen-96.969)-0.00274(SBP-128.397)+0.00062(heart rate-84.888)-0.02728(Hgb-11.746)+0.01832(Leukocytes-7.828)+0.02424(urea nitrogen-15.932)+0.00599(calcium-9.323)-0.00101(platelets-332.437)+0.01093(lymphocytes/100 leukocytes in blood-21.544) +0.00837(AST-23.971)+0.00353(ALP-91.691)-0.01219(protein-68.922)-0.01376(ALT-21.587)-0.03487(albumin-37.78)+0.32377(bilirubin-0.412)-0.03305(lymphocytes-1.526)+0.01351(carbon dioxide-25.341)-0.01256(chloride-102.004)+0.05714(monocytes-0.539)-0.03722(eosinophils/100 leukocytes in blood-2.117) +0.00109(LDH-288.609)+0.40066(tumor stage-3.108).

A RoPro2 score specific to SCLC (Small cell lung cancer) is given by the formula: 0.00891 (age-67.252) +0.22435(Gender-0.481)+0.10511(Smoking-0.985)+0.01216(No. of metastatic sites-0.13)+0.13932(ECOG-0.983) +0.00473(NLR-0.645)-0.00942(BMI-27.722)-0.03779(oxygen-95.68)-0.00509(SBP-127.436)+0.00184(heart rate-85.411)-0.02368(Hgb-12.674)+0.0187(Leukocytes-9.25)+0.00619(urea nitrogen-16.446)+0.04426(calcium-9.3)-0.00107(platelets-282.204)-0.00278(lymphocytes/100 leukocytes in blood-19.709)+0.0059(AST-31.42) +0.00047(ALP-114.166)-0.00912(protein-67.956)-0.00551(ALT-29.195)-0.02229(albumin-37.454)-0.06248(bilirubin-0.52)-0.02092(lymphocytes-1.707)-0.00089(carbon dioxide-26.303)-0.01935(chloride-99.666)-0.03149(monocytes-0.704)-0.0199(eosinophils/100 leukocytes in blood-1.961)+0.00069(LDH-365.223)+0.23314(tumor stage-3.549) +0.47876(SCLCStage-0.648).

A RoPro2 score specific to head and neck cancer is given by the formula: 0.00518(age-64.674)+0.12697(Gender-0.772)-0.02112(Smoking-0.805)+0.09328(No. of metastatic sites-0.111) +0.16008(ECOG-0.894)-0.04024(NLR-0.775)-0.00764(BMI-24.671)-0.03521(oxygen-96.753)-0.00198(SBP-125.17) +0.00672(heart rate-81.443)-0.05918(Hgb-12.345)+0.01424(Leukocytes-8.344)+0.00502(urea nitrogen-17.085) +0.17451 (calcium-9.441)-8e-05(platelets-276.764)-0.01363(lymphocytes/100 leukocytes in blood-16.982) +0.00467(AST-23.164)+0.00271 (ALP-87.966)-0.00349(protein-69.592)-0.00565(ALT-20.505)-0.03886(albumin-38.568)-0.00106(bilirubin-0.483)-0.01571(lymphocytes-1.269)+0.0089(carbon dioxide-26.889)-0.02273(chloride-99.978)+0.10163(monocytes-0.613)-0.02502(eosinophils/100 leukocytes in blood-2.252) +0.00084(LDH-209.625)-0.08805(tumor stage-3.5)-0.22453(HPVStatus-0.472).

A RoPro2 score specific to follicular is given by the formula: 0.0468(age-66.416)+0.70266(Gender-0.516) +1.06061 (No. of metastatic sites-0.012)+0.06024(ECOG-0.556)+0.26978(NLR-0.493)-0.02437(BMI-29.51)-0.17142(oxygen-96.821)+0.00584(SBP-129.754)+0.01086(heart rate-78.578)-0.05796(Hgb-12.858)-0.01222(Leukocytes-7.63)+0.02537(urea nitrogen-17.532)+0.01785(calcium-9.334)-0.00083(platelets-227.01)-0.00522(lymphocytes/100 leukocytes in blood-24.463)+0.01112(AST-22.11)+0.00406(ALP-83.673)+0.01715(protein-67.455)-0.03429(ALT-20.377)-0.07823(albumin-39.938)-0.58047(bilirubin-0.566)-0.04803(lymphocytes-2.253)+0.11677(carbon dioxide-26.334)+0.11495(chloride-102.349)+0.42239(monocytes-0.602)+0.20839(eosinophils/100 leukocytes in blood-2.822)+0.00141(LDH-240.005)-0.14411(tumor stage-3.034).

A RoPro2 score specific to pancreatic cancer is given by the formula: 0.00638(age-67.378)+0.05687(Gender-0.541)-0.62763(Smoking-0.35)+0.00541(No. of metastatic sites-0.093)+0.22063(ECOG-0.894) +0.03629(NLR-0.667)-0.00469(BMI-26.238)-0.02826(oxygen-97.09)-0.00141(SBP-126.099)+0.00776(heart rate-82.714)-0.0175(Hgb-11.903)+0.00485(Leukocytes-8.717)+0.01408(urea nitrogen-15.205)+0.10756(calcium-9.203)-0.00054(platelets-257.947)-0.00733(lymphocytes/100 leukocytes in blood-19.318) +0.00386(AST-37.701)+0.00114(ALP-185.199)-0.00548(protein-66.673)-0.00654(ALT-37.784)-0.04763(albumin-36.334)+0.03451(bilirubin-0.777)-0.10421(lymphocytes-1.539)+0.00822(carbon dioxide-25.679)-0.02157(chloride-100.587)+0.26008(monocytes-0.696)-0.00193(eosinophils/100 leukocytes in blood-2.499) +0.00048(LDH-259.294)+0.00544(tumor stage-3.522)-0.29951(IsSurgery-0.2).

All analyses were conducted with the statistical analysis package R (R Core Team, 2013). **It** will be appreciated that additionally or alternatively, other packages may be employed for analysis.

The systems and methods of the above embodiments may be implemented in a computer system (in particular in computer hardware or in computer software).

The term "computer system" includes the hardware, software and data storage devices for embodying a system or carrying out a method according to the above described embodiments. For example, a computer system may comprise a central processing unit (CPU), input means, output means and data storage. Preferably the computer system has a monitor to provide a visual output display (for example in the design of the business process). The data storage may comprise RAM, disk drives or other computer readable media. The computer system may include a plurality of computing devices connected by a network and able to communicate with each other over that network.

The methods of the above embodiments may be provided as computer programs or as computer program products or computer readable media carrying a computer program which is arranged, when run on a computer, to perform the method(s) described above.

The term "computer readable media" includes, without limitation, any non-transitory medium or media which can be read and accessed directly by a computer or computer system. The media can include, but are not limited to, magnetic storage media such as floppy discs, hard disc storage media and magnetic tape; optical storage media such as optical discs or CD-ROMs; electrical storage media such as memory, including RAM, ROM and flash memory; and hybrids and combinations of the above such as magnetic/optical storage media.

Unless context dictates otherwise, the descriptions and definitions of the features set out above are not limited to any particular aspect or embodiment of the invention and apply equally to all aspects and embodiments which are described.

"and/or" where used herein is to be taken as specific disclosure of each of the two specified features or components with or without the other. For example "A and/or B" is to be taken as specific disclosure of each of (i) A, (ii) B and (iii) A and B, just as if each is set out individually herein.

**It** must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means for example +/- 10%.

Throughout this specification, including the claims which follow, unless the context requires otherwise, the word "comprise" and "include", and variations such as "comprises", "comprising", and "including" will be understood to imply the inclusion of a stated integer or step or group of integers or steps but not the exclusion of any other integer or step or group of integers or steps.

Other aspects and embodiments of the invention provide the aspects and embodiments described above with the term "comprising" replaced by the term "consisting of" or "consisting essentially of", unless the context dictates otherwise.

The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the invention in diverse forms thereof.

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

Table 15 provides details for the 26 parameters included in the Roche prognostic score 1 (RoPro1) and the 29 parameters included in the RoPro2, parameters which may be substituted for the parameters included in the RoPro1 or 2, and known parameters for specific cancer types. The cancer patient information employed in methods described herein may comprise data corresponding to one or more parameters listed in Table 15 as disclosed elsewhere herein.

| Specific Cancer Type | Short variable name | Long variable name | Description | Measurement unit | Levels | Measurement | Modelling in RoPro | Rank in RoPro1 | Rank in RoPro2 | Clinical feasibility (pt = patient) | LOINC | Substitute parameter (correlation) |
|---|---|---|---|---|---|---|---|---|---|---|---|---|
| | Albumin | albumin [mass/volume] in serum or plasma | Albumin [mass/volume] in serum or plasma | g/L | continuous scale | Lab measurement in mass/volume in serum or plasma | | 1 | 1 | | 1751-7 | |
| ECOG | EcogValue | Eastern cooperative oncology group (ECOG) performance status | none | 0,1,2,3,4 | Physician/Nurse assessment: 0: Fully active, able to carry on all pre-disease performance without restriction 1: Restricted in physically strenuous activity but ambulatory and able to carry out work of a light or sedentary nature, e.g., light house work, office work 2: Ambulatory and capable of all self care but unable to carry out any work activities; up and about more than 50% of waking hours 3: Capable of only limited self care; confined to bed or chair more than 50% of waking hours 4: Completely disabled; cannot carry on any self care; totally confined to bed or chair Not documented (ECOG values of 5 are dropped) | 0,1,2,3,4 | 2 | 3 | readily available in the medical history of a pt or through simply asking the pt | | |

EP 3 948 286 B1

| Specific Cancer Type | Short variable name | Long variable name | Description | Measurement unit | Levels | Measurement | Modelling in RoPro | Rank in RoPro1 | Rank in RoPro2 | Clinical feasibility (pt = patient) | LOINC | Substitute parameter (correlation) |
|---|---|---|---|---|---|---|---|---|---|---|---|---|
| | Lymphocytes/ 100 Leukocytes | lymphocytes/ 100 leukocytes in blood | Lymphocytes/ 100 leukocytes ratio in blood | % | continuous scale | Lab measurement as ratio in blood | | 3 | 7 | assessed in routine blood draws | 26478-8 | |
| | Smoking | SmokingStatus | Documented history of smoking | none | History of smoking, No history of smoking, Unknown/ Not documented, (null) | Documented history of smoking. If the patient has ever smoked, they are reported as having a history of smoking. | Dichotomous History of smoking = 1; No history of smoking = 0; unknown/not documented = 0 | 4 | 16 | readily available in the medical history of a pt or through simply asking the pt | | |
| | Age | AgeAt | Age at index date (age at start of line of therapy) | age in years | continuous scale | Calculated as time interval between date of birth and index date (in years) | | 5 | 6 | readily available in the medical history of a pt or through simply asking the pt | | |
| | TNM Stage | GroupStage | Group stage at time of initial diagnosis | none | Stage I, II, IIIa, IIIb, IV, etc. | Summary staging according to TNM values from AJCC version 8 guidelines | Continuous scale I = 1, II = 2 III = 3 IIIa = 2,75 IIIb = 3,25 Etc. ("a" modifies the number by -0.25, "b" by +0.25) | 6 | 9 | readily available in the medical history of a pt or through simply asking the pt | | |
| | Heart rate | heart rate | Heart rate | beats per minute (bpm) | continuous scale | Vital sign parameter measurement as number of heart beats per minute | | 7 | 13 | readily available in the medical history of a pt or through simply asking the pt | 8867-4 | |
| | Chloride | chloride [moles/volume] in serum or plasma | Chloride [moles/volume] in serum or plasma | mmol/L | continuous scale | Lab measurement in mole/volume in serum or plasma | | 8 | 5 | assessed in routine blood draws | 2075-0 | Sodium (r2=0.64) |
| | Urea Nitrogen | urea nitrogen [mass/volume] in serum or plasma | Urea nitrogen [mass/volume] in serum or plasma | mg/dL | continuous scale | Lab measurement in mass/volume in serum or plasma | | 9 | 11 | assessed in routine blood draws | 3094-0 | |

| Specific Cancer Type | Short variable name | Long variable name | Description | Measurement unit | Levels | Measurement | Modelling in RoPro | Rank in RoPro1 | Rank in RoPro2 | Clinical feasibility (pt = patient) | LOINC | Substitute parameter (correlation) |
|---|---|---|---|---|---|---|---|---|---|---|---|---|
| | Gender | Gender | Patient gender | none | Female, Male | | Dichotomous Female = 0; Male = 1; | 10 | 18 | readily available in the medical history of a pt or through simply asking the pt | | |
| | Haemoglobin | haemoglobin [mass/volume] in blood | Haemoglobin [mass/volume] in blood | g/dL | continuous scale | Lab measurement in mass/volume in blood | | 11 | 8 | assessed in routine blood draws | 718-7 or 20509-6 (serum and/or whole blood) | Haematocrit (r2=0.95) |
| | AST | aspartate aminotransferase [enzymatic activity/volume] in serum or plasma | Aspartate aminotransferase [enzymatic activity/volume] in serum or plasma | U/L | continuous scale | Lab measurement as enzymatic activity/volume in serum or plasma | | 12 | 23 | assessed in routine blood draws | 1920-8 | |
| | ALT | alanine aminotransferase [enzymatic activity/volume] in serum or plasma | Alanine aminotransferase [enzymatic activity/volume] in serum or plasma | U/L | continuous scale | Lab measurement as enzymatic activity/volume in serum or plasma | | 13 | 26 | assessed in routine blood draws | 1742-6 | AST (r2=0.65) |
| | Systolic RR | systolic blood pressure | Systolic blood pressure | mmHg | continuous scale | Vital sign parameter measurement as blood pressure during blood ejection of left heart ventricle in mmHg | | 14 | 21 | readily available in the medical history of a pt or through simply asking the pt | 8480-6 | Diastolic RR (r2=0.58) |
| | LDH | lactate dehydrogenase [enzymatic activity/volume] in serum or plasma | Lactate dehydrogenase [enzymatic activity/volume] in serum or plasma | U/L | continuous scale | Lab measurement as [enzymatic activity/volume] in serum or plasma | | 15 | 28 | assessed in routine blood draws | 2532-0 14804-9 | |
| | BMI | BMI | Body mass index | kg/m² | continuous scale | Vital sign parameter computed as body weight/body height^2 | | 16 | 20 | readily available in the medical history of a pt or through simply asking the pt | 39156-5 | |
| | Protein | protein [mass/volume] in serum or plasma | Protein [mass/volume] in serum or plasma | g/L | continuous scale | Lab measurement in mass/volume in serum or plasma | | 17 | 14 | assessed in routine blood draws | 2885-2 | |

| Specific Cancer Type | Short variable name | Long variable name | Description | Measurement unit | Levels | Measurement | Modelling in RoPro | Rank in RoPro1 | Rank in RoPro2 | Clinical feasibility (pt = patient) | LOINC | Substitute parameter (correlation) |
|---|---|---|---|---|---|---|---|---|---|---|---|---|
| | Platelets | platelets [#/volume] in blood | platelets [#/volume] in blood | $10^9$/L | continuous scale | Lab measurement as [#/volume] in blood | | 18 | 10 | assessed in routine blood draws | 26515-7 777-3 778-1 49497-1 | |
| | Metastatic sites | MetSites | Number of metastatic sites at index date (start of line of therapy) | none | continuous scale | Estimated metastatic sites at index date based on ICD-9/ICD-10 codes | Number of metastatic sites | 19 | 27 | not necessarily in patient history and requires prior surgery or tumour biopsy | | |
| | Eosinophils/100 Leukocytes | eosinophils/100 leukocytes in blood | Eosinophils/100 leukocytes ratio in blood | % | continuous scale | Lab measurement as ratio in blood | | 20 | 24 | assessed in routine blood draws | 26450-7 - unspecified 714-6 - manual | |
| | Calcium | calcium [mass/volume] in serum or plasma | Total calcium [mass/volume] in serum or plasma | mg/dL | continuous scale | Lab measurement in mass/volume in serum or plasma | | 21 | 17 | assessed in routine blood draws | 17861-6 | |
| | O$_2$ saturation | oxygen saturation in arterial blood by pulse oximetry | oxygen saturation in arterial blood | % | continuous scale | Vital sign parameter of oxygen saturation in arterial blood by pulse oximetry | | 22 | 19 | readily available in the medical history of a pt or through simply asking the pt | 59408-5 | |
| | ALP | alkaline phosphatase [enzymatic activity/volume] in serum or plasma | alkaline phosphatase [enzymatic activity/volume] in serum or plasma | U/L | continuous scale | Lab measurement as [enzymatic activity/volume] in serum or plasma | | 23 | 12 | assessed in routine blood draws | 1743-4 | |
| | NLR | Neutrophils-to-lymphocytes ratio | Neutrophils [#/volume] in blood to lymphocytes [#/volume] in blood ratio | none (ratio) | continuous scale | Computed as ratio of neutrophils [#/volume] to lymphocytes [#/volume] (both measured in blood) | | 24 | 25 | assessed in routine blood draws | ratio of (26499-4 or 751-8 or 753-4) and (26474-7 or 731-0 or 732-8 or 30364-4) | |
| | Bilirubin | bilirubin.total [mass/volume] in serum or plasma | Total bilirubin [mass/volume] in serum or plasma | mg/dL | continuous scale | Lab measurement in mass/volume in serum or plasma | | 25 | 22 | assessed in routine blood draws | 1975-2 | |

| Specific Cancer Type | Short variable name | Long variable name | Description | Measurement unit | Levels | Measurement | Modelling in RoPro | Rank in RoPro1 | Rank in RoPro2 | Clinical feasibility (pt = patient) | LOINC | Substitute parameter (correlation) |
|---|---|---|---|---|---|---|---|---|---|---|---|---|
| | Leukocytes | leukocytes [#/volume] in blood | Leukocytes [#/volume] in blood | $10^9$/L | continuous scale | Lab measurement as [#/volume] in blood | | 26 | 4 | assessed in routine blood draws | 26464-8 6690-2 | |
| | Lymphocytes | lymphocytes [#/volume] in blood | lymphocytes [#/volume] in blood | $10^9$/L | continuous scale | Lab measurement as [#/volume] in blood | | n/a | 2 | assessed in routine blood draws | 26474-7 | |
| | Carbon dioxide | carbon dioxide, total [moles/volume] in serum or plasma | carbon dioxide, total [moles/volume] in serum or plasma | mmol/L | continuous scale | Lab measurement as [#/volume] in blood | | n/a | 29 | assessed in routine blood draws | 2028-9 | |
| | Monocytes | monocytes [#/volume] in blood | monocytes [#/volume] in blood | $10^9$/L | continuous scale | Lab measurement as [#/volume] in blood | | n/a | 15 | assessed in routine blood draws | 26484-6 | |
| Advanced NSCLC | Squamous Cell | SquamousCell | Squamous cell carcinoma vs non-squamous cell carcinoma | none | dichotomous | histology | Squamous cell carcinoma = 1; non-squamous cell carcinoma = 0 | | | not necessarily in patient history and requires prior surgery or tumour biopsy | | |
| | Primary Site Tumor_PDL1 | PrimarySiteTumor_PDL1 | PDL1 status in primary tumour | none | dichotomous | biomarker status | Positive =1; Negative = 0 | | | not necessarily in patient history and requires prior surgery or tumour biopsy | | |
| | ALK | ALK | Presence of ALK rearrangement, consensus of assessment in blood, tumour site, and metastatic site; Presence of Variants of Unknown Significance (VUS) are recorded as mutation absent | none | dichotomous | biomarker status | Present =1; Absent = 0 | | | not necessarily in patient history and requires prior surgery or tumour biopsy | | |

EP 3 948 286 B1

| Specific Cancer Type | Short variable name | Long variable name | Description | Measurement unit | Levels | Measurement | Modelling in RoPro | Rank in RoPro1 | Rank in RoPro2 | Clinical feasibility (pt = patient) | LOINC | Substitute parameter (correlation) |
|---|---|---|---|---|---|---|---|---|---|---|---|---|
| | EGFR | EGFR | Presence of EGFR mutation, consensus of assessment in blood, tumour site, and metastatic site; Presence of Variants of Unknown Significance (VUS) are recorded as mutation absent | none | dichotomous | biomarker status | Present =1; Absent = 0 | | | not necessarily in patient history and requires prior surgery or tumour biopsy | | |
| | KRAS | KRAS | Presence of KRAS mutation, consensus of assessment in blood, tumour site, and metastatic site; Presence of Variants of Unknown Significance (VUS) are recorded as mutation absent | none | dichotomous | biomarker status | Present =1; Absent = 0 | | | not necessarily in patient history and requires prior surgery or tumour biopsy | | |
| Bladder | Surgery | Surgery | Whether the patient has had a cystectomy or other relevant surgery | none | dichotomous | Clinical assessment | Yes = 1 No = 0 | | | not necessarily in patient history and requires prior surgery or tumour biopsy | | |

| Specific Cancer Type | Short variable name | Long variable name | Description | Measurement unit | Levels | Measurement | Modelling in RoPro | Rank in RoPro1 | Rank in RoPro2 | Clinical feasibility (pt = patient) | LOINC | Substitute parameter (correlation) |
|---|---|---|---|---|---|---|---|---|---|---|---|---|
| | NStage | NStage | N stage at initial diagnosis | none | Stages 1,2,3 | Clinical assessment | Continuous scale N1 = 1 N1a = 0.75 N1b = 1.25 N2 = 2 Etc. ("a" modifies the number by -0.25, "b" by +0.25) | | | not necessarily in patient history and requires prior surgery or tumour biopsy | | |
| | TStage | TStage | T stage at initial diagnosis | none | Stages 1,2,3,4 | Clinical assessment | Continuous scale T1 = 1 T1a = 0.75 T1b = 1.25 T2 = 2 Etc. ("a" modifies the number by -0.25, "b" by +0.25) | | | not necessarily in patient history and requires prior surgery or tumour biopsy | | |
| CLL | hematocrit | hematocrit | hematocrit [volume fraction] of blood | % | continuous | | | | | assessed in routine blood draws | 20570-8 4544-3 | |
| | mono_leuko ratio | mono_leuko ratio | monocytes/100 leukocytes in blood | % | continuous | | | | | assessed in routine blood draws | 26485-3 744-3 5905-5 | |
| | Status17pDel | Status17pDel | Patient's status for the 17p deletion | none | dichotomous | biomarker status | Present =1; Absent = 0 | | | not necessarily in patient history and requires prior surgery or tumour biopsy | | |

44

| Specific Cancer Type | Short variable name | Long variable name | Description | Measurement unit | Levels | Measurement | Modelling in RoPro | Rank in RoPro1 | Rank in RoPro2 | Clinical feasibility (pt = patient) | LOINC | Substitute parameter (correlation) |
|---|---|---|---|---|---|---|---|---|---|---|---|---|
| DLBCL | BM_CD5 | BM_CD5 | CD5 expression status in bone marrow, status of the marker as reported by IHC or Flow Cytometry | none | dichotomous | biomarker status | Positive =1; Negative = 0 | | | not necessarily in patient history and requires prior surgery or tumour biopsy | | |
| HCC | IsAscites | IsAscites | Indicates if the patient had documented evidence of ascites at or within 60 days prior to starting systemic therapy | none | dichotomous | clinical assessment | Present =1; Absent = 0 | | | readily available in the medical history of a pt or through simply asking the pt | | |
| Metastatic Breast Cancer | Status_ER | Status_ER | ER status | none | dichotomous | biomarker status | Positive =1; Negative = 0 | | | not necessarily in patient history and requires prior surgery or tumour biopsy | | |
| | Status_PR | Status_PR | PR status | none | dichotomous | biomarker status | Positive =1; Negative = 0 | | | not necessarily in patient history and requires prior surgery or tumour biopsy | | |
| | Status_HER2 | Status_HER2 | HER2 status, status of the marker as reported by IHC or Flow Cytometry | none | dichotomous | biomarker status | Positive =1; Negative = 0 | | | not necessarily in patient history and requires prior surgery or tumour biopsy | | |
| | granulocytes /100 leukocytes in blood | granulocytes/100 leukocytes in blood | | % | continuous | | | | | assessed in routine blood draws | 30395-8 - unspecified 19023-1 - automated | |

45

| Specific Cancer Type | Short variable name | Long variable name | Description | Measurement unit | Levels | Measurement | Modelling in RoPro | Rank in RoPro1 | Rank in RoPro2 | Clinical feasibility (pt = patient) | LOINC | Substitute parameter (correlation) |
|---|---|---|---|---|---|---|---|---|---|---|---|---|
| Metastatic CRC | Status_BRAF | Status_BRAF | Presence of BRAF mutation; Presence of Variants of Unknown Significance (VUS) are recorded as mutation present | none | dichotomous | biomarker status | Present =1; Absent = 0 | | | not necessarily in patient history and requires prior surgery or tumour biopsy | | |
| | Status_KRAS | Status_KRAS | Presence of KRAS mutation | none | dichotomous | biomarker status | Present =1; Absent = 0 | | | not necessarily in patient history and requires prior surgery or tumour biopsy | | |
| | MSImod_Primary | MSImod_Primary | Assessed in primary tissue, MSI-H and Loss of MMR protein expression are subsumed into one class | none | dichotomous | biomarker status | MSI-H and Loss of MMR protein expression present = 1 MSI-H absent and MMR protein expression normal (loss of MMR protein expression absent) = 0 | | | not necessarily in patient history and requires prior surgery or tumour biopsy | | |
| Metastatic RCC | Nephrectomy | Nephrectomy | Whether the patient has had a nephrectomy | none | dichotomous | clinical assessment | Yes = 1 No = 0 | | | readily available in the medical history of a pt or through simply asking the pt | | |
| | clearCell | clearCell | Clear cell carcinoma yes/no | none | dichotomous | histology | Clear cell carcinoma = 1 Non-clear cell carcinoma = 0 | | | not necessarily in patient history and requires prior surgery or tumour biopsy | | |

| Specific Cancer Type | Short variable name | Long variable name | Description | Measurement unit | Levels | Measurement | Modelling in RoPro | Rank in RoPro1 | Rank in RoPro2 | Clinical feasibility (pt = patient) | LOINC | Substitute parameter (correlation) |
|---|---|---|---|---|---|---|---|---|---|---|---|---|
| Multiple Myeloma | AbnormalitiesIdentified | AbnormalitiesIdentified | Whether the test identified abnormalities | none | dichotomous | genetic test | Present =1; Absent = 0 | | | | | |
| | MProteinIgA | MProteinIgA | Whether the patient's immunoglobulin class of M protein is IgA | none | dichotomous | biomarker status | Yes = 1 No = 0 | | | assessed in routine blood draws (for patients with this indication) | | |
| | MProteinIgG | MProteinIgG | Whether the patient's immunoglobulin class of M protein is IgG | none | dichotomous | biomarker status | Yes = 1 No = 0 | | | assessed in routine blood draws (for patients with this indication) | | |
| | LightChainKappa | LightChainKappa | Whether the patient's involved light chain is Kappa | none | dichotomous | biomarker status | Yes = 1 No = 0 | | | assessed in routine blood draws (for patients with this indication) | | |
| | Light Chain Lambda | LightChainLambda | Whether the patient's involved light chain is Lambda | none | dichotomous | biomarker status | Yes = 1 No = 0 | | | assessed in routine blood draws (for patients with this indication) | | |
| Ovarian | Clear Cell | clearCell | Clear cell carcinoma yes/no | none | dichotomous | histology | Clear cell carcinoma = 1 Non-clear cell carcinoma = 0 | | | not necessarily in patient history and requires prior surgery or tumour biopsy | | |
| SCLC | SCLC Stage | SCLCStage | Extensive disease vs. Limited disease | none | dichotomous | clinical assessment | Extensive disease = 1; Limited disease = 0 | | | | | |

| Specific Cancer Type | Short variable name | Long variable name | Description | Measurement unit | Levels | Measurement | Modelling in RoPro | Rank in RoPro1 | Rank in RoPro2 | Clinical feasibility (pt = patient) | LOINC | Substitute parameter (correlation) |
|---|---|---|---|---|---|---|---|---|---|---|---|---|
| Head/Neck | HPVStatus | HPVStatus | HPV (human papillomavirus) test result (positive vs negative) | none | dichotomous | biomarker status | Positive=1; Negative=0; | | | not necessarily in patient history and requires tumour biopsy | | |
| Pancreatic cancer | IsSurgery | IsSugery | Indication of whether a surgical procedure was performed and removed the patient's primary tumor | none | dichotomous | Clinical assessment | Yes = 1 No = 0 | | | not necessarily in patient history and requires prior surgery or tumour biopsy | | |

**Table 16** shows the average patient times on study BP29428 by RMHS and RoPro1 5% quantiles. The number (N) of patients in each group of low RMHS, high RMHS and each RoPro1 quantile are also shown. The table cells shows patient times (days) on study BP29428 by RMHS (group averages) and RoPro1 5% quantiles classes (running group averages). For RoPro1 the numerical borders of the 5% quantiles are given explicitly.

| RMHS | Days on study (avg) | Number of patients (N) |
|---|---|---|
| high | 86 | 75 |
| low | 125 | 141 |
| **RoPro1 by 5% quantiles** | **Days on study (running avg)** | **Number of patients (N)** |
| > 1.22 | 3 | 11 |
| [0.97; 1.22] | 31 | 11 |
| [0.76; 0.96] | 35 | 11 |
| [0.67; 0.76] | 51 | 11 |
| [0.63; 0.67] | 66 | 11 |
| [0.56; 0.63] | 64 | 11 |
| [0.45; 0.56] | 81 | 11 |
| [0.40; 0.45] | 79 | 11 |
| [0.30; 0.40] | 89 | 11 |
| [0.24; 0.30] | 92 | 11 |
| [0.18; 0.24] | 92 | 11 |
| [0.11; 0.18] | 99 | 11 |
| [0.04; 0.11] | 102 | 11 |
| [-0.01; 0.04] | 106 | 11 |
| [-0.06; -0.01] | 111 | 11 |
| [-0.14; -0.06] | 112 | 11 |
| [-0.24; -0.14] | 111 | 11 |
| [-0.34; -0.24] | 111 | 11 |
| [-0.49; -0.34] | 109 | 11 |
| < -0.49 | 112 | 11 |

**Table 17** shows the average patient times on study BP29428 for patients with a primary diagnosis of bladder cancer by RMHS and RoPro1 10% quantiles. The number (N) of patients in each group of low RMHS, high RMHS and each RoPro1 quantile are also shown. The table cells show patient times (days) on study BP29428 by RMHS (group averages) and RoPro1 10% quantiles classes (running group averages). For RoPro1 the numerical borders of the 10% quantiles are given explicitly. Due to smaller sample size, 10% instead of 5% quantiles are used in this table.

| RMHS | Days on study (avg) | Number of patients (N) |
|---|---|---|
| high | 78 | 21 |
| low | 146 | 41 |
| **RoPro1 by 5% quantiles** | **Days on study (running avg)** | **Number of patients (N)** |
| > 1.21 | 1 | 6 |
| [0.80; 1.21] | 25 | 7 |
| [0.66; 0.80] | 51 | 6 |
| [0.50; 0.66] | 106 | 6 |
| [0.41; 0.50] | 102 | 6 |

(continued)

| RoPro1 by 5% quantiles | Days on study (running avg) | Number of patients (N) |
|---|---|---|
| [0.28; 0.41] | 94 | 6 |
| [0.21; 0.28] | 95 | 6 |
| [0.07; 0.21] | 100 | 6 |
| [-0.08; 0.07] | 121 | 6 |
| < -0.08 | 123 | 7 |

**Table 18** shows the average patient times on study BP29428 for patients by RMHS and RoPro2 10% quantiles. The number (N) of patients in each group of low RMHS, high RMHS and each RoPro2 decile are also shown. The table cells show patient times (days) on study BP29428 by RMHS (group averages) and RoPro2 10% quantiles classes (running group averages). For RoPro2 the numerical borders of the 10% quantiles are given explicitly.

| RMHS | Days on study (mean) | Number of patients (N) |
|---|---|---|
| low | 166 | 141 |
| high | 117 | 75 |
| **RoPro (Deciles)** | **Days on study (mean)** | **Number of patients (N)** |
| [-3.22;-1.26) | - | 0 |
| [-1.26;-0.76) | 203 | 9 |
| [-0.76;-0.42) | 166 | 37 |
| [-0.42;-0.16) | 200 | 43 |
| [-0.16;0.06) | 164 | 39 |
| [0.06;0.27) | 120 | 29 |
| [0.27;0.49) | 136 | 25 |
| [0.49;0.76) | 104 | 14 |
| [0.76;1.13) | 60 | 9 |
| [1.13;3.61) | 29 | 11 |

**Table 19** shows the average patient times on the phase III study OAK for patients by RMHS and RoPro2 10% quantiles. The number (N) of patients in each group of low RMHS, high RMHS and each RoPro2 decile are also shown. The table cells show patient times (days) on the study by RMHS (group averages) and RoPro2 10% quantiles classes (running group averages). For RoPro2 the numerical borders of the 10% quantiles are given explicitly.

| RMHS | Days on study (mean) | Number of patients |
|---|---|---|
| low | 372 | 884 |
| high | 244 | 303 |
| **RoPro (Deciles)** | **Days on study (mean)** | **Number of patients** |
| [-1.94;-0.74) | 471 | 105 |
| [-0.74;-0.52) | 452 | 91 |
| [-0.52;-0.35) | 439 | 111 |
| [-0.35;-0.19) | 395 | 143 |
| [-0.19;-0.05) | 377 | 139 |
| [-0.05;0.11) | 324 | 122 |
| [0.11;0.29) | 314 | 153 |
| [0.29;0.5) | 248 | 134 |

(continued)

| RoPro (Deciles) | Days on study (mean) | Number of patients |
|---|---|---|
| [0.5;0.81) | 201 | 113 |
| [0.81;2.94) | 148 | 76 |

**Table 20:** List of abbreviations

| Abbreviation | Full Name |
|---|---|
| ALP | Alkaline phosphatase |
| ALT | Alanine aminotransferase |
| AST | Aspartate aminotransferase |
| BC | Breast cancer |
| BMI | Body mass index |
| BUN | Blood urea nitrogen |
| CI | Confidence interval |
| CLL | Chronic lymphocytic leukemia |
| CRC | Colorectal cancer |
| CSF1 | Colony stimulating factor-1 |
| DLBCL | Diffuse large B-cell carcinoma |
| ECOG (PS) | Eastern cooperative oncology group (performance status) |
| EHR | Electronic health records |
| HCC | Hepatocellular carcinoma |
| HER2neu or HER2 | Human epidermal growth factor receptor 2 |
| HR | Hazard ratio |
| IPI | International prognostic index |
| KM | Kaplan-Meier |
| LDH | Lactate dehydrogenase |
| MM | Multiple myeloma |
| NLR | Neutrophil-to-lymphocyte ratio |
| NSCLC | Non-small cell lung cancer |
| OS | Overall survival |
| Oxygen | Oxygen saturation in arterial blood |
| PD-L1 | Programmed death-ligand 1 |
| PFS | Progression-free survival |
| RMHS | Royal Marsden Hospital prognostic score |
| RCC | Renal cell carcinoma |
| RoPro | Roche prognostic score |
| RR | Blood pressure |
| rSq | Generalized r-squared ($r^2$) |
| SCLC | Small cell lung cancer |
| TNM | TNM system (Tumor, Node, Metastasis) |
| UK | United Kingdom |

(continued)

| Abbreviation | Full Name |
|---|---|
| LOINC | Logical Observation Identifiers Names and Codes |

## References

A predictive model for aggressive non-Hodgkin's lymphoma. N. Engl.J. Med. 329:987-94, 1993

Altman D, Simon R: Statistical aspects of prognostic factor studies in oncology. Br J Cancer 69:979-985, 1994

Banks E, Joshy G, Abhayaratna WP, et al.: Erectile dysfunction severity as a risk marker for cardiovascular disease hospitalisation and all-cause mortality: a prospective cohort study. PLoS Med 10:e1001372, 2013

Charlson ME, Pompei P, Ales KL, et al.: A new method of classifying prognostic comorbidity in longitudinal studies: Development and validation. J Chronic Dis 373-383, 1987

Cox DR.: Regression Models and Life-Tables. Journal of the Royal Statistical Society Series B (Methodological) Vol. 34

Curtis MD, Griffith SD, Tucker M, et al.: Development and Validation of a High-Quality Composite Real-World Mortality Endpoint. Health Serv Res 53:4460-4476, 2018

Derek Grose, Graham Devereux, Louise Brown, et al.: Simple and Objective Prediction of Survival in Patients with Lung Cancer: Staging the Host Systemic Inflammatory Response. Lung Cancer International Volume 2014, Article ID 731925s.

Ganna A, Ingelsson E: 5 year mortality predictors in 498 103 UK Biobank participants: a prospective population-based study. The Lancet 386:533-540, 2015

Graf E, Schmoor C, Sauerbrei W, et al.: Assessment and comparison of prognostic classification schemes for survival data. Stat Med 18:2529-2545, 1999

Halabi S, Owzar K: The Importance of Identifying and Validating Prognostic Factors in Oncology. Semin Oncol 37:e9-e18, 2010

Hu FB, Willett WC, Li T, et al.: Adiposity as compared with physical activity in predicting mortality among women. N Engl J Med 351:2694-2703, 2004

Jin J, Hu K, Zhou Y, Li W: Clinical utility of the modified Glasgow prognostic score in lung cancer: A meta-analysis. PLoS ONE 12(9): e0184412, 2017.

Kahn MG, Callahan TJ, Barnard J, et al.: A Harmonized Data Quality Assessment Terminology and Framework for the Secondary Use of Electronic Health Record Data. EGEMs Gener Evid Methods Improve Patient Outcomes 4:18, 2016

Kinoshita A, Onoda H, Imai N, et al.: The Glasgow Prognostic Score, an inflammation based prognostic score, predicts survival in patients with hepatocellular carcinoma. BMC Cancer 52, 2013

McGee DL, Liao Y, Cao G, et al.: Self-reported health status and mortality in a multiethnic US cohort. Am J Epidemiol 149:41-46, 1999

Nieder C, Dalhaug A: A new prognostic score derived from phase I study participants with advanced solid tumours is also valid in patients with brain metastasis. Anticancer Res. 977-9, 2010

# EP 3 948 286 B1

R Core Team (2013). R: A language and environment for statistical computing. R Foundation for Statistical Computing, Vienna, Austria. URL http://www.R-project.org/.

Reid VL, McDonald R, Nwosu AC, Mason SR, Probert C, Ellershaw JE, Coyle S: A systematically structured review of biomarkers of dying in cancer patients in the last months of life; An exploration of the biology of dying. PLoS One 12(4), 2017

Rittmeyer A, Barlesi F, Waterkamp D, et al.: Atezolizumab versus docetaxel in patients with previously treated non-small-cell lung cancer (OAK): a phase 3, open-label, multicentre randomised controlled trial. Lancet 389:255-265, 2017

Stock C, Mons U, Brenner H: Projection of cancer incidence rates and case numbers until 2030: A probabilistic approach applied to German cancer registry data (1999-2013). Cancer Epidemiol. 57:110-119, 2018

Sudlow C, Gallacher J, Allen N, et al.: UK Biobank: An Open Access Resource for Identifying the Causes of a Wide Range of Complex Diseases of Middle and Old Age. PLoS Med. 12:e1001779, 2015

Tadahiro Nozoe, Rumi Matono, Hideki Ijichi, Takefumi Ohga, Takahiro Ezaki: Glasgow Prognostic Score (GPS) Can Be a Useful Indicator to Determine Prognosis of Patients With Colorectal Carcinoma. Int. Surg. 99:512-517, 2014.

Thun MJ, Peto R, Lopez AD, et al.: Alcohol consumption and mortality among middle-aged and elderly U.S. adults. N. Engl. J. Med. 337:1705-1714, 1997

Tota-Maharaj R, Blaha MJ, McEvoy JW, et al.: Coronary artery calcium for the prediction of mortality in young adults <45 years old and elderly adults >75 years old. Eur. Heart J. 33:2955-2962, 2012

## Claims

1. A computer-implemented method of assessing risk of mortality of a cancer patient, the method comprising;

using a model to generate a score indicative of a risk of mortality of the cancer patient by inputting cancer patient information to the model; and
assessing the risk of mortality by comparing the generated score to one or more predetermined threshold values, or by comparing the generated score to generated scores for other cancer patients in a same group, wherein the patient information comprises data corresponding to each of the following parameters:

(i) Level of albumin in serum or plasma;
(ii) Eastern cooperative oncology group (ECOG) performance status;
(iii) Ratio of lymphocytes to leukocytes in blood;
(iv) smoking status;
(v) Age;
(vi) TNM classification of malignant tumours stage;
(vii) Heart rate;
(viii) Chloride or sodium level in serum or plasma;
(ix) Urea nitrogen level in serum or plasma;
(x) Gender;
(xi) Haemoglobin or haematocrit level in blood;
(xii) Aspartate aminotransferase enzymatic activity level in serum or plasma; and
(xiii) Alanine aminotransferase enzymatic activity level in serum or plasma.

2. The method of claim 1 wherein the patient information further comprises data corresponding to one or more parameters selected from:

(xiv) Systolic or diastolic blood pressure;
(xv) Lactate dehydrogenase enzymatic activity level in serum or plasma;
(xvi) Body mass index;
(xvii) Protein level in serum or plasma;

(xviii) Platelet level in blood;
(xix) Number of metastatic sites;
(xx) Ratio of eosinophils to leukocytes in blood;
(xxi) Calcium level in serum or plasma;
(xxii) Oxygen saturation level in arterial blood;
(xxiii) Alkaline phosphatase enzymatic activity level in serum or plasma;
(xxiv) Neutrophil to lymphocyte ratio (NLR) in blood;
(xxv) Total bilirubin level in serum or plasma; and
(xxvi) Leukocyte level in blood.

3. The method of claim 1 or 2 wherein the patient information further comprises data corresponding to one or more parameters selected from:

(xxvii) Lymphocyte level in blood;
(xxviii) Carbon dioxide level in blood; and
(xxix) Monocyte level in blood.

4. The method of any of claims 1-3, wherein the assessing of the risk of mortality comprises predicting a treatment response of the cancer patient to an anti-cancer therapy.

5. The method of claim 4, wherein the treatment response is progression-free survival, partial response, complete response, or cancer progression.

6. The method of any preceding claim, the method further comprising forming the model by performing multivariable cox regression analysis on training data, the training data including the parameters selected from the list for a plurality of subjects, preferably at least 1000 subjects.

7. The method of claim 6, wherein forming the model comprises:

assigning a respective weighting, w, to each of the respective parameters selected from the list and assigning a respective mean, m, to each of the respective parameters selected from the list for the plurality of subjects, and

wherein the output of the model is given by a sum over the selected parameters according to the formula:

$$output = \Sigma\ w(input - m).$$

8. A method of assessing risk of mortality of a cancer patient according to any one of claims 1 to 3, wherein the method further comprises assessing whether the risk of mortality is high risk or low risk.

9. A method of selecting a cancer patient for inclusion in a clinical trial, the method comprising assessing whether the cancer patient is at high risk or low risk of mortality using a method according to claim 8, and selecting a patient assessed to be at low risk of mortality for inclusion in the clinical trial.

10. A method of selecting a cancer patient for treatment with an anti-cancer therapy, the method comprising assessing whether the cancer patient is at high risk or low risk of mortality using a method according to claim 8, and selecting a cancer patient assessed to be at low risk of mortality for treatment with the anti-cancer therapy.

11. A method of monitoring a cancer patient during treatment with an anti-cancer therapy, the method comprising assessing whether the cancer patient is at high risk or low risk of mortality using a method according to claim 8, wherein a cancer patient assessed to be at low risk of mortality is selected for continued treatment with the anti-cancer therapy, and a cancer patient assessed to be at high risk of mortality is selected to discontinue treatment with the anti-cancer therapy.

12. A method of evaluating the results of a clinical trial for an anti-cancer therapy carried out on cancer patients, the method comprising assessing whether the cancer patients taking part in the clinical trial are at high risk or low risk of mortality using a method according to claim 8.

13. A method of selecting cancer patients for inclusion in a clinical trial, the method comprising identifying a first and a second cancer patient with the same risk of mortality using a method according to claim 8, and including said patients in the clinical trial.

14. The method according to any preceding claim, wherein the cancer is selected from the group consisting of: melanoma, non-small-cell lung carcinoma (NSCLC), bladder cancer, chronic lymphocytic leukaemia (CLL), diffuse large B-cell lymphoma (DLBCL), hepatocellular carcinoma (HCC), metastatic breast cancer, metastatic colorectal cancer (CRC), metastatic renal cell carcinoma (RCC), multiple myeloma, ovarian cancer, small-cell lung carcinoma (SCLC), follicular lymphoma, pancreatic cancer, and head & neck cancer.

**Patentansprüche**

1. Computerimplementiertes Verfahren zum Bewerten des Mortalitätsrisikos eines Krebspatienten, wobei das Verfahren Folgendes umfasst:

    Verwenden eines Modells zum Erzeugen eines Scores, der ein Mortalitätsrisiko des Krebspatienten angibt, durch Eingeben von Krebspatienteninformationen in das Modell; und
    Bewerten des Mortalitätsrisikos durch Vergleichen des erzeugten Scores mit einem oder mehreren vorbestimmten Schwellenwert(en) oder durch Vergleichen des erzeugten Scores mit erzeugten Scores für andere Krebspatienten in einer selben Gruppe, wobei die Patienteninformationen Daten umfassen, die jedem der folgenden Parameter entsprechen:

        (i) Gehalt von Albumin in Serum oder Plasma;
        (ii) Leistungsstatus gemäß der Eastern Cooperative Oncology Group (ECOG);
        (iii) Verhältnis von Lymphozyten zu Leukozyten im Blut;
        (iv) Raucherstatus;
        (v) Alter;
        (vi) TNM-Klassifikation des Stadiums maligner Tumore;
        (vii) Herzfrequenz;
        (viii) Chlorid- oder Natriumgehalt in Serum oder Plasma;
        (ix) Harnstoffstickstoffgehalt in Serum oder Plasma;
        (x) Geschlecht;
        (xi) Hämoglobin- oder Hämatokritgehalt im Blut;
        (xii) Aspartat-Aminotransferase-Enzymaktivitätsgrad in Serum oder Plasma und
        (xiii) Alanin-Aminotransferase-Enzymaktivitätsgrad in Serum oder Plasma.

2. Verfahren nach Anspruch 1, wobei die Patienteninformationen ferner Daten umfassen, die einem oder mehreren Parameter(n) entsprechen, der/die aus Folgenden ausgewählt ist/sind:

        (xiv) systolischer oder diastolischer Blutdruck;
        (xv) Lactat-Dehydrogenase-Enzymaktivitätsgrad in Serum oder Plasma;
        (xvi) Körpermasseindex;
        (xvii) Proteingehalt in Serum oder Plasma;
        (xviii) Blutplättchenzahl im Blut;
        (xix) Anzahl an Metastasestellen;
        (xx) Verhältnis von Eosinophilen zu Leukozyten im Blut;
        (xxi) Calciumgehalt in Serum oder Plasma;
        (xxii) Sauerstoffsättigungsgrad im Arterienblut;
        (xxiii) alkalische Phosphatase-Enzymaktivitätsgrad in Serum oder Plasma;
        (xxiv) Neutrophile-zu-Lymphozyten-Verhältnis (NLR) im Blut;
        (xxv) Gesamtbilirubingehalt in Serum oder Plasma und
        (xxvi) Leukozytenzahl im Blut.

3. Verfahren nach Anspruch 1 oder 2, wobei die Patienteninformationen ferner Daten umfassen, die einem oder mehreren Parameter(n) entsprechen, der/die aus Folgenden ausgewählt ist/sind:

        (xxvii) Lymphozytenzahl im Blut;

(xxviii) Kohlendioxidgehalt im Blut und

(xxix) Monozytenzahl im Blut.

4. Verfahren nach einem der Ansprüche 1-3, wobei das Bewerten des Mortalitätsrisikos das Vorhersagen eines Behandlungsansprechens des Krebspatienten auf eine Antikrebstherapie umfasst.

5. Verfahren nach Anspruch 4, wobei das Behandlungsansprechen progressionsfreies Überleben, teilweises Ansprechen, vollständiges Ansprechen oder Krebsprogression ist.

6. Verfahren nach einem vorstehenden Anspruch, wobei das Verfahren ferner das Bilden des Modells mittels Durchführen einer multivariablen Cox-Regressions-Analyse an Trainingsdaten umfasst, wobei die Trainingsdaten die Parameter einschließen, die aus der Liste für eine Vielzahl von Individuen, vorzugsweise mindestens 1.000 Individuen, ausgewählt sind.

7. Verfahren nach Anspruch 6, wobei das Bilden des Modells Folgendes umfasst:

Zuordnen einer jeweiligen Wichtung, w, zu jedem der jeweiligen Parameter, die aus der Liste ausgewählt sind, und

Zuordnen eines jeweiligen Mittelwerts, m, zu jedem der jeweiligen Parameter, die aus der Liste für die Vielzahl von Individuen ausgewählt sind, und

wobei die Ausgabe des Modells durch eine Summe über die ausgewählten Parameter gemäß der folgenden Formel angegeben wird:

$$\text{Ausgabe} = \Sigma \ w(\text{Eingabe} - m).$$

8. Verfahren zum Bewerten des Mortalitätsrisikos eines Krebspatienten nach einem der Ansprüche 1 bis 3, wobei das Verfahren ferner das Bewerten, ob das Mortalitätsrisiko hohes Risiko oder geringes Risiko ist, umfasst.

9. Verfahren zum Auswählen eines Krebspatienten für die Aufnahme in eine klinische Prüfung, wobei das Verfahren das Bewerten, ob der Krebspatient hohes oder geringes Mortalitätsrisiko aufweist, unter Anwendung eines Verfahrens nach Anspruch 8 und das Auswählen eines Patienten, bei dem bewertet wurde, dass er ein geringes Mortalitätsrisiko aufweist, für die Aufnahme in die klinische Prüfung umfasst.

10. Verfahren zum Auswählen eines Krebspatienten für die Behandlung mit einer Antikrebstherapie, wobei das Verfahren das Bewerten, ob der Krebspatient hohes oder geringes Mortalitätsrisiko aufweist, unter Anwendung eines Verfahrens nach Anspruch 8 und das Auswählen eines Krebspatienten, bei dem bewertet wurde, dass er ein geringes Mortalitätsrisiko aufweist, für die Behandlung mit der Antikrebstherapie umfasst.

11. Verfahren zum Überwachen eines Krebspatienten während der Behandlung mit einer Antikrebstherapie, wobei das Verfahren das Bewerten, ob der Krebspatient ein hohes oder geringes Mortalitätsrisiko aufweist, unter Anwendung eines Verfahrens nach Anspruch 8 umfasst, wobei ein Krebspatient, bei dem bewertet wurde, dass er ein geringes Mortalitätsrisiko aufweist, für eine weiterführende Behandlung mit der Antikrebstherapie ausgewählt wird und ein Krebspatient, bei dem bewertet wurde, dass er ein hohes Mortalitätsrisiko aufweist, für einen Abbruch der Behandlung mit der Antikrebstherapie auswählt wird.

12. Verfahren zum Auswerten der Ergebnisse einer klinischen Prüfung für eine Antikrebstherapie, die an Krebspatienten durchgeführt wurde, wobei das Verfahren das Bewerten, ob die Krebspatienten, die an der klinischen Prüfung teilnehmen, ein hohes oder geringes Mortalitätsrisiko aufweisen, unter Anwendung eines Verfahrens nach Anspruch 8 umfasst.

13. Verfahren zum Auswählen von Krebspatienten für das Aufnehmen in eine klinische Prüfung, wobei das Verfahren das Identifizieren eines ersten und eines zweiten Krebspatienten mit demselben Mortalitätsrisiko unter Anwendung eines Verfahrens nach Anspruch 8 und das Aufnehmen der Patienten in die klinische Prüfung umfasst.

14. Verfahren nach einem vorstehenden Anspruch, wobei der Krebs aus der Gruppe ausgewählt ist, die aus Folgendem besteht: Melanom, nichtkleinzelligem Lungenkarzinom (NSCLC), Blasenkrebs, chronischer lymphatischer Leukämie (CLL), diffusem großzelligem B-Zell-Lymphom (DLBCL), Leberzellenkarzinom (HCC), metastatischem Brustkrebs, metastatischem Kolorektalkrebs(CRC), metastatischem Nierenzellenkarzinom (RCC), multiplem Myelom, Eierstockkrebs, kleinzelligem Lungenkarzinom (SCLC), follikulärem Lymphom, Bauchspeicheldrüsenkrebs und Kopf- und Halskrebs.

**Revendications**

1. Procédé mis en œuvre par ordinateur d'évaluation du risque de mortalité d'un patient atteint de cancer, le procédé comprenant :

   l'utilisation d'un modèle pour générer un score indiquant un risque de mortalité du patient atteint de cancer en entrant des informations du patient atteint de cancer dans le modèle ; et
   l'évaluation du risque de mortalité en comparant le score généré à une ou plusieurs valeurs seuil prédéterminées, ou en comparant le score généré à des scores générés pour d'autres patients atteints de cancer dans un même groupe, dans lequel les informations du patient comprennent des données correspondant à chacun des paramètres suivants :

   (i) Taux d'albumine dans le sérum ou le plasma ;
   (ii) Score de performance selon l'Eastem Cooperative Oncology Group (ECOG) ;
   (iii) Rapport lymphocytes sur leucocytes dans le sang ;
   (iv) Tabagisme ;
   (v) Âge ;
   (vi) Classification TNM du stade des tumeurs malignes ;
   (vii) Rythme cardiaque ;
   (viii) Taux de chlorure ou de sodium dans le sérum ou le plasma ;
   (ix) Taux d'azote uréique dans le sérum ou le plasma ;
   (x) Sexe ;
   (xi) Taux d'hémoglobine ou d'hématocrite dans le sang ;
   (xii) Taux d'activité enzymatique de l'aspartate aminotransférase dans le sérum ou le plasma ; et
   (xiii) Taux d'activité enzymatique de l'alanine aminotransférase dans le sérum ou le plasma.

2. Procédé selon la revendication 1 dans lequel les informations du patient comprennent en outre des données correspondant à un ou plusieurs paramètres choisis parmi :

   (xiv) Tension artérielle systolique ou diastolique ;
   (xv) Taux d'activité enzymatique de la lactate déshydrogénase dans le sérum ou le plasma ;
   (xvi) Indice de masse corporelle ;
   (xvii) Taux de protéines dans le sérum ou le plasma ;
   (xviii) Taux de plaquettes dans le sang ;
   (xix) Nombre de sites métastatiques ;
   (xx) Rapport des éosinophiles aux leucocytes dans le sang ;
   (xxi) Taux de calcium dans le sérum ou le plasma ;
   (xxii) Taux de saturation en oxygène dans le sang artériel ;
   (xxiii) Taux d'activité enzymatique de la phosphatase alcaline dans le sérum ou le plasma;
   (xxiv) Rapport neutrophiles sur lymphocytes (RNL) dans le sang ;
   (xxv) Taux de bilirubine totale dans le sérum ou le plasma ; et
   (xxvi) Taux de leucocytes dans le sang.

3. Procédé selon la revendication 1 ou 2 dans lequel les informations du patient comprennent en outre des données correspondant à un ou plusieurs paramètres choisis parmi :

   (xxvii) Taux de lymphocytes dans le sang ;
   (xxviii) Taux de dioxyde de carbone dans le sang ; et
   (xxix) Taux de monocytes dans le sang.

4. Procédé selon l'une quelconque des revendications 1 à 3, dans lequel l'évaluation du risque de mortalité comprend la prédiction d'une réponse au traitement du patient atteint de cancer à une thérapie anticancéreuse.

**5.** Procédé selon la revendication 4, dans lequel la réponse au traitement est une survie sans progression, une réponse partielle, une réponse complète ou une progression du cancer.

**6.** Procédé selon une quelconque revendication précédente, le procédé comprenant en outre la formation du modèle en réalisant une analyse par régression de Cox multivariable sur des données d'entraînement, les données d'entraînement comprenant les paramètres choisis dans la liste pour une pluralité de sujets, de préférence au moins 1000 sujets.

**7.** Procédé selon la revendication 6, dans lequel la formation du modèle comprend :

l'attribution d'une pondération, w, respective à chacun des paramètres respectifs choisis dans la liste et l'attribution d'une moyenne, m, respective à chacun des paramètres respectifs choisis dans la liste pour la pluralité de sujets, et
dans lequel la sortie du modèle est donnée par une somme sur les paramètres choisis selon la formule :

$$\text{sortie} = \Sigma \; \text{w}(\text{entrée} - \text{m}).$$

**8.** Procédé d'évaluation du risque de mortalité d'un patient atteint de cancer selon l'une quelconque des revendications 1 à 3, dans lequel le procédé comprend en outre l'évaluation si le risque de mortalité est un risque élevé ou un risque faible.

**9.** Procédé de sélection d'un patient atteint de cancer pour inclusion dans un essai clinique, le procédé comprenant l'évaluation si le patient atteint de cancer présente un risque élevé ou un risque faible de mortalité en utilisant un procédé selon la revendication 8, et la sélection d'un patient évalué comme présentant un risque faible de mortalité pour inclusion dans l'essai clinique.

**10.** Procédé de sélection d'un patient atteint de cancer pour traitement avec une thérapie anticancéreuse, le procédé comprenant l'évaluation si le patient atteint de cancer présente un risque élevé ou un risque faible de mortalité en utilisant un procédé selon la revendication 8, et la sélection d'un patient atteint de cancer évalué comme présentant un risque faible de mortalité pour traitement avec la thérapie anticancéreuse.

**11.** Procédé de surveillance d'un patient atteint de cancer pendant le traitement avec une thérapie anticancéreuse, le procédé comprenant l'évaluation si le patient atteint de cancer présente un risque élevé ou un risque faible de mortalité en utilisant un procédé selon la revendication 8, dans lequel un patient atteint de cancer évalué comme présentant un risque faible de mortalité est choisi pour poursuivre le traitement avec la thérapie anticancéreuse, et un patient atteint de cancer évalué comme présentant un risque élevé de mortalité est choisi pour interrompre le traitement avec la thérapie anticancéreuse.

**12.** Procédé d'évaluation des résultats d'un essai clinique pour une thérapie anticancéreuse réalisée sur des patients atteints de cancer, le procédé comprenant l'évaluation si les patients atteints de cancer participant à l'essai clinique présentent un risque élevé ou un risque faible de mortalité en utilisant un procédé selon la revendication 8.

**13.** Procédé de sélection de patients atteints de cancer pour inclusion dans un essai clinique, le procédé comprenant l'identification d'un premier et d'un second patient atteint de cancer avec le même risque de mortalité en utilisant un procédé selon la revendication 8, et l'inclusion desdits patients dans l'essai clinique.

**14.** Procédé selon une quelconque revendication précédente, dans lequel le cancer est choisi dans le groupe constitué par : un mélanome, un carcinome pulmonaire non à petites cellules (CPNPC), un cancer de la vessie, une leucémie lymphocytaire chronique (LLC), un lymphome diffus à grandes cellules B (LDGCB), un carcinome hépatocellulaire (CHC), un cancer du sein métastatique, un cancer colorectal (CCR) métastatique, un carcinome à cellules rénales (CCR) métastatique, un myélome multiple, un cancer de l'ovaire, un carcinome pulmonaire à petites cellules (CPPC), un lymphome folliculaire, un cancer du pancréas et un cancer de la tête et du cou.

# Fig. 1A

| Parameter | Hazard Ratio | HR | 95% CI |
|---|---|---|---|
| **Host** | | | |
| Albumin | | 0.84 | 0.83-0.85 |
| Lymphocytes/100 leukocytes | | 0.86 | 0.84-0.87 |
| Chloride | | 0.91 | 0.90-0.92 |
| Hemoglobin | | 0.92 | 0.91-0.93 |
| ALT | | 0.94 | 0.93-0.95 |
| Systolic RR | | 0.94 | 0.94-0.95 |
| Protein | | 0.95 | 0.94-0.96 |
| Platelets | | 0.95 | 0.94-0.96 |
| Eosinophils/100 leukocytes | | 0.96 | 0.95-0.96 |
| Oxygen | | 0.97 | 0.96-0.97 |
| Bilirubin | | 1.02 | 1.01-1.03 |
| Leukocytes | | 1.02 | 1.01-1.03 |
| ALP | | 1.03 | 1.02-1.04 |
| NLR | | 1.03 | 1.02-1.04 |
| Calcium | | 1.04 | 1.03-1.05 |
| LDH | | 1.05 | 1.04-1.06 |
| AST | | 1.07 | 1.06-1.09 |
| Urea nitrogen | | 1.09 | 1.08-1.10 |
| Gender | | 1.09 | 1.08-1.10 |
| Heart rate | | 1.10 | 1.09-1.11 |
| Age | | 1.12 | 1.11-1.13 |
| ECOG | | 1.18 | 1.17-1.19 |
| | | | |
| **Lifestyle** | | | |
| BMI | | 0.95 | 0.94-0.96 |
| Smoking | | 1.13 | 1.11-1.15 |
| | | | |
| **Tumor** | | | |
| Metastatic sites | | 1.05 | 1.04-1.06 |
| TNM Stage | | 1.12 | 1.11-1.13 |

0.8    0.9    1    1.1    1.2

Protective  Detrimental

# Fig. 1B

EP 3 948 286 B1

Protective

Detrimental

LIFESTYE*

HOST*

TUMOR*

BMI

Platelets
Oxygen ALT
Lymphocytes/100 leukocytes
Albumin
Protein Chloride
Hemoglobin
Systolic RR
Eosinophils/100 leukocytes

Smoking

Urea nitrogen
Gender Age
Bilirubin ALP
ECOG
Heart rate NLR
AST Calcium Leukocytes
LDH

TNM Stage
Metastatic sites

* word size corresponds to the
strength of association
in each category

# Fig. 1C

| Parameter | Hazard Ratio | HR* | 95% CI* |
|---|---|---|---|
| **Host** | | | |
| Albumin | | 0.43 | 0.42-0.45 |
| Lymphocytes | | 0.51 | 0.43-0.61 |
| Chloride | | 0.63 | 0.61-0.66 |
| Lymphocytes/100 leukocytes | | 0.66 | 0.59-0.73 |
| Hemoglobin | | 0.70 | 0.68-0.73 |
| Platelets | | 0.71 | 0.68-0.73 |
| Protein | | 0.75 | 0.72-0.78 |
| Oxygen saturation | | 0.78 | 0.76-0.81 |
| Systolic blood pressure | | 0.80 | 0.77-0.83 |
| Eosinophils/100 leukocytes | | 0.82 | 0.80-0.86 |
| ALT | | 0.83 | 0.80-0.87 |
| Carbon dioxide | | 0.95 | 0.92-0.98 |
| LDH | | 1.14 | 1.09-1.18 |
| NLR | | 1.20 | 1.14-1.27 |
| AST | | 1.23 | 1.17-1.29 |
| Bilirubin | | 1.24 | 1.19-1.28 |
| Gender | | 1.28 | 1.23-1.32 |
| Calcium | | 1.30 | 1.25-1.35 |
| Monocytes | | 1.32 | 1.24-1.41 |
| Heart rate | | 1.39 | 1.34-1.43 |
| ALP | | 1.40 | 1.34-1.45 |
| Urea nitrogen | | 1.40 | 1.36-1.45 |
| Age | | 1.56 | 1.50-1.62 |
| Leukocytes | | 1.61 | 1.40-1.85 |
| ECOG | | 1.81 | 1.74-1.87 |
| **Lifestyle** | | | |
| BMI | | 0.80 | 0.77-0.82 |
| Smoking | | 1.32 | 1.27-1.38 |
| **Tumor** | | | |
| Number of metastatic sites | | 1.15 | 1.12-1.18 |
| Tumor stage | | 1.42 | 1.36-1.48 |

0.4   0.75 1   1.5

# Fig. 1D

LIFESTYE*

HOST*

TUMOR*

Oxygen saturation
Hemoglobin

## Lymphocytes
## Albumin

BMI

Chloride Protein
Carbon dioxide

Lymphocytes/100 leukocytes

Platelets    ALT

Systolic blood pressure
Eosinophils/100 leukocytes

Tumor stage
Number of metastatic sites

Smoking

Monocytes
Urea nitrogen

AST ALP Age LDH
Bilirubin

ECOG NLR

Leukocytes

Heart rate  Gender
Calcium

Protective

Risk

* word size corresponds to the
strength of association
in each category

# Fig. 1D(Cont.)

**LIFESTYE\***  **HOST\***  **TUMOR\***

Protective

Detrimental

Oxygen saturation
Hemoglobin
**Lymphocytes**
**Albumin**
**Chloride** Protein
Carbon dioxide
**Lymphocytes/100 leukocytes**
**Platelets** ALT
Systolic blood pressure
Eosinophils/100 leukocytes

BMI

Tumor stage
Number of metastatic sites

Smoking

Monocytes
Urea nitrogen
AST ALP Age LDH Bilirubin
ECOG NLR
Leukocytes
Heart rate Gender
Calcium

EP 3 948 286 B1

## Fig. 2A

# Fig. 2B

## Fig. 3A

Course of RoPro Prior to Different Outcome Events

Days before event

- Patients with complete response
- Patients who died
- Patients with partial response
- Patients with progression
- Patients with stable disease

Fig. 3B

Course of RoPro Prior to Different Outcome Events

Patients with complete response
Patients who died
Patients with partial response
Patients with progression
Patients with stable disease

Days before event

RoPro

# Fig. 4A

RMHS:  ☐ High  ☐ Low

Number at Risk

| RMHS: | 0 | 180 | 360 | 540 | 720 | 900 | 1080 | 1260 | 1440 |
|---|---|---|---|---|---|---|---|---|---|
| High | 92715 | 67208 | 48176 | 35331 | 26528 | 19767 | 14720 | 10987 | 8150 |
| Low | 6534 | 3216 | 1877 | 1196 | 817 | 562 | 388 | 270 | 201 |

Time after first line of treatment initiation (days)

Fig. 4B

RoPro Class: ▭ Remaining 95%   ▭ Upper 5%

Number at Risk

| RoPro Class: | 0 | 180 | 360 | 540 | 720 | 900 | 1080 | 1260 | 1440 |
|---|---|---|---|---|---|---|---|---|---|
| Remaining 95% | 94286 | 68932 | 49475 | 36243 | 27208 | 20248 | 15063 | 11235 | 8336 |
| Upper 5% | 4963 | 1492 | 578 | 284 | 137 | 81 | 45 | 22 | 15 |

Time after first line of treatment initiation (days)

Fig. 4C

# Fig. 4D

A. KM plot (OS) by high/low RMHS

RMHS: low high

Survival probability vs. Time after first line of treatment initiation (days)

Number at risk

| RMHS: | | | | | | | | | |
|---|---|---|---|---|---|---|---|---|---|
| low | 100458 | 73061 | 51969 | 38203 | 28788 | 21819 | 16630 | 12513 | 9438 |
| high | 10080 | 4720 | 2726 | 1740 | 1201 | 837 | 603 | 431 | 320 |

Time after first line of treatment initiation (days)

# Fig. 4E

B. KM plot (OS) by high 10% RoPro vs remaining 90% RoPro

RoPro class: ▬ remaining 90%  ▬ upper 10%

| Number at risk | | | | | | | | |
|---|---|---|---|---|---|---|---|---|
| remaining 90% | 99484 | 74350 | 53249 | 39213 | 29591 | 22430 | 17093 | 12867 | 9707 |
| upper 10% | 11054 | 3431 | 1446 | 730 | 398 | 226 | 140 | 77 | 51 |

Time after first line of treatment initiation (days)

EP 3 948 286 B1

EP 3 948 286 B1

## Fig. 14F

C. KM plot (OS) by RoPro deciles

RoPro decile: decile 1 — decile 3 — decile 5 — decile 7 — decile 9
decile 2 — decile 4 — decile 6 — decile 8 — decile 10

Survival probability vs. Time after first line of treatment initiation (days)

Number at risk

| RoPro decile: | 0 | 180 | 360 | 540 | 720 | 900 | 1080 | 1260 | 1440 |
|---|---|---|---|---|---|---|---|---|---|
| decile 1 | 11054 | 9784 | 8648 | 7566 | 6575 | 5605 | 4735 | 3936 | 3268 |
| decile 2 | 11054 | 9453 | 7854 | 6480 | 5267 | 4214 | 3351 | 2601 | 1979 |
| decile 3 | 11054 | 9277 | 7358 | 5779 | 4488 | 3414 | 2558 | 1877 | 1367 |
| decile 4 | 11053 | 9017 | 6797 | 4994 | 3647 | 2681 | 1964 | 1385 | 1019 |
| decile 5 | 11054 | 8645 | 6089 | 4187 | 3010 | 2140 | 1515 | 1077 | 763 |
| decile 6 | 11054 | 8356 | 5496 | 3635 | 2471 | 1671 | 1152 | 791 | 532 |
| decile 7 | 11053 | 7605 | 4603 | 2907 | 1906 | 1274 | 891 | 589 | 392 |
| decile 8 | 11054 | 6655 | 3703 | 2184 | 1330 | 861 | 557 | 366 | 235 |
| decile 9 | 11054 | 5558 | 2701 | 1481 | 897 | 570 | 370 | 245 | 152 |
| decile 10 | 11054 | 3431 | 1446 | 730 | 398 | 226 | 140 | 77 | 51 |

Time after first line of treatment initiation (days)

Fig. 5A

Fig. 5B

RoPro at Baseline and at
Event Time (death)

P = 6.5e-14

Baseline    At Event

Fig. 5C

RoPro at Baseline and at
Event Time (progression)

P = 3.9e-11

Baseline    At Event

Fig. 5D

RoPro at Baseline and at
Event Time (response)

P = 0.16

Baseline    At Event

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- *N. Engl. J. Med.*, 1993, vol. 329, 987-94
- **COX DR**. *Journal of the Royal Statistical Society Series B (Methodological)*, vol. 34
- *R Core Team*, 2013
- A predictive model for aggressive non-Hodgkin's lymphoma. *N. Engl.J. Med.*, 1993, vol. 329, 987-94
- **ALTMAN D** ; **SIMON R**. Statistical aspects of prognostic factor studies in oncology.. *Br J Cancer*, 1994, vol. 69, 979-985
- **BANKS E** ; **JOSHY G** ; **ABHAYARATNA WP et al.** : Erectile dysfunction severity as a risk marker for cardiovascular disease hospitalisation and all-cause mortality: a prospective cohort study. *PLoS Med*, 2013, vol. 10, e1001372
- **CHARLSON ME** ; **POMPEI P** ; **ALES KL et al.** : A new method of classifying prognostic comorbidity in longitudinal studies: Development and validation. *J Chronic Dis*, 1987, 373-383
- Cox DR.: Regression Models and Life-Tables. *Journal of the Royal Statistical Society Series B (Methodological)*, vol. 34
- **CURTIS MD** ; **GRIFFITH SD** ; **TUCKER M et al.** : Development and Validation of a High-Quality Composite Real-World Mortality Endpoint.. *Health Serv Res*, 2018, vol. 53, 4460-4476
- **DEREK GROSE** ; **GRAHAM DEVEREUX** ; **LOUISE BROWN et al.** Simple and Objective Prediction of Survival in Patients with Lung Cancer: Staging the Host Systemic Inflammatory Response. *Lung Cancer International*, vol. 2014
- **GANNA A**. Ingelsson E: 5 year mortality predictors in 498 103 UK Biobank participants: a prospective population-based study.. *The Lancet*, 2015, vol. 386, 533-540
- **GRAF E** ; **SCHMOOR C** ; **SAUERBREI W et al.** : Assessment and comparison of prognostic classification schemes for survival data. *Stat Med*, 1999, vol. 18, 2529-2545
- **HALABI S** ; **OWZAR K**. The Importance of Identifying and Validating Prognostic Factors in Oncology. *Semin Oncol*, 2010, vol. 37, e9-e18
- **HU FB** ; **WILLETT WC** ; **LI T et al.** : Adiposity as compared with physical activity in predicting mortality among women. *N Engl J Med*, 2004, vol. 351, 2694-2703
- **JIN J** ; **HU K** ; **ZHOU Y** ; **LI W**. Clinical utility of the modified Glasgow prognostic score in lung cancer: A meta-analysis. *PLoS ONE*, 2017, vol. 12 (9), e0184412
- **KAHN MG** ; **CALLAHAN TJ** ; **BARNARD J et al.** : A Harmonized Data Quality Assessment Terminology and Framework for the Secondary Use of Electronic Health Record Data. *EGEMs Gener Evid Methods Improve Patient Outcomes*, 2016, vol. 4, 18
- **KINOSHITA A** ; **ONODA H** ; **IMAI N et al.** The Glasgow Prognostic Score, an inflammation based prognostic score, predicts survival in patients with hepatocellular carcinoma. *BMC Cancer*, 2013, vol. 52
- **MCGEE DL** ; **LIAO Y** ; **CAO G et al.** : Self-reported health status and mortality in a multiethnic US cohort. *Am J Epidemiol*, 1999, vol. 149, 41-46
- **NIEDER C** ; **DALHAUG A**. A new prognostic score derived from phase I study participants with advanced solid tumours is also valid in patients with brain metastasis. *Anticancer Res.*, 2010, 977-9
- R: A language and environment for statistical computing. R Foundation for Statistical Computing. *R Core Team*, 2013, http://www.R-project.org/
- **REID VL** ; **MCDONALD R** ; **NWOSU AC** ; **MASON SR** ; **PROBERT C** ; **ELLERSHAW JE** ; **COYLE S**. A systematically structured review of biomarkers of dying in cancer patients in the last months of life; An exploration of the biology of dying. *PLoS One*, 2017, vol. 12 (4)
- **RITTMEYER A** ; **BARLESI F** ; **WATERKAMP D et al.** : Atezolizumab versus docetaxel in patients with previously treated non-small-cell lung cancer (OAK): a phase 3, open-label, multicentre randomised controlled trial. *Lancet*, 2017, vol. 389, 255-265
- **STOCK C** ; **MONS U** ; **BRENNER H**. Projection of cancer incidence rates and case numbers until 2030: A probabilistic approach applied to German cancer registry data (1999-2013).. *Cancer Epidemiol.*, 2018, vol. 57, 110-119
- **SUDLOW C** ; **GALLACHER J** ; **ALLEN N et al.** UK Biobank: An Open Access Resource for Identifying the Causes of a Wide Range of Complex Diseases of Middle and Old Age. *PLoS Med*, 2015, vol. 12, e1001779

- **TADAHIRO NOZOE** ; **RUMI MATONO** ; **HIDEKI IJICHI** ; **TAKEFUMI OHGA** ; **TAKAHIRO EZAKI**. Glasgow Prognostic Score (GPS) Can Be a Useful Indicator to Determine Prognosis of Patients With Colorectal Carcinoma. *Int. Surg*, 2014, vol. 99, 512-517

- **THUN MJ** ; **PETO R** ; **LOPEZ AD et al.** Alcohol consumption and mortality among middle-aged and elderly U.S. adults. *N. Engl. J. Med.*, 1997, vol. 337, 1705-1714
- **TOTA-MAHARAJ R** ; **BLAHA MJ** ; **MCEVOY JW et al.** : Coronary artery calcium for the prediction of mortality in young adults <45 years old and elderly adults >75 years old. *Eur. Heart J.*, 2012, vol. 33, 2955-2962